# EUROPÄISCHE PATENTANMELDUNG

(11) **EP 4 826 757 A2**
(43) Veröffentlichungstag der Anmeldung: **21.10.2026**
(21) Anmeldenummer: 26186110.8
(22) Anmeldetag: 26.03.2025
(51) Int. Cl.: H01J 35/16

(54) **RÖNTGENEINHEIT, BILDGEBUNGSGERÄT, VERFAHREN ZUM BETRIEB EINER RÖNTGENEINHEIT, VERFAHREN ZUM ERFASSEN VON BILDDATEN UND COMPUTERPROGRAMMPRODUKT**

(62) Dokumentnummer(n) der früheren Anmeldung(en) nach Art. 76 EPÜ: 25166270.6 / 4 815 023
(71) Anmelder: Siemens Healthineers AG, 91301 Forchheim (DE)
(72) Erfinder: Batzke, Martin, 91301 Forchheim (DE); Freudenberger, Jörg, 91301 Forchheim (DE); Glawion, Sebastian, 91301 Forchheim (DE); Hall, Katrin, 91341 Röttenbach (DE); Jung, Nico, 07407 Rudolstadt (DE); Kappler, Steffen, 91341 Röttenbach (DE); Nanke, Ralf, 91301 Forchheim (DE); Radicke, Marcus, 91301 Forchheim (DE); Späth, Stefan, 91301 Forchheim (DE)
(74) Vertreter: Siemens Healthineers Patent Attorneys

(57) **Zusammenfassung**

Die vorliegende Erfindung betrifft eine Röntgeneinheit, umfassend eine Röntgenröhre und eine Spule,
wobei die Röntgenröhre eine Kathode, eine Drehanode und ein Vakuumgehäuse umfasst, wobei die Kathode und die Drehanode innerhalb des Vakuumgehäuses angeordnet sind,
wobei die Spule außerhalb des Vakuumgehäuses angeordnet ist,
wobei die Spule und die Röntgenröhre voneinander galvanisch getrennt sind,
wobei die Spule dazu ausgebildet ist, ein Magnetfeld zu erzeugen,
wobei das Magnetfeld dazu ausgebildet ist, einen in einem Brennfleck der Röntgenröhre auf den Anodenteller auftreffenden, von der Kathode ausgehenden Elektronenstrahl abzulenken, wobei das Vakuumgehäuse ein Strahlenaustrittsfenster zum Durchlass einer von dem Brennfleck auf dem Anodenteller ausgehenden Röntgenstrahlung aufweist.

Die vorliegende Erfindung betrifft ferner ein Bildgebungsgerät, ein Verfahren zum Betrieb einer Röntgeneinheit, ein Verfahren zum Erfassen von Bilddaten mittels eines Bildgebungsgeräts sowie ein Computerprogrammprodukt.

## Beschreibung

Die vorliegende Erfindung betrifft eine Röntgeneinheit, ein Bildgebungsgerät, ein Verfahren zum Betrieb einer Röntgeneinheit, ein Verfahren zum Erfassen von Bilddaten mittels eines Bildgebungsgeräts sowie ein Computerprogrammprodukt.

Die vorliegende Erfindung bezieht sich auf das Gebiet der medizinischen Bildgebung, insbesondere auf Röntgeneinheiten und Bildgebungsgeräte, die im Rahmen einer Diagnostik und/oder bei medizinischen Untersuchungen eingesetzt werden. Solche Geräte finden breite Anwendung in verschiedenen Bereichen der Medizin, wie beispielsweise in der Mammographie, der Computertomographie und/oder der allgemeinen Radiologie.

In der medizinischen Bildgebung wird Röntgenstrahlung verwendet, um Aufnahmen eines Untersuchungsobjekts, beispielsweise eines menschlichen Körpers oder Körperteils, zu erstellen. Dabei kann eine Röntgenröhre eingesetzt werden, die Röntgenstrahlung erzeugt, welche dann durch das zu untersuchende Gewebe des Untersuchungsobjekts geleitet und von einem Röntgendetektor erfasst wird. Die Intensität der erfassten Röntgenstrahlung kann je nach Dichte und Zusammensetzung des durchstrahlten Gewebes variieren, wodurch ein Bild der inneren Strukturen entsteht.

Herkömmliche Röntgeneinheiten umfassen typischerweise eine Röntgenröhre mit einer Kathode und einer Anode, die in einem Vakuumgehäuse angeordnet sind. Die Kathode emittiert Elektronen, die durch eine Hochspannung zur Anode beschleunigt werden. Beim Auftreffen der Elektronen auf die Anode wird Röntgenstrahlung erzeugt, die durch ein Strahlenaustrittsfenster aus der Röntgenröhre austritt.

Ein bekanntes Problem bei herkömmlichen Röntgeneinheiten ist die begrenzte Kontrolle über den Elektronenstrahl innerhalb der Röntgenröhre. Dies kann zu Einschränkungen in der Bildqualität und der Flexibilität bei der Bildgebung führen. Insbesondere bei fortgeschrittenen Bildgebungstechniken wie der Tomosynthese, bei der mehrere Aufnahmen aus verschiedenen Winkeln gemacht werden, kann eine präzise Steuerung des Elektronenstrahls von großer Bedeutung sein.

Ein Brennfleck, insbesondere ein Bereich auf der Anode, auf dem der Elektronenstrahl auftrifft und die Röntgenstrahlung erzeugt wird, kann eine Bildqualität entscheidend beeinflussen. Bei Techniken wie der Tomosynthese bewegt sich die Röntgenröhre relativ zum Röntgendetektor, was zu einer Bewegung des Brennflecks bezüglich des Röntgendetektors führt. Diese Bewegung kann Unschärfen und Verzerrungen im Bild verursachen, da sich der Ursprung der Röntgenstrahlung während der Aufnahme verändert. Hierdurch kann eine räumliche Auflösung und ein Kontrast der Bilddaten beeinträchtigt werden, was besonders bei der Erkennung feiner Strukturen wie Mikrokalzifikationen problematisch ist.

Es ist daher die Aufgabe der vorliegenden Erfindung, eine verbesserte Bildqualität bei Aufnahmen mit einer bewegten Röntgeneinheit zu ermöglichen.

Alle personenbezogenen Formulierungen sind geschlechtsneutral zu verstehen.

Die Aufgabe wird erfindungsgemäß gelöst durch den Gegenstand der unabhängigen Ansprüche. Vorteilhafte Ausführungsformen mit zweckmäßigen Weiterbildungen sind Gegenstand der Unteransprüche.

Die Erfindung betrifft in einem ersten Aspekt eine Röntgeneinheit. Die Röntgeneinheit umfasst eine Röntgenröhre und eine Spule. Die Röntgenröhre umfasst eine Kathode, eine Drehanode und ein Vakuumgehäuse. Die Drehanode umfasst einen um eine Anodenachse rotierbar gelagerten Anodenteller. Dabei kann die Drehanode eine Ausführungsform einer Anode darstellen. Die Kathode und die Drehanode sind innerhalb des Vakuumgehäuses angeordnet. Die Spule ist außerhalb des Vakuumgehäuses angeordnet. Die Spule und die Röntgenröhre sind voneinander galvanisch getrennt. Die Spule ist dazu ausgebildet, ein Magnetfeld zu erzeugen, welches einen Zwischenraum zwischen der Kathode und dem Anodenteller durchdringt. Das Magnetfeld ist dazu ausgebildet, einen in einem Brennfleck der Röntgenröhre auf den Anodenteller auftreffenden, von der Kathode ausgehenden Elektronenstrahl abzulenken. Das Vakuumgehäuse weist an einer bezüglich der Anodenachse radial angeordneten Seite ein Strahlenaustrittsfenster auf. Das Strahlenaustrittsfenster ist zum Durchlass einer von dem Brennfleck auf dem Anodenteller ausgehenden Röntgenstrahlung ausgebildet.

Die Röntgeneinheit, insbesondere auch als Röntgenquelle bezeichnet, kann eine Vorrichtung sein, die zur Erzeugung und Emission von Röntgenstrahlung ausgebildet ist. Die Röntgeneinheit kann verschiedene Komponenten umfassen, die zusammenwirken, um die Röntgenstrahlung zu erzeugen und zu steuern.

Die Röntgenröhre kann als eine Vakuumröhre zur Erzeugung von Röntgenstrahlung ausgebildet sein. Die Röntgenröhre umfasst die Kathode, die Drehanode und das Vakuumgehäuse.

Die Kathode kann als eine Elektrode ausgebildet sein, die dazu ausgebildet ist, Elektronen, insbesondere den Elektronenstrahl, zu emittieren. Die Kathode kann beispielsweise aus einem Material wie Wolfram hergestellt sein. Zudem kann die Kathode dazu ausgebildet sein, durch elektrische Erhitzung Elektronen freizusetzen. Die Kathode kann eine zum Anodenteller hin orientierte Elektronenemissionsfläche aufweisen. Der Elektronenstrahl kann als ein gerichteter Strom von Elektronen ausgebildet sein, der von der Kathode emittiert und zum Anodenteller beschleunigt wird. Dabei müssen nicht sämtliche von der Kathode emittierten Elektronen innerhalb eines definierten räumlichen Bereichs, insbesondere einer definierten Fläche, auf dem Anodenteller auftreffen. Der Brennfleck kann als der räumliche Bereich auf dem Anodenteller ausgebildet sein, auf den der Elektronenstrahl auftrifft und wo die Röntgenstrahlung erzeugt wird. Eine Größe und/oder Form des Brennflecks können eine Qualität der erzeugten Röntgenstrahlung beeinflussen. Ein Teil der von der Kathode emittierten Elektronen kann aufgrund unterschiedlicher Faktoren wie Streuung, Ablenkung durch Magnetfelder und/oder thermische Bewegung außerhalb des Brennflecks auf den Anodenteller gelangen.

Die Drehanode kann als eine rotierbare Anode, insbesondere ein rotierbarer Anodenteller, ausgebildet sein, die dazu ausgebildet ist, die von der Kathode emittierten Elektronen aufzufangen und Röntgenstrahlung zu erzeugen. Röntgenstrahlung kann als eine Form elektromagnetischer Strahlung mit Wellenlängen im Bereich von etwa 0,002 bis 1 Nanometern ausgebildet sein. Die Röntgenstrahlung kann durch das Abbremsen hochenergetischer Elektronen im Anodenmaterial des Anodentellers erzeugt werden. Die Drehanode umfasst den Anodenteller, der um die Anodenachse rotierbar gelagert ist. Der Anodenteller kann als eine scheibenförmige Struktur ausgebildet sein, die eine Zielfläche für den Elektronenstrahl darstellt. Der Anodenteller kann insbesondere rotationssymmetrisch bezüglich eines Rotationszentrums ausgebildet sein. Der Anodenteller kann an einem Ende einer Welle befestigt sein, welche entlang der Anodenachse verläuft. Der Anodenteller kann aus einem Grundkörper, insbesondere umfassend eine Molybdänlegierung, mit einer Beschichtung aus einem hochschmelzenden Material wie Wolfram oder einer Wolfram-Rhenium-Legierung hergestellt sein. Die Anodenachse kann als eine Rotationsachse des Anodentellers ausgebildet sein, um die der Anodenteller rotierbar gelagert ist. Dabei kann die Anodenachse durch einen, insbesondere geometrischen, Mittelpunkt des Anodentellers und senkrecht zu einer Oberfläche, insbesondere Ebene, der scheibenförmigen Struktur des Anodentellers verlaufen. Vorteilhafterweise kann der Anodenteller in einem Betriebszustand der Röntgeneinheit um die Anodenachse rotieren.

Das Vakuumgehäuse kann als ein luftdichtes Gehäuse ausgebildet sein, das die Kathode und die Drehanode umschließt und in einem Betriebszustand der Röntgeneinheit ein Vakuum, insbesondere ein Hochvakuum, aufrechterhält. Das Vakuumgehäuse kann aus einem Material wie Glas, Keramik und/oder Metall hergestellt sein. Das Vakuumgehäuse kann dazu ausgebildet sein, ein Vakuum im Inneren aufrechtzuerhalten und die Komponenten der Röntgenröhre zu umschließen. Das Vakuumgehäuse kann eine komplexe dreidimensionale Form aufweisen, die dazu ausgebildet sein kann, die internen Komponenten der Röntgenröhre aufzunehmen und gleichzeitig die Vakuumintegrität aufrechtzuerhalten. Beispielsweise kann das Vakuumgehäuse einen im Wesentlichen zylindrischen Hauptkörper mit einem oder mehreren Vorsprüngen oder Erweiterungen zur Aufnahme bestimmter Komponenten aufweisen. Der zylindrische Hauptkörper des Vakuumgehäuses kann sich entlang einer Längsachse der Röntgenröhre erstrecken. An einem Ende des zylindrischen Hauptkörpers kann das Vakuumgehäuse einen Kathodengehäuseabschnitt aufweisen. Der Kathodengehäuseabschnitt kann etwas breiter als der Hauptkörper sein und eine kuppelförmige oder halbkugelförmige Endkappe aufweisen. An einem gegenüberliegenden Ende des zylindrischen Hauptkörpers kann das Vakuumgehäuse einen Anodengehäuseabschnitt aufweisen. Der Anodengehäuseabschnitt kann durch eine konische oder sich verjüngende Form gekennzeichnet sein, die sich zum Ende hin verengt. Der Anodengehäuseabschnitt kann in einer flachen oder leicht abgerundeten Endfläche enden. Das Vakuumgehäuse kann eine seitliche Erweiterung oder Ausbuchtung zur Aufnahme des Strahlenaustrittsfensters aufweisen. Diese Erweiterung kann radial zur Anodenachse angeordnet sein und aus dem zylindrischen Hauptkörper herausragen. Eine Form dieser Erweiterung kann von der Seite betrachtet rechteckig oder trapezförmig sein, wobei die Abmessungen an eine Größe des Strahlenaustrittsfensters angepasst sind.

Des Weiteren kann das Vakuumgehäuse Kühlrippen und/oder -kanäle auf seiner Außenfläche aufweisen, insbesondere in Bereichen nahe der Drehanode. Diese Rippen können kreisförmig um den Hauptkörper herum oder in einem spiralförmigen Muster angeordnet sein.

Das Vakuumgehäuse kann ferner Befestigungspunkte oder Flansche an verschiedenen Stellen aufweisen, beispielsweise ein kreisförmiger Flansch an einem kathodenseitigen Ende des Vakuumgehäuses und ähnliche Befestigungspunkte an dem anodenseitigen Ende des Vakuumgehäuses, insbesondere zur Sicherung der Drehanode und/oder eines zugehörigen Rotationsmechanismus.

Eine Oberfläche des Vakuumgehäuses kann glatt und poliert sein, um eine Ausgasung zu minimieren und eine Vakuumintegrität aufrechtzuerhalten. In Bereichen des Vakuumgehäuses, in denen die Spule extern angeordnet ist, kann das Vakuumgehäuse eine reduzierte Wandstärke aufweisen oder aus einem Material gefertigt sein, das eine effiziente Durchdringung des Magnetfelds ermöglicht.

Eine Gesamtform und/oder Abmessungen des Vakuumgehäuses können optimiert sein, um Faktoren wie ein internes Volumen, eine Oberfläche für Wärmeableitung, eine mechanische Festigkeit und/oder eine Kompatibilität mit externen Komponenten, beispielsweise der Spule und/oder etwaigen Abschirmungs- oder Kühlsystemen, auszugleichen.

Die Spule kann als eine elektrische Komponente ausgebildet sein. Die Spule kann aus einem elektrisch leitfähigen Material, beispielsweise Kupferdraht, gewickelt sein. Die Spule kann dazu ausgebildet sein, das Magnetfeld zu erzeugen. Das Magnetfeld kann als ein Bereich ausgebildet sein, in dem magnetische Kräfte wirken. Die Spule ist dazu ausgebildet, das Magnetfeld so zu erzeugen, dass es den Zwischenraum zwischen der Kathode und dem Anodenteller durchdringt. Der Zwischenraum zwischen der Kathode und dem Anodenteller kann als der räumliche Bereich innerhalb der Röntgenröhre ausgebildet sein, durch den der Elektronenstrahl von der Kathode zum Anodenteller verläuft. Das von der Spule erzeugbare Magnetfeld kann dazu ausgebildet sein, den Elektronenstrahl in seiner Flugbahn zu beeinflussen, insbesondere abzulenken. Die Ablenkung des Elektronenstrahls kann als eine Änderung der Flugbahn des Elektronenstrahls durch das Magnetfeld ausgebildet sein. Die Ablenkung kann dazu dienen, eine Position des Brennflecks auf dem Anodenteller zu steuern. Durch Variation einer Bestromung der Spule kann eine Stärke des Magnetfelds und damit die Ablenkung des Elektronenstrahls gesteuert werden. Das Magnetfeld ist dazu ausgebildet, den in einem Brennfleck der Röntgenröhre auf den Anodenteller auftreffenden, von der Kathode ausgehenden Elektronenstrahl abzulenken.

Die Spule und die Röntgenröhre sind voneinander galvanisch getrennt. Die galvanische Trennung kann als eine elektrische Isolation zwischen der Spule und der Röntgenröhre ausgebildet sein. Im Kontext der Röntgeneinheit bedeutet dies, dass die Spule und die Röntgenröhre elektrisch voneinander isoliert sind. Durch die galvanische Trennung zwischen der Röntgenröhre und der Spule kann vorteilhaft eine, insbesondere wechselseitige, Beeinflussung zwischen der Hochspannung in der Röntgenröhre und der Spule, insbesondere dem Magnetfeld der Spule, vermieden werden. Insbesondere können durch die galvanische Trennung Überschläge in der Röntgenröhre (engl. arcing) vorteilhafterweise keinen Einfluss auf Ablenkung des Elektronenstrahls und die Ablenkung des Elektronenstrahls keinen Einfluss auf eine Hochspannungsstabilität der Röntgenröhre bei Ablenkung haben.

Die Spule, die als Ablenkspule für den Elektronenstrahl ausgebildet ist, ist außerhalb des Vakuumgehäuses der Röntgenröhre angeordnet und steht vorteilhafterweise in keiner direkten elektrischen Verbindung mit den Komponenten der Röntgenröhre. Hierdurch kann ein Isolationsaufwand innerhalb der Röntgenröhre reduziert werden, da keine zusätzlichen Hochspannungsisolationen für die Spule erforderlich sind. Die Spule kann sich somit auf einer separaten "Isolationsinsel" befinden, die elektrisch unabhängig von der Röntgenröhre ist.

Die galvanische Trennung kann beispielsweise durch einen Luftspalt und/oder ein isolierendes Material zwischen der Spule und dem Vakuumgehäuse der Röntgenröhre realisiert sein. Der Luftspalt und/oder das Isolationsmaterial können so dimensioniert sein, dass sie eine erforderliche elektrische Isolation gewährleisten, während sie gleichzeitig eine effektive magnetische Kopplung für die Ablenkung des Elektronenstrahls ermöglicht.

Um die galvanische Trennung aufrechtzuerhalten, kann die Spule von einer separaten Stromversorgungseinheit gespeist, insbesondere bestromt, werden, die unabhängig von einer Hochspannungsversorgung der Röntgenröhre arbeitet. Steuerungssignale für die Spule können über optische und/oder kapazitive Koppler übertragen werden, um eine vollständige elektrische Isolation zu gewährleisten.

Die Spule kann einen Öffnungsbereich, insbesondere eine Öffnung und/oder Bohrung, aufweisen, der dazu ausgebildet ist, die Röntgenröhre, insbesondere das Vakuumgehäuse, zumindest teilweise aufzunehmen. Ein Durchmesser des Öffnungsbereichs kann gleich oder minimal größer sein als ein Außendurchmesser des Vakuumgehäuses, um einen Abstand zwischen der Spule und dem Elektronenstrahl zu minimieren. Vorteilhafterweise kann die Spule so geformt sein, dass sie den Konturen der Röntgenröhre, insbesondere des Vakuumgehäuses, eng folgt, was eine Effizienz einer Magnetfeldwechselwirkung mit dem Elektronenstrahl verbessern kann. Die Spule kann eine zylindrische Form oder eine komplexe Geometrie aufweisen. Vorteilhafterweise kann die Spule einen oder mehrere Abschnitte, insbesondere Segmente, aufweisen.

Weist die Spule mehrere Abschnitte auf, so können die mehreren Abschnitte unabhängig voneinander steuerbar sein, um eine präzisere Manipulation des Magnetfelds zu ermöglichen. Die Spule kann vorteilhafterweise eine axiale Ausdehnung aufweisen, dass das erzeugbare Magnetfeld einen gesamten Weg des Elektronenstrahls von der Kathode bis zum Anodenteller ausreichend abdeckt. Vorteilhafterweise kann die Spule in einem Gehäuse eingekapselt und/oder eingegossen sein, beispielsweise in ein Harz. Dieses Gehäuse kann auch dazu dienen, die Spule vor äußeren Beschädigungen und Verunreinigungen zu schützen. Des Weiteren kann die Spule auf einer Trägerstruktur montiert sein, die eine Feineinstellung einer Position der Spule relativ zur Röntgenröhre ermöglicht. Diese Einstellbarkeit kann eine präzise Ausrichtung des Magnetfelds bezüglich des Elektronenstrahls ermöglichen, was eine Genauigkeit der Ablenkung verbessern kann.

Das Strahlenaustrittsfenster, insbesondere auch als Austrittsfenster bezeichnet, kann als ein Bereich des Vakuumgehäuses ausgebildet sein, der für Röntgenstrahlung durchlässig ist. Insbesondere kann das Strahlenaustrittsfenster zum Durchlass der von dem Brennfleck auf dem Anodenteller ausgehenden Röntgenstrahlung ausgebildet sein. Das Strahlenaustrittsfenster kann aus einem Material hergestellt sein, das eine hohe Transmission für Röntgenstrahlung aufweist, beispielsweise Beryllium, einer dünnen Metallfolie und/oder einem Verbundmaterial. Das Strahlenaustrittsfenster ist an einer bezüglich der Anodenachse radial angeordneten Seite angeordnet. Die radiale Anordnung des Strahlenaustrittsfensters bezüglich der Anodenachse kann verschiedene Konfigurationen umfassen. Das Strahlenaustrittsfenster kann in einer Position angeordnet sein, die seitlich zur Anodenachse liegt. Beispielsweise kann eine Durchlassrichtung des Strahlenaustrittsfensters in einem Winkel zwischen 60 und 120°, insbesondere 90°, zur Anodenachse angeordnet sein. Dabei muss das Strahlenaustrittsfenster nicht notwendigerweise parallel zur Anodenachse ausgerichtet sein. Diese Konfiguration kann es ermöglichen, dass die vom Brennfleck auf dem Anodenteller erzeugte Röntgenstrahlung effektiv aus dem Vakuumgehäuse austreten kann, ohne dass das Fenster direkt in der Verlängerung der Anodenachse oder genau in einer Richtung senkrecht zur Anodenachse liegen muss.

Vorteilhafterweise kann die Spule derart bezüglich der Röntgenröhre, insbesondere des Vakuumgehäuses, angeordnet sein, dass der Öffnungsbereich der Spule den Bereich der Röntgenröhre, insbesondere des Vakuumgehäuses, zumindest teilweise aufnimmt, in welchem die Röntgenstrahlung von dem Brennfleck auf der Anode zu dem Strahlenaustrittsfenster verläuft. Insbesondere kann die Spule an der gleichen, radial bezüglich der Anodenachse angeordneten, Seite angeordnet sein, wie das Strahlenaustrittsfenster.

Das Strahlenaustrittsfenster kann eine planare oder gekrümmte Form aufweisen. Die Krümmung kann konvex, konkav oder komplexer gestaltet sein, um spezifische Strahlungscharakteristiken zu erzielen und/oder eine strukturelle Integrität des Fensters zu verbessern.

Die vorgeschlagene Röntgeneinheit kann eine präzise Steuerung des Brennflecks auf dem Anodenteller ermöglichen, ohne eine Vakuumintegrität der Röntgenröhre zu beeinträchtigen. Dies kann zu einer verbesserten Bildqualität und Flexibilität bei bildgebenden Verfahren beitragen.

Die Röntgeneinheit kann ferner einen Röntgenfilter umfassen. Der Röntgenfilter kann dazu ausgebildet sein, niedrige Energieanteile der Röntgenstrahlung zu absorbieren und/oder ein Röntgenspektrum der Röntgenstrahlung zu optimieren. Der Röntgenfilter kann Aluminium, Kupfer, Titan, Rhodium, Silber und/oder Molybdän umfassen. Der Röntgenfilter kann zwischen dem Brennfleck auf dem Anodenteller und dem Strahlenaustrittsfenster angeordnet sein, beispielsweise an oder in einer Blendeneinheit. Dies kann eine präzise Filterung der Röntgenstrahlung ermöglichen, unmittelbar nachdem die Röntgenstrahlung im Brennfleck erzeugt wurde. Dadurch kann der Röntgenfilter niedrigenergetische Photonen effektiv herausfiltern, bevor die Röntgenstrahlung das Strahlenaustrittsfenster passiert.

Der Röntgenfilter kann eine gleichmäßigere Bestrahlung eines Zielbereichs und eine Reduktion von Bildrauschen ermöglichen, was zu klareren und diagnostisch aussagekräftigeren Röntgenbildern führen kann. Zudem kann eine Strahlendosis für ein zu untersuchendes Untersuchungsobjekt vorteilhaft reduziert werden.

Das Untersuchungsobjekt kann beispielsweise eine menschliche und/oder tierische Patientin und/oder ein menschlicher und/oder tierischer Patient und/oder ein Untersuchungsphantom sein.

Gemäß einer vorteilhaften Ausführungsform kann die Röntgeneinheit jeweilige Stromversorgungseinheiten zur separaten Bestromung der Röntgenröhre und der Spule aufweisen. Diese Konfiguration kann eine präzisere Steuerung der einzelnen Komponenten ermöglichen.

Die Stromversorgungseinheit zur Bestromung der Röntgenröhre kann eine Hochspannungsquelle umfassen, die dazu ausgebildet sein kann, eine Spannung im Bereich von etwa 10 kV bis 300 kV zwischen Kathode und Drehanode bereitzustellen. Diese Hochspannungsquelle kann beispielsweise einen Hochspannungstransformator, einen Gleichrichter und einen Spannungsregler umfassen. Insbesondere kann die Hochspannungsquelle als Resonanzwandler ausgeführt sein, um eine hohe Effizienz und geringe Welligkeit der Ausgangsspannung zu erreichen. Zusätzlich zur Hochspannungsversorgung kann die Stromversorgungseinheit zur Bestromung der Röntgenröhre eine separate Niederspannungsquelle für eine Heizung der Kathode umfassen. Diese Heizstromversorgung kann beispielsweise einen 10 mA bis 20000 mA liefern, abhängig von den spezifischen Anforderungen der Kathode.

Die Stromversorgungseinheit zur Bestromung der Spule kann als separate Einheit ausgeführt sein. Die Stromversorgungseinheit zur Bestromung der Spule kann dazu ausgebildet sein, einen präzise steuerbaren Strom zu liefern. Die Stromversorgungseinheit zur Bestromung der Spule kann beispielsweise einen Gleichstromwandler mit einer Ausgangsspannung von 0 V bis 50 V und einer maximalen Stromstärke von 0 A bis 20 A umfassen. Insbesondere kann die Spulenstromversorgung zur Bestromung der Spule als Vier-Quadranten-Stromquelle ausgeführt sein, um schnelle Änderungen des Magnetfelds zu ermöglichen.

Die Trennung der Stromversorgungseinheiten für Röntgenröhre und Spule kann mehrere technische Vorteile bieten. Zum einen kann dadurch die gegenseitige Beeinflussung der Stromkreise minimiert werden, was zu einer verbesserten Stabilität und Genauigkeit der Elektronenstrahlsteuerung führen kann. Die separate Spannungsversorgung kann es ermöglichen, die Spule unabhängig von der Hochspannung der Röntgenröhre zu betreiben, was eine flexiblere Steuerung des Magnetfelds erlaubt. Darüber hinaus kann die Trennung der Stromversorgungen dazu beitragen, elektromagnetische Interferenzen zu reduzieren. Die Hochspannungsversorgung der Röntgenröhre kann potenziell Störungen erzeugen, die die präzise Steuerung des Ablenkstroms beeinträchtigen könnten. Durch die Verwendung separater, abgeschirmter Stromversorgungseinheiten kann dieses Risiko minimiert werden.

Vorteilhafterweise können die Stromversorgungseinheiten jeweils mit digitalen Steuerungsschnittstellen ausgestattet sein, welche eine präzise und schnelle Anpassung der Betriebsparameter ermöglichen. Dies kann beispielsweise eine Verwendung von digitalen Signalprozessoren (DSPs) oder Field Programmable Gate Arrays (FPGAs) zur Echtzeit-Steuerung der jeweiligen Bestromung umfassen.

Insgesamt kann die Verwendung separater Stromversorgungseinheiten für die Röntgenröhre und die Spule zu einer verbesserten Leistung, Stabilität und Flexibilität der Röntgeneinheit beitragen. Die Möglichkeit, die Betriebsparameter der einzelnen Komponenten unabhängig voneinander zu optimieren, kann die Bildqualität verbessern.

In einer weiteren vorteilhaften Ausführungsform der vorgeschlagenen Röntgeneinheit kann der Anodenteller als Einwinkelteller ausgebildet sein.

Ein Einwinkelteller kann einen Anodenteller bezeichnen, der eine einzige geneigte Oberfläche aufweist. Die geneigte Oberfläche kann dabei den Zielbereich für den auftreffenden Elektronenstrahl umfassen. Die geneigte Oberfläche kann dabei einen ring- und/oder streifenförmigen Abschnitt auf einer der Kathode zugewandten Seite des Anodentellers umfassen. Vorteilhafterweise kann der Anodenteller bezüglich eines Rotationszentrums rotationssymmetrisch ausgebildet sein. Dabei kann die geneigte Oberfläche in einem radialen Querschnitt des Einwinkeltellers geradlinig verlaufen. Der Winkel des Einwinkeltellers kann als Anodenwinkel bezeichnet werden. Der Anodenwinkel kann einen Winkel zwischen der geneigten Oberfläche und einer Rotationsebene des Anodentellers bezeichnen, wobei die Rotationsebene des Anodentellers senkrecht zur Anodenachse verläuft. Dieser Winkel kann beispielsweise zwischen 7° und 20° betragen.

Der Einwinkelteller, insbesondere der Winkel der geneigten Oberfläche, kann eine gezielte Ausrichtung der erzeugten Röntgenstrahlung ermöglichen. Dies kann vorteilhaft sein, um den Röntgenstrahl, insbesondere die Röntgenstrahlung, auf das Strahlenaustrittsfenster und nachfolgende Komponenten wie einen Röntgendetektor auszurichten.

Die Verwendung eines Einwinkeltellers kann eine gleichmäßige Wärmeverteilung auf der Anodenoberfläche ermöglichen, da der Elektronenstrahl auf einer größeren Fläche auftrifft. Dies kann eine Lebensdauer des Anodentellers verlängern.

Die Ausbildung als Einwinkelteller kann auch eine Fokussierung der Röntgenstrahlung beeinflussen. Insbesondere kann der Einwinkelteller dazu beitragen, eine Größe des Brennflecks zu optimieren. Durch den Winkel der geneigten Oberfläche kann eine effektive Größe des Brennflecks beeinflusst werden, was sich auf die Bildqualität auswirken kann. Durch den Winkel kann der Brennfleck auf der Drehanode aus Sicht des Strahlenaustrittsfensters verkleinert erscheinen, was zu einer verbesserten Bildqualität beitragen kann.

Der Einwinkelteller kann aus einem hochschmelzenden Material gefertigt sein, das eine hohe Temperaturbeständigkeit und gute Wärmeleitfähigkeit aufweist. Mögliche Materialien umfassen beispielsweise Wolfram, Molybdän oder Legierungen dieser Metalle, beispielsweise eine Wolfram-Rhenium-Legierung. Die geneigte Oberfläche des Einwinkeltellers kann zusätzlich mit einer dünnen Schicht aus einem Material mit hoher Ordnungszahl, wie zum Beispiel reinem Wolfram, beschichtet sein, um eine effiziente Röntgenstrahlungserzeugung zu ermöglichen.

In einer weiteren vorteilhaften Ausführungsform der vorgeschlagenen Röntgeneinheit kann der Einwinkelteller einen Winkel von 7 bis 20°, insbesondere 16°, aufweisen.

Vorteilhafterweise kann der Einwinkelteller, insbesondere die geneigte Oberfläche, einen Winkel, insbesondere einen Anodenwinkel, von 7 bis 20°, insbesondere 16°, aufweisen, insbesondere bezüglich der Rotationsebene des Einwinkeltellers. Dieser Anodenwinkel kann dazu beitragen, die Röntgenstrahlung effektiv auf das Strahlenaustrittsfenster zu fokussieren, insbesondere auszurichten, und gleichzeitig eine gleichmäßige Verteilung der Wärme auf der Oberfläche des Einwinkeltellers zu gewährleisten. Die auf den Anodenteller auftreffenden Elektronen können ihre Energie auf einer größeren Fläche abgeben, was zu einer verbesserten Wärmeverteilung führen kann.

Der Anodenwinkel von 7 bis 20°, insbesondere 16°, kann vorteilhaft sein, um eine optimale Verteilung der Röntgenstrahlung auf dem Strahlenaustrittsfenster zu erreichen. Ferner kann der Anodenwinkel von 7 bis 20°, insbesondere 16°, vorteilhaft dazu beitragen, einen Heel-Effekt zu minimieren, der zu einer ungleichmäßigen Intensitätsverteilung der Röntgenstrahlung führen kann. Der Heel-Effekt beschreibt eine Intensitätsabnahme der Röntgenstrahlung am anodenseitigen Rand des Strahlenfeldes. Darüber hinaus kann der Anodenwinkel von 7 bis 20°, insbesondere 16°, eine effiziente Nutzung des Brennflecks ermöglichen, was zu einer verbesserten Bildqualität beitragen kann. Durch den Anodenwinkel von 7 bis 20°, insbesondere 16°, kann der Brennfleck auf dem Anodenteller in Richtung des Strahlenaustrittsfensters geneigt sein. Dies kann dazu führen, dass die vom Brennfleck ausgehende Röntgenstrahlung effizienter durch das Strahlenaustrittsfenster austreten kann.

In einer weiteren vorteilhaften Ausführungsform der vorgeschlagenen Röntgeneinheit kann der Anodenteller einen Durchmesser von 50 mm bis 200 mm, insbesondere 90 mm, aufweisen.

Der Anodenteller kann einen Durchmesser von 50 mm bis 200 mm, insbesondere 90 mm, aufweisen. Der Durchmesser des Anodentellers kann als der maximale Abstand zwischen zwei Punkten auf einem äußeren Rand des Anodentellers definiert sein. Ein Anodenteller mit einem Durchmesser von 50 mm bis 200 mm, insbesondere 90 mm, kann eine ausgewogene Lösung darstellen, die eine effiziente Röntgenstrahlungserzeugung bei gleichzeitig kompakter Bauweise der Röntgeneinheit ermöglicht.

In einer weiteren vorteilhaften Ausführungsform der vorgeschlagenen Röntgeneinheit kann der Anodenteller ein Trägersubstrat umfassen. Auf einer der Kathode zugewandten Seite kann eine Schicht aus einem weiteren Substrat aufgebracht sein.

Der Anodenteller kann ein Trägersubstrat umfassen. Das Trägersubstrat kann als Grundkörper oder Basisstruktur des Anodentellers ausgebildet sein. Das Trägersubstrat kann aus einem Material mit hoher mechanischer Festigkeit und guter Wärmeleitfähigkeit gefertigt sein, um die bei der Röntgenstrahlungserzeugung entstehende Wärme effektiv abzuleiten. Das Trägersubstrat kann beispielsweise eine Metalllegierung oder einen Verbundwerkstoff umfassen.

Auf einer der Kathode zugewandten Seite des Trägersubstrats kann eine Schicht aus dem weiteren Substrat aufgebracht sein. Diese Schicht kann als Targetschicht oder aktive Schicht des Anodentellers ausgebildet sein, auf der der Elektronenstrahl auftrifft und die Röntgenstrahlung erzeugt wird. Das weitere Substrat kann aus einem Material mit hoher Ordnungszahl gefertigt sein, um eine effiziente Röntgenstrahlungserzeugung zu ermöglichen. Die Schicht aus dem weiteren Substrat kann mittels verschiedener Beschichtungsverfahren wie Aufdampfen, Sputtern und/oder galvanische Abscheidung auf das Trägersubstrat aufgebracht worden sein.

Die Targetschicht kann bei einer Ausbildung des Anodentellers als Einwinkelteller an, insbesondere auf, der geneigten Oberfläche angeordnet sein. Dabei kann die Targetschicht ring- oder streifenförmig ausgeführt sein. Diese Konfiguration kann eine optimierte Verteilung des weiteren Substrats auf der Anodenoberfläche ermöglichen. Die ring- oder streifenförmige Anordnung der Targetschicht kann dazu beitragen, die thermische Belastung gleichmäßiger zu verteilen und die Effizienz der Röntgenerzeugung zu verbessern.

Die Anordnung eines Trägersubstrats mit einer darauf aufgebrachten Schicht aus einem weiteren Substrat kann vorteilhaft sein, da sie eine Kombination der positiven Eigenschaften beider Materialien ermöglicht. Das Trägersubstrat kann für mechanische Stabilität und Wärmeableitung sorgen, während die Schicht aus dem weiteren Substrat verbesserte Eigenschaften für die Röntgenstrahlungserzeugung aufweisen kann.

In einer weiteren vorteilhaften Ausführungsform der vorgeschlagenen Röntgeneinheit kann das Trägersubstrat Titan-Zirkonium-Molybdän umfassen.

Titan-Zirkonium-Molybdän kann als eine Legierung ausgebildet sein, die Titan, Zirkonium und Molybdän enthält. Titan-Zirkonium-Molybdän kann vorteilhafte Eigenschaften für die Verwendung als Trägersubstrat eines Anodentellers aufweisen. Diese Legierung kann eine hohe Wärmeleitfähigkeit, gute mechanische Stabilität und Beständigkeit gegen thermische Verformung bei hohen Temperaturen aufweisen. Zudem kann Titan-Zirkonium-Molybdän eine gute Haftung für weitere Beschichtungen ermöglichen. Die Verwendung von Titan-Zirkonium-Molybdän als Trägersubstrat kann die Lebensdauer und Leistungsfähigkeit des Anodentellers verbessern. Das Trägersubstrat kann dazu ausgebildet sein, eine mechanische Stabilität des Anodentellers und eine geringe thermische Ausdehnung zu gewährleisten und eine effiziente Wärmeableitung zu ermöglichen.

Die gute mechanische Stabilität der Titan-Zirkonium-Molybdän-Legierung kann eine hohe Belastbarkeit des Anodentellers bei den auftretenden Rotationsgeschwindigkeiten und thermischen Belastungen ermöglichen. Dies kann dazu beitragen, Verformungen und/oder Beschädigungen des Anodentellers während des Betriebs zu minimieren.

Die geringe thermische Ausdehnung der Titan-Zirkonium-Molybdän-Legierung kann thermische Spannungen im Anodenteller reduzieren. Dies kann dazu beitragen, Rissbildungen und/oder Verformungen des Anodentellers aufgrund von Temperaturänderungen zu verhindern.

Zusätzlich kann die Titan-Zirkonium-Molybdän-Legierung eine gute Beständigkeit gegen Oxidation und Korrosion aufweisen. Dies kann eine Langzeitstabilität des Anodentellers unter den Betriebsbedingungen in der Röntgenröhre verbessern.

Die Verwendung von Titan-Zirkonium-Molybdän als Trägersubstrat kann somit verschiedene vorteilhafte Eigenschaften für den Anodenteller ermöglichen. Diese Eigenschaften können zu einer verbesserten Leistungsfähigkeit, Zuverlässigkeit und Lebensdauer der Röntgenröhre beitragen.

In einer weiteren vorteilhaften Ausführungsform der vorgeschlagenen Röntgeneinheit kann das weitere Substrat eine Wolfram-Rhenium-Legierung umfassen.

Die Wolfram-Rhenium-Legierung kann als Beschichtungsmaterial für den Anodenteller, insbesondere die Targetschicht des Anodentellers, der Röntgenröhre verwendet werden. Die Wolfram-Rhenium-Legierung kann beispielsweise einen Rheniumanteil von bis zu 30 % aufweisen, insbesondere zwischen 5 und 12,5 %. Der Rheniumanteil kann vorteilhafterweise die thermische und mechanische Belastbarkeit der Beschichtung erhöhen. Ferner kann der Rhenium-Zusatz eine Duktilität und Zähigkeit der Legierung im Vergleich zu reinem Wolfram erhöhen. Die Wolfram-Rhenium-Legierung kann als dünne Targetschicht auf das Trägersubstrat des Anodentellers aufgebracht worden sein, beispielsweise mittels Plasmaspritzen, Sputtern und/oder chemischer Gasphasenabscheidung. Eine Dicke der Targetschicht kann zwischen 5 µm und 1000 µm betragen. Die Wolfram-Rhenium-Legierung kann aufgrund ihrer hohen Schmelztemperatur, guten Wärmeleitfähigkeit und hohen Dichte als Targetschicht für die Erzeugung von Röntgenstrahlung dienen. Durch die Verwendung einer Wolfram-Rhenium-Legierung als weiteres Substrat kann die Lebensdauer und Leistungsfähigkeit des Anodentellers verbessert werden.

Die erhöhte Zähigkeit der Wolfram-Rhenium-Legierung im Vergleich zu reinem Wolfram kann eine Lebensdauer des Anodentellers verlängern, da Rissbildung und Abplatzen der Beschichtung reduziert werden können. Die gute Wärmeleitfähigkeit der Wolfram-Rhenium-Legierung kann zu einer effizienteren Wärmeableitung vom Brennfleck beitragen, was höhere Leistungsdichten ermöglichen kann. Die hohe Dichte der Wolfram-Rhenium-Legierung kann eine gute Röntgenstrahlungsausbeute gewährleisten.

In einer weiteren vorteilhaften Ausführungsform der vorgeschlagenen Röntgeneinheit können die Kathode und der Anodenteller einen mittleren Abstand von 5 mm bis 50 mm, insbesondere 8 mm, aufweisen.

Der mittlere Abstand kann als ein durchschnittlicher oder typischer Abstand zwischen der Kathode und dem Anodenteller verstanden werden. Dieser Abstand kann vorteilhafterweise so gewählt sein, dass er eine optimale Fokussierung des Elektronenstrahls auf den Anodenteller ermöglicht. Dieser Abstand ermöglicht vorteilhafterweise eine effiziente Beschleunigung der Elektronen von der Kathode zum Anodenteller, während gleichzeitig ein ausreichender Raum für die Ablenkung des Elektronenstrahls durch das Magnetfeld der Spule bereitgestellt wird.

Der mittlere Abstand von 5 mm bis 50 mm, insbesondere 8 mm, kann verschiedene technische Vorteile bieten. Zum einen kann dieser Abstand eine ausreichende elektrische Isolierung zwischen Kathode und Drehanode, insbesondere dem Anodenteller, gewährleisten, um Spannungsüberschläge zu vermeiden. Zum anderen kann dieser Abstand eine effiziente Fokussierung des Elektronenstrahls auf den Anodenteller ermöglichen, was zu einer verbesserten Bildqualität beitragen kann.

Die Wahl des mittleren Abstands kann auch Einfluss auf eine Größe und Geometrie des Brennflecks haben. Ein geeigneter Abstand kann dazu beitragen, einen möglichst kleinen und scharf definierten Brennfleck zu erzeugen, was beispielsweise für eine hohe räumliche Auflösung von erzeugbaren Bilddaten, insbesondere Röntgenbilddaten, vorteilhaft sein kann.

Ferner kann der mittlere Abstand von 5 mm bis 50 mm, insbesondere 8 mm, in Zusammenhang mit anderen Parametern der Röntgenröhre stehen, wie beispielsweise der angelegten Hochspannung, einer Geometrie der Kathode und/oder des Anodentellers sowie einer Stärke des zur Ablenkung des Elektronenstrahls erzeugten Magnetfelds. Diese Parameter können aufeinander abgestimmt sein, um eine optimale Leistung der Röntgenröhre zu erzielen.

Der mittlere Abstand von 5 mm bis 50 mm, insbesondere 8 mm, kann durch verschiedene konstruktive Maßnahmen in der Röntgenröhre realisiert sein. Beispielsweise kann die Kathode auf einer entsprechenden Halterung innerhalb des Vakuumgehäuses montiert sein, die den gewünschten Abstand zum Anodenteller gewährleistet. Ebenso kann eine Lagerung des Anodentellers so ausgelegt sein, dass der erforderliche Abstand zur Kathode eingehalten wird.

Es ist zu beachten, dass der tatsächliche Abstand zwischen Kathode und Anodenteller an verschiedenen Punkten leicht variieren kann, beispielsweise aufgrund der Rotation des Anodentellers oder thermischer Ausdehnung während des Betriebs. Der angegebene mittlere Abstand von 5 mm bis 50 mm, insbesondere 8 mm, stellt daher einen Durchschnittswert dar, der für die Auslegung und Funktion der Röntgenröhre maßgeblich ist.

Die Einhaltung dieses spezifischen Abstands kann durch verschiedene Komponenten und Mechanismen innerhalb der Röntgenröhre sichergestellt werden, beispielsweise präzise Positionierungselemente, Abstandshalter und/oder spezielle Montagetechniken für Kathode und Anodenteller.

Vorteilhafterweise kann der mittlere Abstand von 5 mm bis 50 mm, insbesondere 8 mm, zwischen Kathode und Anodenteller zu einer verbesserten Effizienz der Röntgenstrahlenerzeugung beitragen. Dieser optimierte Abstand kann eine präzise Steuerung des Elektronenstrahls ermöglichen, was wiederum zu einer höheren Bildqualität bei gleichzeitig reduzierter Strahlenbelastung für das Untersuchungsobjekt führen kann. Zudem kann dieser spezifische Abstand dazu beitragen, die Wärmeentwicklung in der Röntgenröhre zu verringern und somit die Lebensdauer der Komponenten zu verlängern.

In einer weiteren vorteilhaften Ausführungsform der vorgeschlagenen Röntgeneinheit kann das Vakuumgehäuse jeweilige Durchleitungen zur Bestromung der Kathode und der Drehanode aufweisen.

Eine Durchleitung kann als eine elektrisch leitfähige Verbindung ausgebildet sein, die durch das Vakuumgehäuse hindurchgeführt ist. Die Durchleitungen können dazu ausgebildet sein, eine elektrische Verbindung zwischen Komponenten innerhalb des Vakuumgehäuses und einer externen Stromversorgung, insbesondere einer jeweiligen externen Stromversorgung, herzustellen. Insbesondere können die Durchleitungen eine elektrische Kontaktierung der Kathode und der Drehanode von außerhalb des Vakuumgehäuses ermöglichen.

Die Durchleitungen zur Bestromung der Kathode können dazu ausgebildet sein, einen Heizstrom für die Kathode bereitzustellen. Dieser Heizstrom kann die Kathode auf eine Temperatur erhitzen, bei der eine Elektronenemission ermöglicht wird.

Die Durchleitungen zur Bestromung der Drehanode können dazu ausgebildet sein, eine Hochspannung an die Drehanode anzulegen, insbesondere die Drehanode auf ein positives elektrisches Potenzial gegenüber der Kathode zu bringen. Diese Hochspannung kann eine Beschleunigung der von der Kathode emittierten Elektronen in Richtung der Drehanode bewirken.

Die Durchleitungen können beispielsweise als metallische Leiter ausgebildet sein, die durch vakuumdichte Isolatoren im Vakuumgehäuse geführt sind. Die Leiter können beispielsweise Kupfer, Silber oder andere gut leitfähige Metalle umfassen. Die Leiter können eine ausreichende Dicke aufweisen, um den erforderlichen Strom ohne übermäßige Erwärmung zu leiten. Die Isolatoren können aus Keramik, Glas oder anderen vakuumtauglichen Isoliermaterialien bestehen.

Die Durchleitungen können so dimensioniert und angeordnet sein, dass sie den hohen Spannungen und Strömen in einer Röntgenröhre standhalten. Zudem können sie so ausgelegt sein, dass sie die Vakuumdichtigkeit des Gehäuses nicht beeinträchtigen.

Durch die Verwendung separater Durchleitungen für Kathode und Drehanode kann eine unabhängige Steuerung und Versorgung dieser Komponenten ermöglicht werden. Dies kann zu einer verbesserten Kontrolle und Stabilität des Elektronenstrahls und damit der Röntgenstrahlungserzeugung beitragen.

Die Durchleitungen können innerhalb des Vakuumgehäuses mit entsprechenden Anschlussstellen an der Kathode und der Drehanode verbunden sein. Außerhalb des Vakuumgehäuses können die Durchleitungen mit einer jeweiligen externen Stromversorgung verbindbar sein.

Die Verwendung von Durchleitungen zur Bestromung der Kathode und der Drehanode kann mehrere technische Vorteile bieten. Zum einen ermöglicht sie eine zuverlässige und effiziente Energieversorgung der Komponenten innerhalb des Vakuumgehäuses. Zum anderen kann durch die vakuumdichte Ausführung der Durchleitungen die Integrität des Vakuums im Inneren des Gehäuses gewährleistet werden. Dies kann zu einer verbesserten Lebensdauer und Leistungsfähigkeit der Röntgenröhre beitragen.

Vorteilhafterweise können die Durchleitungen, insbesondere zumindest die Durchleitungen zur Bestromung der Kathode, an einer kathodenseitigen Wand des Vakuumgehäuses angeordnet sein. Diese Positionierung kann vorteilhaft sein, da sie einen kurzen Leitungsweg zur Kathode ermöglicht und gleichzeitig genügend Abstand zur Drehanode bietet, um elektrische Überschläge zu vermeiden. Ferner können die Durchleitungen zur Bestromung der Anode anodenseitig bezüglich des Vakuumgehäuses angeordnet sein.

Der Abstand zwischen benachbarten Durchleitungen kann so gewählt sein, dass er mindestens dem 1,5-fachen des Durchmessers einer einzelnen Durchleitung entspricht. Dieser Mindestabstand kann dazu beitragen, elektrische Überschläge zwischen den Durchleitungen zu verhindern.

In einer weiteren vorteilhaften Ausführungsform der vorgeschlagenen Röntgeneinheit kann das Vakuumgehäuse aus einem Substrat gebildet werden, das eine Glas-Keramik umfasst.

Eine Glas-Keramik kann als ein Material definiert sein, das sowohl glasartige als auch kristalline Eigenschaften aufweist. Glas-Keramiken können durch kontrollierte Kristallisation von Gläsern hergestellt werden. Das Substrat aus Glas-Keramik kann vorteilhafte Eigenschaften wie hohe mechanische Festigkeit, geringe thermische Ausdehnung und gute elektrische Isolationseigenschaften aufweisen. Diese Eigenschaften können für die Anwendung als Vakuumgehäuse einer Röntgenröhre vorteilhaft sein.

Beispiele für Glas-Keramiken, die als Substrat für das Vakuumgehäuse verwendet werden können, umfassen Lithiumalumosilikat-Glas-Keramiken, Magnesiumalumosilikat-Glas-Keramiken, Borosilikatglas und/oder Zinkalumosilikat-Glas-Keramiken. Eine genaue Zusammensetzung der Glas-Keramik kann an die spezifischen Anforderungen der Röntgenröhre angepasst sein.

Das Vakuumgehäuse aus dem Substrat umfassend eine Glas-Keramik kann durch verschiedene Verfahren hergestellt worden sein. Beispielsweise kann zunächst ein Glasrohling geformt und anschließend einer kontrollierten Wärmebehandlung unterzogen werden, um eine teilweise Kristallisation zu erreichen. Alternativ kann das Vakuumgehäuse auch durch Sintern von Glas-Keramik-Pulvern hergestellt worden sein.

Die Verwendung eines Substrats aus Glas-Keramik für das Vakuumgehäuse kann im Vergleich zu herkömmlichen Materialien wie Metallen oder reinen Keramiken mehrere Vorteile bieten. Die Glas-Keramik kann eine verbesserte Vakuumdichtigkeit ermöglichen und gleichzeitig eine gute elektrische Isolation zwischen den Komponenten im Inneren der Röntgenröhre gewährleisten. Darüber hinaus kann die geringe thermische Ausdehnung der Glas-Keramik dazu beitragen, thermische Spannungen im Vakuumgehäuse zu reduzieren, was eine Lebensdauer und Zuverlässigkeit der Röntgenröhre erhöhen kann. Die Glas-Keramik kann auch eine hohe Beständigkeit gegenüber Röntgenstrahlung aufweisen, was die Lebensdauer des Vakuumgehäuses verlängern kann. Ein weiterer Vorteil der Verwendung von Glas-Keramik als Substrat für das Vakuumgehäuse kann die Möglichkeit sein, komplexe Formen und Strukturen herzustellen. Dies kann die Integration von Durchführungen für elektrische Anschlüsse, insbesondere Durchleitungen, und/oder Kühlkanäle erleichtern.

Zusammenfassend kann die Verwendung eines Vakuumgehäuses aus einem Substrat, das eine Glas-Keramik umfasst, zu einer verbesserten Leistungsfähigkeit, Zuverlässigkeit und Langlebigkeit der Röntgenröhre beitragen.

In einer weiteren vorteilhaften Ausführungsform der vorgeschlagenen Röntgeneinheit kann die Röntgeneinheit weiterhin ein leckdichtes Eintankgehäuse umfassen. Die Röntgenröhre und die Spule können innerhalb des Eintankgehäuses angeordnet sein. Das Eintankgehäuse kann mit einem Kühl-Isoliermedium gefüllt sein.

Das leckdichte Eintankgehäuse kann als ein geschlossenes Gehäuse ausgebildet sein, welches dazu konfiguriert ist, ein Austreten von Flüssigkeiten oder Gasen zu verhindern. Das leckdichte Eintankgehäuse kann aus verschiedenen Materialien hergestellt sein, beispielsweise aus Metall, insbesondere Aluminium oder Edelstahl, Kunststoff, einem Verbundwerkstoff oder einer Kombination davon. Das Eintankgehäuse kann verschiedene Dichtungselemente wie O-Ringe und/oder Flachdichtungen aufweisen, um die Leckdichtheit sicherzustellen. Das leckdichte Eintankgehäuse kann zusätzlich mit einem strahlungsabsorbierenden Material ausgekleidet sein. Das strahlungsabsorbierende Material kann Blei oder einen Verbundwerkstoff, umfassend einen Kunststoff und ein Metall, umfassen.

Die Röntgenröhre und die Spule können innerhalb des Eintankgehäuses angeordnet sein. Dies bedeutet, dass sowohl die Röntgenröhre als auch die Spule vollständig von dem Eintankgehäuse umschlossen sein können. Die Anordnung innerhalb des Eintankgehäuses kann es vorteilhaft ermöglichen, die Röntgenröhre und die Spule vor äußeren Einflüssen zu schützen und eine kontrollierte Umgebung für deren Betrieb bereitzustellen.

Das Eintankgehäuse kann mit einem Kühl-Isoliermedium gefüllt sein. Ein Kühl-Isoliermedium kann als eine Substanz definiert sein, die sowohl kühlende als auch isolierende Eigenschaften aufweist. Das Kühl-Isoliermedium kann eine Flüssigkeit, ein Gas oder eine Mischung davon umfassen. Beispiele für mögliche Kühl-Isoliermedien umfassen Öle, Gase, insbesondere Schwefelhexafluorid, oder spezielle dielektrische Flüssigkeiten.

Das Kühl-Isoliermedium kann dazu ausgebildet sein, Wärme von den Komponenten im Inneren des Eintankgehäuses aufzunehmen und abzuführen, um eine Überhitzung zu verhindern. Das Kühl-Isoliermedium kann ferner als elektrischer Isolator ausgebildet sein, um Spannungsüberschläge zwischen den Komponenten zu verhindern. Das Kühl-Isoliermedium kann zudem korrosionshemmende Eigenschaften aufweisen und so die Lebensdauer der Komponenten verlängern. Des Weiteren kann das Kühl-Isoliermedium dazu ausgebildet sein, eine Wärmeübertragung zwischen den Komponenten und dem Eintankgehäuse zu verbessern.

Die Verwendung eines leckdichten Eintankgehäuses, in dem die Röntgenröhre und die Spule angeordnet sind und das mit einem Kühl-Isoliermedium gefüllt ist, kann zu einer kompakten und effizienten Bauweise der Röntgeneinheit beitragen. Diese Konfiguration kann eine verbesserte Kühlung, elektrische Isolation und einen Schutz der Komponenten ermöglichen, was zu einer erhöhten Leistungsfähigkeit und Zuverlässigkeit der Röntgeneinheit führen kann.

Das Eintankgehäuse kann beispielsweise eine zylindrische Grundform aufweisen. An einer Außenseite kann das Eintankgehäuse Kühlrippen aufweisen, insbesondere können an der Außenseite des Eintankgehäuses Kühlrippen angebracht sein, die zur Wärmeabfuhr ausgebildet sind. Für eine Befüllung und Entleerung des Eintankgehäuses mit dem Kühl-Isoliermedium kann das Eintankgehäuse Anschlüsse aufweisen. Diese Anschlüsse können sich beispielsweise an einem oberen und unteren Ende des Eintankgehäuses befinden, um eine vollständige Befüllung und Entleerung zu ermöglichen.

Das Eintankgehäuse kann ferner Vorrichtungen zur Montage und Ausrichtung der internen Komponenten aufweisen, beispielsweise Halterungen und/oder Schienen, welche eine präzise Positionierung der Röntgenröhre und der Spule ermöglichen.

Zur Überwachung der Betriebsparameter kann das Eintankgehäuse Sensoren umfassen. Die Sensoren können dazu ausgebildet sein, eine Temperatur, einen Druck und/oder einen Füllstand des Kühl-Isoliermediums zu erfassen. Die Sensoren können an verschiedenen Stellen im Eintankgehäuse angeordnet sein, um eine umfassende Überwachung zu gewährleisten.

In einer weiteren vorteilhaften Ausführungsform der vorgeschlagenen Röntgeneinheit kann die Röntgeneinheit weiterhin eine Hochspannungseinheit umfassen. Die Hochspannungseinheit kann dazu ausgebildet sein, eine Hochspannung an die Röntgenröhre bereitzustellen. Die Hochspannungseinheit kann zusätzlich innerhalb des Eintankgehäuses angeordnet sein.

Die Hochspannungseinheit kann beispielsweise einen Hochspannungsgenerator umfassen, der dazu ausgebildet ist, eine Hochspannung von mehreren Kilovolt bis zu mehreren hundert Kilovolt zu erzeugen, beispielsweise im Bereich von 10 kV bis 300 kV. Die Hochspannungseinheit kann ferner eine Steuerelektronik umfassen, die dazu ausgebildet ist, die erzeugte Hochspannung zu regulieren und an die Röntgenröhre bereitzustellen.

Die Hochspannungseinheit kann zusätzlich innerhalb des Eintankgehäuses angeordnet sein. Durch die Anordnung der Hochspannungseinheit innerhalb des Eintankgehäuses kann eine kompakte Bauweise der Röntgeneinheit erreicht werden. Ferner kann die Hochspannungseinheit durch das Eintankgehäuse vor äußeren Einflüssen geschützt sein. Zudem kann das Kühl-Isoliermedium die Hochspannungseinheit im Eintankgehäuse kühlen und elektrisch isolieren.

Die Anordnung der Hochspannungseinheit innerhalb des Eintankgehäuses kann verschiedene Vorteile bieten. Zum einen kann dadurch eine bessere elektromagnetische Abschirmung erreicht werden, da das Eintankgehäuse als Faradayscher Käfig wirken kann. Zum anderen kann die räumliche Nähe zur Röntgenröhre kürzere Hochspannungsleitungen ermöglichen, was Verluste reduzieren und eine Effizienz erhöhen kann.

In einer weiteren vorteilhaften Ausführungsform der vorgeschlagenen Röntgeneinheit kann das Eintankgehäuse jeweilige Durchleitungen zum Bereitstellen einer Bestromung der Hochspannungseinheit und der Spule aufweisen.

Die Durchleitungen können als elektrisch isolierte Durchführungen durch das Eintankgehäuse ausgebildet sein. Die Durchleitungen können beispielsweise als Kabeldurchführungen, Kabeleinführungen und/oder Kabelverschraubungen ausgebildet sein.

Das Bereitstellen der Bestromung der Hochspannungseinheit beziehungsweise der Spule kann das Zuführen von elektrischem Strom zu der Hochspannungseinheit beziehungsweise der Spule umfassen. Die Bestromung kann beispielsweise durch Anschließen an eine Stromquelle oder durch Aktivieren eines Schalters erfolgen.

Das Eintankgehäuse mit den jeweiligen Durchleitungen kann dazu ausgebildet sein, eine sichere und effiziente Stromversorgung der Hochspannungseinheit und der Spule zu ermöglichen, während gleichzeitig eine Abdichtung des Gehäuses gewährleistet wird. Die Durchleitungen können so ausgelegt sein, dass sie den spezifischen Anforderungen an Spannung, Strom und Isolierung für die Hochspannungseinheit und die Spule entsprechen.

Die Verwendung von Durchleitungen zum Bereitstellen der Bestromung kann den Vorteil haben, dass eine sichere und zuverlässige elektrische Verbindung zwischen den externen Stromquellen und den internen Komponenten der Röntgeneinheit hergestellt werden kann, ohne die Integrität des Eintankgehäuses zu beeinträchtigen.

Für die Bestromung der Hochspannungseinheit können die Durchleitungen beispielsweise Kabel umfassen, die für die entsprechenden hohen Spannungen ausgelegt sind. Diese Kabel können durch spezielle Durchführungen, insbesondere Kabeldurchführungen, im Eintankgehäuse geführt sein.

Für die Bestromung der Spule können die Durchleitungen Stromkabel mit geeignetem Querschnitt umfassen, um die erforderlichen Stromstärken für die Erzeugung des Magnetfelds zu übertragen. Diese Kabel können durch separate Durchführungen im Eintankgehäuse geführt werden.

Die Durchleitungen können so angeordnet und ausgeführt sein, dass sie eine galvanische Trennung zwischen der Hochspannungseinheit, der Spule und dem Eintankgehäuse gewährleisten. Dies kann durch geeignete Isolationsmaterialien und Abstände erreicht werden.

Zusätzlich zu den Durchleitungen für die elektrische Versorgung können weitere Durchführungen für Steuer- und/oder Messleitungen vorgesehen sein, beispielsweise zur Überwachung und Regelung der Komponenten im Inneren des Eintankgehäuses.

Die separaten Durchleitungen für die Hochspannungseinheit und die Spule können vorteilhaft mögliche Störeinflüsse zwischen den verschiedenen elektrischen Systemen minimieren. Dadurch kann eine präzise Steuerung und Regelung sowohl der Hochspannung für die Röntgenröhre als auch des Magnetfelds für die Elektronenstrahlablenkung ermöglicht werden.

In einer weiteren vorteilhaften Ausführungsform der vorgeschlagenen Röntgeneinheit kann die Röntgeneinheit weiterhin eine Blendeneinheit, insbesondere eine Kollimatoreinheit, umfassen, welche zur räumlichen Begrenzung der durch das Strahlenaustrittsfenster austretenden Röntgenstrahlung ausgebildet ist.

Die Blendeneinheit kann bewegliche Blenden, insbesondere Blendenlamellen, umfassen, welche dazu ausgebildet sind, einen Querschnitt der austretenden Röntgenstrahlung zu begrenzen und anzupassen. Insbesondere kann die Blendeneinheit zur Kollimation der austretenden Röntgenstrahlung ausgebildet sein. Die Blenden können aus einem strahlungsabsorbierenden Material, beispielsweise Blei, Stahl oder Wolfram, gefertigt sein.

Die Blendeneinheit kann motorisch verstellbar ausgebildet sein, um eine Anpassung der Strahlenbegrenzung während eines Betriebs der Röntgeneinheit zu ermöglichen. Die Steuerung der Blendeneinheit kann dabei durch eine Steuerungseinheit der Röntgeneinheit oder eines Bildgebungsgeräts umfassend die Röntgeneinheit erfolgen. Die Blendeneinheit kann bezüglich einer Richtung der Röntgenstrahlung hinter dem Strahlenaustrittsfenster angeordnet sein, insbesondere zwischen dem Strahlenaustrittsfenster und einem abzubildenden Untersuchungsobjekt.

Die Blendeneinheit kann in ihrer Größe so dimensioniert sein, dass sie den gesamten Querschnitt des Röntgenstrahls, insbesondere der Röntgenstrahlung, abdeckt.

Die räumliche Begrenzung der Röntgenstrahlung durch die Blendeneinheit kann vorteilhaft eine Strahlenexposition des Untersuchungsobjekts auf einen relevanten Bereich begrenzen und einen Bildkontrast verbessern, indem Streustrahlung reduziert wird.

In einer weiteren vorteilhaften Ausführungsform der vorgeschlagenen Röntgeneinheit kann die Anodenachse einen vorgegebenen Winkel, insbesondere 6°, bezüglich einer Blendenebene der Blendeneinheit aufweisen.

Die Blendenebene kann eine Ebene bezeichnen, in der sich die Blendeneinheit befindet, insbesondere in der die Blenden oder Blendenlamellen der Blendeneinheit angeordnet und/oder verfahrbar sind, oder auf die sich die Blendeneinheit bezieht. Insbesondere kann die Blendenebene eine Querschnittsebene eines Öffnungsbereichs der Blendeneinheit bezeichnen.

Der vorgegebene Winkel zwischen der Anodenachse und der Blendenebene kann eine verbesserte Ausrichtung der Röntgenstrahlung relativ zur Blendeneinheit ermöglichen. Dabei kann der vorgegebene Winkel vorteilhafterweise derart vorgegeben werden, dass die Anodenachse bezüglich der Blendenebene kathodenseitig fallend und anodenseitig steigend angeordnet ist. Der vorgegebene Winkel kann es ermöglichen, die Strahlgeometrie für spezifische Anwendungen zu optimieren, wie beispielsweise für eine Mammographie oder andere spezialisierte radiologische Untersuchungen.

Der vorgegebene Winkel von 6° kann als ein Beispiel für einen vorteilhaften Winkel verstanden werden. Andere Winkel zwischen 0 und 30° können ebenfalls verwendet werden, abhängig von den spezifischen Anforderungen und der Geometrie der Röntgeneinheit. Der vorgegebene Winkel zwischen der Anodenachse und der Blendenebene kann dazu dienen, eine optimierte Ausrichtung der Röntgenstrahlung zu ermöglichen. Durch diese Anordnung kann eine verbesserte Strahlführung und/oder -fokussierung erreicht werden, was zu einer erhöhten Bildqualität und einer potenziell reduzierten Strahlenbelastung für das Untersuchungsobjekt führen kann. Zudem kann diese Anordnung dazu beitragen, eine Effizienz der Röntgeneinheit zu steigern, indem sie eine optimale Nutzung der erzeugten Röntgenstrahlung ermöglicht.

Der vorgegebene Winkel zwischen 0 und 30°, insbesondere 6°, zwischen der Anodenachse und der Blendenebene kann insbesondere bei einer Verwendung der Röntgeneinheit bei einer Mammographieuntersuchung vorteilhaft sein. Die Mammographie ist ein bildgebendes Verfahren zur Untersuchung der weiblichen Brust mittels Röntgenstrahlung. Sie kann eine Früherkennung von Brustkrebs und anderen Veränderungen im Brustgewebe ermöglichen. Der vorgegebene Winkel, insbesondere der 6-Grad-Winkel, zwischen der Anodenachse und der Blendenebene kann bei der Mammographie eine gleichmäßiger Intensitätsverteilung der Röntgenstrahlung, insbesondere auf einer brustwandnahen Seite, einen verkürzten Abstand zwischen dem Brennfleck und der Brustwand des Untersuchungsobjekts und eine verbesserte Strahlgeometrie der Röntgenstrahlung ermöglichen. Die Brustwand bezieht sich auf einen Bereich des Untersuchungsobjekts, wo die Brust an einem Brustkorb ansetzt. Vorteilhafterweise kann im Rahmen eines Mammographieverfahrens eine möglichst vollständige Abbildung der Brust des Untersuchungsobjekts bis zur Brustwand ermöglicht werden. Durch die Neigung der Anodenachse bezüglich der Blendenebene um den vorgegebenen Winkel von 6° kann ein Abstand zwischen dem Brennfleck und der Brustwand um etwa 10 % reduziert werden. Dies kann eine bessere Abbildung von brustwandnahem Gewebe ermöglichen. Durch die verbesserte Strahlgeometrie kann ferner eine Detailerkennbarkeit erhöht werden.

In einer weiteren vorteilhaften Ausführungsform der vorgeschlagenen Röntgeneinheit kann die Spule eine Spulengeometrie aufweisen, welche an eine Geometrie der Röntgenröhre, insbesondere des Vakuumgehäuses, angepasst ist.

Die Spulengeometrie der Spule kann eine räumliche Anordnung, Größe, Form, eine Anzahl von Windungen, insbesondere Spulenwindungen, einen Durchmesser, eine Länge und/oder eine Form eines Spulenquerschnitts der Spule umfassen, insbesondere charakterisieren. Die Geometrie der Röntgenröhre kann eine äußere Form und/oder Größe der Röntgenröhre, insbesondere des Vakuumgehäuses, umfassen, insbesondere charakterisieren.

Vorteilhafterweise kann die Spulengeometrie derart an die Geometrie der Röntgenröhre, insbesondere des Vakuumgehäuses, angepasst sein, dass die Spule eine, insbesondere gekrümmte und/oder gewölbte, Form aufweist, die einer Form und/oder Außenkontur der Röntgenröhre, insbesondere des Vakuumgehäuses, folgt. Beispielsweise kann die Spule eine zylindrische Form aufweisen, wenn die Röntgenröhre, insbesondere das Vakuumgehäuse, in dem Bereich der Anordnung der Spule eine zylindrische Form aufweist. Zum anderen kann die Größe der Spule an die Größe des Vakuumgehäuses angepasst sein. Die Spule kann vorteilhafterweise so dimensioniert sein, dass sie die Röntgenröhre, insbesondere das Vakuumgehäuse, in einem vordefinierten räumlichen Bereich möglichst eng umschließt, ohne dabei einen Betrieb der Röntgenröhre zu beeinträchtigen.

Des Weiteren kann die Anpassung der Spulengeometrie auch eine Positionierung der Spule relativ zu der Röntgenröhre, insbesondere dem Vakuumgehäuse, umfassen. Die Spule kann vorteilhafterweise derart bezüglich der Röntgenröhre, insbesondere des Vakuumgehäuses, positioniert sein, dass eine möglichst effektive Magnetfelderzeugung ermöglicht wird. Dies kann beispielsweise bedeuten, dass die Spule konzentrisch um einen bestimmten Bereich der Röntgenröhre, insbesondere des Vakuumgehäuses, angeordnet ist.

Die Anpassung der Spulengeometrie an die Geometrie der Röntgenröhre, insbesondere des Vakuumgehäuses, kann verschiedene Vorteile bieten. Die angepasste Spulengeometrie kann eine effiziente Erzeugung des Magnetfelds mittels der Spule ermöglichen, da die Spule näher an dem Bereich positioniert werden kann, in dem das Magnetfeld benötigt wird. Dies kann zu einer Verringerung des benötigten Stroms und damit zu einer Reduzierung einer Wärmeentwicklung führen. Zudem kann eine angepasste Spulengeometrie dazu beitragen, dass das erzeugte Magnetfeld homogener ist, was wiederum zu einer präziseren Ablenkung des Elektronenstrahls und damit zu einer verbesserten Steuerung der Ablenkung des Brennflecks auf dem Anodenteller führen kann.

In einer weiteren vorteilhaften Ausführungsform der vorgeschlagenen Röntgeneinheit kann die Spulengeometrie eine Querschnittsgeometrie der Spule umfassen. Die Querschnittsgeometrie der Spule kann zumindest an einer Innenseite der Spule an die Geometrie des Vakuumgehäuses innerhalb eines Öffnungsbereichs der Spule angepasst sein.

Die Querschnittsgeometrie kann sich auf eine Form und/oder Abmessungen eines Querschnitts der Spule beziehen. Der Querschnitt der Spule kann durch einen virtuellen Schnitt einer Ebene, insbesondere einer Querschnittsebene, durch eine Achse, insbesondere eine Symmetrieachse, der Spule gebildet werden. Bei einer symmetrischen, insbesondere rotationssymmetrischen, Ausbildung der Spule kann die Achse die Symmetrieachse der Spule darstellen. Alternativ oder zusätzlich kann die Achse entlang einer Normalenrichtung zu einem Öffnungsbereich, insbesondere einer Ebene des Öffnungsbereichs, der Spule angeordnet sein. Somit kann die Querschnittsebene der Spule senkrecht zu der Ebene des Öffnungsbereichs der Spule verlaufen. Die Querschnittsgeometrie der Spule kann beispielsweise kreisförmig, rechteckig, polygonal, trapezförmig, quadratisch und/oder oval ausgebildet sein.

Die Innenseite der Spule kann eine dem Vakuumgehäuse zugewandte Seite der Spule bezeichnen. Der Öffnungsbereich der Spule kann einen räumlichen Bereich innerhalb der Spulenwindungen umfassen, durch den das Vakuumgehäuse hindurchragt. Der Öffnungsbereich der Spule kann den räumlichen Bereich umfassen, in dem die Spule das Vakuumgehäuse umgibt.

Die Anpassung der Querschnittsgeometrie der Spule an der Innenseite der Spule an die Geometrie des Vakuumgehäuses innerhalb des Öffnungsbereichs der Spule kann durch verschiedene Maßnahmen erfolgen. Beispielsweise kann eine Form der Spulenwindungen, insbesondere Wicklungen, an die äußere Form des Vakuumgehäuses angepasst sein. Die Anpassung der Querschnittsgeometrie der Spule kann ferner durch verschiedene Fertigungsverfahren realisiert sein. Beispielsweise kann die Spule gewickelt, geformt und/oder gegossen worden sein, um die gewünschte Querschnittsgeometrie zu erzielen. Alternativ oder zusätzlich kann die Spule aus mehreren Segmenten zusammengesetzt sein, deren Form jeweils an die Geometrie des Vakuumgehäuses angepasst ist, insbesondere abschnittsweise. Alternativ oder zusätzlich kann die Anpassung der Querschnittsgeometrie durch eine geeignete Anordnung und Ausrichtung der Spule relativ zum Vakuumgehäuse realisiert sein. Weiterhin können eine Anzahl und/oder Anordnung der Spulenwindungen angepasst sein.

Die Anpassung der Querschnittsgeometrie an die Geometrie des Vakuumgehäuses kann eine verbesserte, insbesondere homogene, Magnetfeldverteilung im Bereich des Elektronenstrahls ermöglichen. Beispielsweise kann die Innenseite der Spule eine, insbesondere gekrümmte, Form aufweisen, die einer Außenform, insbesondere einer Außenkontur, des Vakuumgehäuses entspricht.

Die Anpassung der Querschnittsgeometrie kann eine möglichst geringe Distanz zwischen der Spule und dem Vakuumgehäuse ermöglichen. Dies kann eine erhöhte magnetische Feldstärke im Bereich des Elektronenstrahls ermöglichen. Zudem kann eine angepasste Querschnittsgeometrie eine gleichmäßigere Verteilung des Magnetfelds bewirken. Hierdurch kann eine präzisere Ablenkung des Elektronenstrahls erreicht werden, da das Magnetfeld im relevanten Bereich homogener ist.

In einer weiteren vorteilhaften Ausführungsform der vorgeschlagenen Röntgeneinheit kann das Magnetfeld dazu ausgebildet sein, den Elektronenstrahl derart abzulenken, dass der Brennfleck auf dem Anodenteller um bis zu 2,5 mm bezüglich einer, insbesondere unausgelenkten, Nullposition auslenkbar sein kann.

Die Ablenkung des Elektronenstrahls kann durch das mittels der Spule erzeugbare Magnetfeld erfolgen. Das Magnetfeld kann dabei so konfiguriert sein, dass es den Elektronenstrahl in einem definierten Bereich ablenken kann. Dieser definierte Bereich kann eine maximale Auslenkung des Brennflecks auf dem Anodenteller von bis zu 2,5 mm bezüglich der unausgelenkten Nullposition ermöglichen. Die Auslenkung des Brennflecks um zu 2,5 mm kann in verschiedene, insbesondere entgegengesetzte, Richtungen erfolgen, beispielsweise in einer Ebene parallel zur Oberfläche des Anodentellers. Beispielsweise kann der Brennfleck um jeweils bis zu 2,5 mm bezüglich der Nullposition entlang zwei entgegengesetzter Richtungen auslenkbar sein. Somit kann das Magnetfeld dazu ausgebildet sein, den Elektronenstrahl derart abzulenken, dass der Brennfleck auf dem Anodenteller um jeweils bis zu 2,5 mm bezüglich der Nullposition auslenkbar ist, insbesondere entlang verschiedener, insbesondere entgegengesetzter, Richtungen.

Die genaue Auslenkung des Brennflecks auf dem Anodenteller kann durch eine Stärke und/ oder Richtung des Magnetfelds gesteuert werden. Das Magnetfeld kann dabei durch die Bestromung der Spule erzeugt und variiert werden.

Die Auslenkung des Brennflecks um bis zu 2,5 mm kann eine ausreichende Flexibilität bei der Positionierung des Brennflecks bieten, ohne die Stabilität und Fokussierung des Elektronenstrahls zu beeinträchtigen. Der Wert von 2,5 mm kann als maximale Auslenkung verstanden werden, wobei kleinere Auslenkungen ebenfalls möglich sind.

In einer weiteren vorteilhaften Ausführungsform der vorgeschlagenen Röntgeneinheit kann der Elektronenstrahl in einem Betriebszustand der Röntgeneinheit zumindest teilweise innerhalb eines Öffnungsbereichs der Spule angeordnet sein.

Der Öffnungsbereich der Spule kann einen räumlichen Bereich innerhalb der Spule umfassen, durch den das von der Spule erzeugbare Magnetfeld verläuft. Der Betriebszustand der Röntgeneinheit kann einen Zustand umfassen, in dem die Röntgeneinheit zur Erzeugung von Röntgenstrahlung aktiviert ist. Die zumindest teilweise Anordnung des Elektronenstrahls innerhalb des Öffnungsbereichs der Spule kann ermöglichen, dass das Magnetfeld der Spule effektiv auf den Elektronenstrahl einwirken und diesen ablenken kann.

Die Anordnung des Elektronenstrahls zumindest teilweise innerhalb des Öffnungsbereichs der Spule kann verschiedene Vorteile bieten. Zum einen kann dadurch eine präzise Ablenkung des Elektronenstrahls ermöglicht werden, da das Magnetfeld im Öffnungsbereich der Spule besonders homogen und stark sein kann. Zum anderen kann durch die Anordnung des Elektronenstrahls im Öffnungsbereich der Spule eine kompakte Bauweise der Röntgeneinheit erreicht werden, da die Spule nah an der Röntgenröhre positioniert ist.

Der Öffnungsbereich der Spule kann verschiedene Geometrien aufweisen. Beispielsweise kann der Öffnungsbereich zylindrisch, konisch, rechteckig, elliptisch, prismatisch, kegelabschnittsförmig oder quaderförmig ausgebildet sein. Die Geometrie, insbesondere Form, des Öffnungsbereichs kann an die Geometrie der Röntgenröhre, insbesondere des Vakuumgehäuses, und eine Geometrie, insbesondere einen räumlichen Verlauf und/oder eine räumliche Verteilung und/ oder Ausdehnung, des Elektronenstrahls angepasst sein.

Die zumindest teilweise Anordnung des Elektronenstrahls im Öffnungsbereich der Spule kann bedeuten, dass ein Teil des Elektronenstrahls oder der gesamte Elektronenstrahl innerhalb des Öffnungsbereichs verläuft. Beispielsweise kann der Elektronenstrahl vollständig innerhalb des Öffnungsbereichs verlaufen oder nur ein Abschnitt des Elektronenstrahls kann innerhalb des Öffnungsbereichs angeordnet sein.

Die Anordnung des Elektronenstrahls im Öffnungsbereich der Spule kann durch eine geeignete Positionierung und Ausrichtung der Spule relativ zur Röntgenröhre realisiert werden. Dabei kann die Spule so angeordnet sein, dass ihr Öffnungsbereich den Zwischenraum zwischen Kathode und Anodenteller zumindest teilweise umschließt.

In dem Betriebszustand der Röntgeneinheit kann der Elektronenstrahl von der Kathode emittiert und in Richtung des Anodentellers beschleunigt werden. Dabei durchquert der Elektronenstrahl den Öffnungsbereich der Spule und kann durch das dort vorhandene Magnetfeld abgelenkt werden. Die Stärke und Richtung der Ablenkung können durch die Bestromung der Spule steuerbar sein.

Die zumindest teilweise Anordnung des Elektronenstrahls im Öffnungsbereich der Spule ermöglicht eine präzise und kontrollierte Ablenkung des Strahls. Dies kann genutzt werden, um den Auftreffpunkt des Elektronenstrahls auf dem Anodenteller gezielt zu steuern und somit die Position des Brennflecks zu beeinflussen.

Ein technischer Vorteil dieser Anordnung kann darin bestehen, dass eine effektive Ablenkung des Elektronenstrahls mit vergleichsweise geringen Magnetfeldstärken erreicht werden kann.

Dies kann zu einem energieeffizienten Betrieb der Röntgeneinheit beitragen und/oder eine thermische Belastung der Spule reduzieren.

In einer weiteren vorteilhaften Ausführungsform der vorgeschlagenen Röntgeneinheit kann der Brennfleck der Röntgenröhre in dem Betriebszustand der Röntgeneinheit innerhalb des Öffnungsbereichs der Spule angeordnet sein.

Durch diese Anordnung kann das von der Spule erzeugte Magnetfeld den Elektronenstrahl in dem Bereich durchdringen, in dem der Elektronenstrahl auf den Anodenteller trifft. Dies kann eine präzise Steuerung der Position des Brennflecks auf dem Anodenteller ermöglichen.

Vorteilhafterweise kann die Spule so dimensioniert und positioniert sein, dass ihr Öffnungsbereich den Bereich zwischen Kathode und Anodenteller vollständig umschließt. Alternativ kann die Spule so angeordnet sein, dass nur ein Teil des Bereichs zwischen Kathode und Anodenteller vom Öffnungsbereich der Spule umfasst wird.

Diese Anordnung kann verschiedene technische Vorteile bieten. Sie kann eine effiziente Nutzung des von der Spule erzeugten Magnetfelds ermöglichen, da das Magnetfeld direkt auf den Bereich wirkt, in dem die Ablenkung des Elektronenstrahls erfolgen soll. Dadurch kann eine präzise Steuerung der Brennfleckposition mit relativ geringen Spulenströmen erreicht werden.

In einer weiteren vorteilhaften Ausführungsform der vorgeschlagenen Röntgeneinheit kann die Spule als Luftspule ausgebildet sein.

Eine Luftspule kann als eine Spule ausgebildet sein, die keinen ferromagnetischen Kern aufweist. Die Luftspule kann aus einem elektrisch leitfähigen Material, wie beispielsweise Kupfer oder Aluminium, gewickelt sein. Die Windungen der Luftspule können um einen Luftkern, beispielsweise einen Hohlraum oder Luftspalt, oder einen Kern aus einem nicht-ferromagnetischen Material angeordnet sein.

Die Luftspule kann dazu ausgebildet sein, ein Magnetfeld zu erzeugen, wenn sie von einem elektrischen Strom durchflossen wird. Im Vergleich zu Spulen mit ferromagnetischem Kern weist eine Luftspule typischerweise eine geringere Induktivität auf. Dies kann vorteilhaft sein, um schnelle Änderungen des Magnetfelds zu ermöglichen.

Die Ausbildung der Spule als Luftspule kann vorteilhaft sein, um Sättigungseffekte zu vermeiden, die bei ferromagnetischen Kernen auftreten können. Dadurch kann eine präzise und lineare Steuerung des erzeugten Magnetfelds ermöglicht werden. Ferner kann eine Luftspule vorteilhaft auch bei hohen Frequenzen eingesetzt werden, da keine Wirbelstromverluste in einem Kernmaterial entstehen.

Die Luftspule kann in verschiedenen Formen und/oder Geometrien ausgebildet sein, beispielsweise als Zylinderspule, Toroidspule oder Flachspule. Die Geometrie und/oder Form der Luftspule kann an die jeweiligen Anforderungen und den verfügbaren Bauraum der Röntgeneinheit angepasst sein.

In einer weiteren vorteilhaften Ausführungsform der vorgeschlagenen Röntgeneinheit kann eine Windungszahl der Spule in Abhängigkeit einer vorgegebenen Maximalablenkung des Elektronenstrahls angepasst sein.

Die Windungszahl kann eine Anzahl der Windungen oder Wicklungen der Spule bezeichnen, insbesondere eine Anzahl der Windungen oder Wicklungen insgesamt oder innerhalb eines vorgegebenen räumlichen Abschnitts der Spule. Die Windungszahl der Spule kann dabei so gewählt sein, dass sie die gewünschte Maximalablenkung des Elektronenstrahls ermöglicht. Dabei können vorteilhafterweise die Maximalablenkung, eine Geometrie der Röntgenröhre, eine Feldstärke des zu erzeugenden Magnetfelds, ein räumlicher Abstand zwischen der Spule und dem Elektronenstrahl, eine maximale Stromstärke, die durch die Spule fließen kann, eine Ansprechzeit der Spule, eine thermische Belastung der Spule und/oder eine Energie des Elektronenstrahls berücksichtigt worden sein.

Die vorgegebene Maximalablenkung des Elektronenstrahls kann einen maximalen Auslenkungswinkel oder eine maximale Auslenkungsstrecke des Elektronenstrahls auf dem Anodenteller bezeichnen.

Eine Erhöhung der Windungszahl der Spule kann, insbesondere bei gleichem Strom, zu einem stärkeren Magnetfeld und damit zu einer größeren möglichen Ablenkung des Elektronenstrahls führen. Eine zu hohe Windungszahl kann auch zu einer erhöhten Induktivität der Spule führen, was eine Anstiegszeit des Magnetfeldes verlängern kann.

Eine Verringerung der Windungszahl kann, insbesondere bei gleichem Strom, zu einem schwächeren Magnetfeld und einer geringeren Ablenkung führen. Eine geringere Windungszahl kann zu einer schnelleren Ansprechzeit der Spule führen, da die Induktivität geringer ist. Dies kann für Anwendungen vorteilhaft sein, bei denen eine schnelle Änderung der Ablenkung erforderlich ist.

Zusätzlich kann eine räumliche Verteilung, insbesondere Dichte, der Windungen entlang der Spule angepasst sein, um eine verbesserte Feldgeometrie für die gewünschte Ablenkung zu erzielen. Ein Durchmesser der einzelnen Windungen kann variiert werden, um die Feldstärke des Magnetfelds lokal anzupassen.

Die Anpassung der Windungszahl kann durch Simulationen und/oder experimentelle Untersuchungen optimiert worden sein, um die gewünschte Maximalablenkung bei gleichzeitig möglichst geringem Energieverbrauch und kompakter Bauweise der Spule zu erreichen.

Durch die Anpassung der Windungszahl an die vorgegebene Maximalablenkung kann eine effiziente und präzise Steuerung des Elektronenstrahls ermöglicht werden. Dies kann zu einer verbesserten Bildqualität und Flexibilität des Röntgengeräts beitragen.

In einer weiteren vorteilhaften Ausführungsform der vorgeschlagenen Röntgeneinheit können die Spule und der Elektronenstrahl durch einen aus einem unmagnetischen metallischen Material geringer elektrischer Leitfähigkeit gebildeten Wandabschnitt des Vakuumgehäuses voneinander getrennt sein.

Der Wandabschnitt des Vakuumgehäuses kann die Spule und den Elektronenstrahl voneinander trennen. Das unmagnetische metallische Material geringer elektrischer Leitfähigkeit kann beispielsweise eine Chrom-Nickel-Stahl-Legierung umfassen.

Die Verwendung eines unmagnetischen Materials für den Wandabschnitt kann verhindern, dass das Magnetfeld der Spule durch den Wandabschnitt beeinflusst oder abgeschwächt wird. Dadurch kann eine effektive Ablenkung des Elektronenstrahls durch das Magnetfeld ermöglicht werden. Die geringe elektrische Leitfähigkeit des Materials kann eine Bildung von Wirbelströmen im Wandabschnitt reduzieren. Wirbelströme könnten sonst zu einer unerwünschten Erwärmung des Wandabschnitts führen und/oder das Magnetfeld beeinflussen.

Der Wandabschnitt kann so ausgebildet sein, dass er den Zwischenraum zwischen der Kathode und dem Anodenteller vom Bereich der Spule trennt. Insbesondere kann der Wandabschnitt des Vakuumgehäuses zwischen der Spule und dem Elektronenstrahl angeordnet sein.

Die Dicke des Wandabschnitts kann so gewählt sein, dass einerseits eine ausreichende mechanische Stabilität erreicht wird, andererseits aber die Beeinflussung des Magnetfelds minimiert wird. Die Dicke des Wandabschnitts kann im Bereich von 0,5 mm bis 5 mm liegen.

Der Wandabschnitt kann eine zylindrische oder leicht konische Form aufweisen, die der Geometrie des Vakuumgehäuses angepasst ist. Eine axiale Ausdehnung des Wandabschnitts kann so gewählt sein, dass sie den gesamten Bereich abdeckt, in dem die Ablenkung des Elektronenstrahls erfolgen soll.

In einer weiteren vorteilhaften Ausführungsform der vorgeschlagenen Röntgeneinheit kann der Wandabschnitt des Vakuumgehäuses eine Chrom-Nickel-Stahl-Legierung umfassen.

Vorteilhafterweise kann der Wandabschnitt des Vakuumgehäuses, der die Spule und den Elektronenstrahl trennt, aus einem Material umfassend eine Chrom-Nickel-Stahl-Legierung gebildet werden, insbesondere aus einer Chrom-Nickel-Strahl-Legierung gebildet werden. Die Chrom-Nickel-Stahl-Legierung kann eine Legierung aus Eisen, Chrom und Nickel umfassen, wobei ein Chromgehalt typischerweise zwischen 10% und 30% und ein Nickelgehalt zwischen 8% und 35% liegen können. Chrom-Nickel-Stahl-Legierungen können aufgrund ihrer Korrosionsbeständigkeit, Hitzebeständigkeit und nichtmagnetischen Eigenschaften für den Einsatz in Röntgenröhren vorteilhaft geeignet sein. Beispiele für Chrom-Nickel-Stahl-Legierungen umfassen Edelstähle der 300er Serie wie 304, 316 oder 321.

Die Verwendung einer Chrom-Nickel-Stahl-Legierung für den Wandabschnitt des Vakuumgehäuses kann vorteilhaft sein, da diese Legierung eine geringe elektrische Leitfähigkeit aufweisen und gleichzeitig unmagnetisch sein kann, wodurch Wechselwirkungen mit dem Magnetfeld der Spule minimiert werden können.

In einer weiteren vorteilhaften Ausführungsform der vorgeschlagenen Röntgeneinheit kann die Chrom-Nickel-Stahl-Legierung als austenitische Stahl-Legierung ausgebildet sein.

Eine austenitische Stahl-Legierung ist eine spezielle Art von Edelstahl, die sich durch ihre besondere Kristallstruktur auszeichnet. Diese Legierung kann einen hohen Anteil an Chrom und Nickel aufweisen. Die austenitische Struktur kann durch einen hohen Nickelgehalt in der Legierung erreicht werden. Typische Zusammensetzungen für austenitische Chrom-Nickel-Stähle können beispielsweise 18% Chrom und 8% Nickel (18/8-Stahl) oder 18% Chrom und 10% Nickel (18/10-Stahl) umfassen. Weitere Legierungselemente wie Molybdän, Titan oder Niob können hinzugefügt werden, um spezifische Eigenschaften zu verbessern.

Austenitische Stahl-Legierungen können vorteilhafterweise eine hohe Korrosionsbeständigkeit, gute Verformbarkeit, geringe magnetische Permeabilität und gute Schweißbarkeit aufweisen. Austenitische Stahl-Legierungen können ferner bei Raumtemperatur eine geringe elektrische Leitfähigkeit aufweisen und unmagnetisch sein. Beispiele für austenitische Stahl-Legierungen umfassen 1.4301 (AISI 304) oder 1.4401 (AISI 316).

Die Verwendung einer austenitischen Chrom-Nickel-Stahl-Legierung für den Wandabschnitt des Vakuumgehäuses kann mehrere Vorteile bieten. Die geringe magnetische Permeabilität kann eine Beeinflussung des Magnetfelds der Spule minimieren. Die hohe Korrosionsbeständigkeit kann eine Langlebigkeit der Röntgenröhre verbessern. Die gute Verformbarkeit kann eine Herstellung komplexer Gehäuseformen ermöglichen.

Darüber hinaus kann die austenitische Struktur auch bei höheren Temperaturen, wie sie in Röntgenröhren auftreten können, stabil bleiben. Dies kann zur Aufrechterhaltung der mechanischen und magnetischen Eigenschaften des Wandabschnitts während des Betriebs der Röntgenröhre beitragen.

Die Verwendung einer austenitischen Chrom-Nickel-Stahl-Legierung für den Wandabschnitt des Vakuumgehäuses kann somit eine vorteilhafte Kombination aus mechanischen, thermischen und magnetischen Eigenschaften bieten, die für die Funktion und Leistungsfähigkeit der Röntgeneinheit förderlich sein können.

In einer weiteren vorteilhaften Ausführungsform der vorgeschlagenen Röntgeneinheit kann die Kathode zumindest einen Emitter aufweisen, welcher zur Emission des Elektronenstrahls ausgebildet sein kann.

Der zumindest eine Emitter, insbesondere auch als Elektronenemitter bezeichnet, kann beispielsweise als Flachemitter, Glühkathode, Feldemissionskathode oder Photokathode ausgebildet sein. Bei einer Ausbildung des zumindest einen Emitters als Glühkathode kann der zumindest eine Emitter durch Erhitzen zur Emission von Elektronen angeregt werden. Bei einer Ausbildung des zumindest einen Emitters als Feldemissionskathode kann der zumindest eine Emitter durch Anlegen eines starken elektrischen Feldes zur Emission von Elektronen angeregt werden. Bei einer Ausbildung des zumindest einen Emitters als Photokathode kann der zumindest eine Emitter durch Bestrahlung mit Licht zur Emission von Elektronen angeregt werden.

Der zumindest eine Emitter kann zur Emission des Elektronenstrahls ausgebildet sein. Insbesondere kann der zumindest eine Emitter dazu ausgebildet sein, bei Anlegen einer geeigneten Spannung und/oder Bestromung Elektronen zu emittieren und so den Elektronenstrahl zu erzeugen. Die Emission des Elektronenstrahls kann durch thermische Emission, Feldemission oder eine Kombination aus beiden erfolgen.

Der Emitter kann aus einem geeigneten Emittermaterial gebildet sein. Als Emittermaterial können beispielsweise Wolfram, eine Wolframlegierung, Lanthanborid, Lanthanhexaborid, Bariumoxid, Cerborid oder andere geeignete Materialien verwendet werden.

Die Kathode kann einen oder mehrere Emitter umfassen. Bei mehreren Emittern können diese gleich oder verschieden ausgebildet sein. Beispielsweise kann die Kathode zwei verschieden ausgebildete Emitter aufweisen, welche jeweils zur Emission eines Elektronenstrahls ausgebildet sein können.

In einer weiteren vorteilhaften Ausführungsform der vorgeschlagenen Röntgeneinheit kann der zumindest eine Emitter als Flachemitter ausgebildet sein, welcher ein geschlitztes Emitterblech zur Emission des Elektronenstrahls aufweist.

Der zumindest eine Flachemitter kann als eine flache Elektrode ausgebildet sein, die Elektronen emittieren kann. Der zumindest eine Flachemitter kann das geschlitzte Emitterblech aufweisen. Das geschlitzte Emitterblech kann eine flache und/oder dünne Metallplatte mit einem oder mehreren Schlitzen und/oder einer oder mehreren Öffnungen umfassen. Der zumindest eine Schlitz und/oder die zumindest eine Öffnung können eine Verbesserung und/oder Steuerung der Emission von Elektronen ermöglichen. Die Schlitze und/oder Öffnungen im Emitterblech können in verschiedenen Mustern angeordnet sein, um eine vordefinierte Strahlform und/oder -intensität des Elektronenstrahls zu erzielen. Das Emitterblech kann beispielsweise aus Wolfram, Lantanborid oder einer Wolframlegierung gebildet werden. Der zumindest eine Flachemitter kann ferner als Vorratskathodenemitter, insbesondere umfassend Lanthanhexaborid oder Bariumoxid, oder Feldeffektemitter, insbesondere umfassend Kohlenstoff-Nanoröhren, ausgebildet sein.

Die Emission des Elektronenstrahls aus dem zumindest einen Flachemitter kann durch thermische Emission, Feldemission oder eine Kombination aus beiden erfolgen. Bei der thermischen Emission werden die Elektronen durch Erhitzen des Emitterblechs freigesetzt. Bei der Feldemission werden die Elektronen durch ein starkes elektrisches Feld aus dem Material des Emitterblechs herausgezogen.

Der zumindest eine Flachemitter kann dazu ausgebildet sein, einen flachen, bandförmigen Elektronenstrahl zu erzeugen. Dies kann vorteilhaft sein, um eine gleichmäßige Belastung des Anodentellers zu erreichen.

Die Kathode mit dem Flachemitter kann in Verbindung mit anderen Komponenten der Röntgenröhre arbeiten, wie beispielsweise einem Fokuskopf, um den Elektronenstrahl präzise auf den Anodenteller zu richten. Die Schlitze und/oder Öffnungen im Emitterblech können dazu ausgebildet sein, den Elektronenstrahl vorzuformen und/oder zu fokussieren, bevor er auf weitere fokussierende Elemente trifft.

Die Verwendung eines Flachemitters aus einem geschlitzten Emitterblech kann mehrere technische Vorteile bieten. Zum einen kann eine gleichmäßigere Elektronenverteilung im Strahl erreicht werden, was zu einer verbesserten Bildqualität führen kann. Zum anderen kann die flache Geometrie des Flachemitters zu einer kompakteren Bauweise der Kathode und damit der gesamten Röntgenröhre beitragen. Darüber hinaus kann das geschlitzte Design eine bessere Wärmeableitung ermöglichen, was die Lebensdauer des Flachemitters verlängern und die Stabilität des Elektronenstrahls über längere Betriebszeiten verbessern kann.

In einer weiteren vorteilhaften Ausführungsform der vorgeschlagenen Röntgeneinheit kann die Kathode zwei verschieden ausgebildete Emitter aufweisen, welche jeweils zur Emission eines Elektronenstrahls ausgebildet sind.

Die zwei Emitter können unterschiedliche Geometrien, Größen und/oder Emissionseigenschaften aufweisen, um verschiedene Elektronenstrahlprofile, insbesondere verschiedene Brennfleckgrößen auf dem Anodenteller, zu erzeugen. Beispielsweise kann ein erster Emitter für einen fokussierten Elektronenstrahl mit hoher Intensität ausgebildet sein, während ein zweiter Emitter für einen breiteren Elektronenstrahl mit geringerer Intensität ausgebildet sein kann. Beispielsweise kann ein erster Emitter für einen kleineren Brennfleck und ein zweiter Emitter für einen größeren Brennfleck ausgebildet sein. Dies ermöglicht eine Anpassung der Brennfleckgröße an verschiedene Bildgebungsanforderungen.

Die zwei Emitter können in der Kathode der Röntgenröhre angeordnet sein. Sie können dabei nebeneinander und/oder in einem definierten Abstand und/oder in einer vordefinierten Ausrichtung zueinander positioniert sein.

Jeder der zwei Emitter kann individuell ansteuerbar sein, um eine selektive Emission von Elektronenstrahlen zu ermöglichen. Dies kann eine flexible Steuerung der Röntgenstrahlcharakteristik ermöglichen, indem beispielsweise zwischen verschiedenen Brennfleckgrößen und/oder - formen gewechselt werden kann. Die Verwendung von zwei verschieden ausgebildeten Emittern kann vorteilhafterweise eine Anpassung der Röntgenstrahlcharakteristik an unterschiedliche Bildgebungsanforderungen ermöglichen. Beispielsweise kann für hochauflösende Aufnahmen ein fokussierter Elektronenstrahl verwendet werden, während für Übersichtsaufnahmen ein breiterer Elektronenstrahl vorteilhaft sein kann.

In einer weiteren vorteilhaften Ausführungsform der vorgeschlagenen Röntgeneinheit kann die Kathode einen Fokuskopf zur Fokussierung des Elektronenstrahls aufweisen.

Der Fokuskopf kann eine elektrostatische und/oder elektromagnetische Fokussierelemente umfassen, insbesondere zur elektrostatischen und/oder elektromagnetischen Fokussierung des Elektronenstrahls ausgebildet sein. Die Fokussierung des Elektronenstrahls kann eine Bündelung des Elektronenstrahls umfassen. Durch die Fokussierung kann ein Querschnitt des Elektronenstrahls anpassbar, insbesondere verringerbar, sein. Eine Verringerung des Querschnitts des Elektronenstrahls kann beispielsweise zu einer höheren Stromdichte des Elektronenstrahls im Brennfleck auf dem Anodenteller führen. Dies kann zu einer verbesserten Ortsauflösung der erzeugten Röntgenstrahlung führen.

Der Fokuskopf kann eine oder mehrere Elektroden umfassen, insbesondere als Fokussierelemente. Die Elektroden können beispielsweise als Flach-, Platten- und/oder Ringelektroden ausgebildet sein. Durch Anlegen jeweiliger, insbesondere unterschiedlicher, elektrischer Potenziale an die zumindest eine Elektrode kann ein elektrostatisches Feld erzeugt werden, welches zur Fokussierung des Elektronenstrahls ausgebildet ist. Gemäß einer Ausführungsform können die Elektroden in einem Betriebszustand der Röntgeneinheit auf einem gleichen elektrischen Potenzial, insbesondere dem gleichen elektrischen Potenzial wie die Kathode, insbesondere der zumindest eine Emitter, gehalten sein.

Alternativ oder zusätzlich kann der Fokuskopf eine oder mehrere Spulen umfassen. Die Spulen können zur Erzeugung eines Magnetfelds ausgebildet sein, welches zur Fokussierung des Elektronenstrahls ausgebildet ist.

Die Form des Fokuskopfs kann zur Fokussierung des Elektronenstrahls beitragen. Beispielsweise kann der Fokuskopf eine konische oder trichterförmige Geometrie aufweisen. Diese Geometrie kann die Feldlinien des elektrostatischen und/oder elektromagnetischen Felds so beeinflussen, dass eine Fokussierung des Elektronenstrahls bewirkt wird.

Der Fokuskopf kann aus einem elektrisch leitfähigen Material gefertigt sein, um die Erzeugung des elektrostatischen Felds zu ermöglichen. Geeignete Materialien umfassen beispielsweise Metalle oder Metalllegierungen.

Die Fokussierung des Elektronenstrahls mittels des Fokuskopfes kann in Kombination mit der magnetischen Ablenkung durch die Spule erfolgen. Dabei kann der Fokuskopf eine Vorfokussierung des Elektronenstrahls bewirken, während die Spule die präzise Positionierung des Brennflecks auf dem Anodenteller ermöglicht.

Der Fokuskopf kann als integraler Bestandteil der Kathode ausgebildet oder als separate Komponente in der Nähe der Kathode angeordnet sein.

Durch die Verwendung eines Fokuskopfes zur Fokussierung des Elektronenstrahls kann die Größe des Brennflecks auf dem Anodenteller reduziert werden, was zu einer verbesserten räumlichen Auflösung der erzeugten Röntgenstrahlung führen kann.

In einer weiteren vorteilhaften Ausführungsform der vorgeschlagenen Röntgeneinheit kann der Fokuskopf einstückig ausgebildet sein.

Ein einstückig ausgebildeter Fokuskopf kann als ein einzelnes, zusammenhängendes Bauteil ohne separate Komponenten ausgebildet sein. Der einstückig ausgebildete Fokuskopf kann aus einem einzigen Werkstück gefertigt sein. Beispielsweise kann der Fokuskopf aus einem Metallblock gefräst, gedreht, geschmiedet oder anderweitig spanend bearbeitet worden sein. Alternativ oder zusätzlich kann der Fokuskopf durch additive Fertigungsverfahren wie 3D-Druck hergestellt sein.

Die einstückige Ausbildung des Fokuskopfes kann verschiedene Vorteile bieten. Zum einen kann dadurch eine höhere mechanische Stabilität erreicht werden, da keine Verbindungsstellen oder Fügestellen vorhanden sind, die potenzielle Schwachstellen darstellen könnten. Zum anderen kann eine einstückige Konstruktion die Herstellung vereinfachen und Kosten reduzieren, da weniger Einzelteile gefertigt und montiert werden müssen. Darüber hinaus kann ein einstückiger Fokuskopf eine präzisere Formgebung ermöglichen, was für die Fokussierung des Elektronenstrahls von Vorteil sein kann.

Der einstückig ausgebildete Fokuskopf kann verschiedene Formen aufweisen, die für die Fokussierung des Elektronenstrahls optimiert sind. Beispielsweise kann der Fokuskopf eine konische, konkave, parabolische oder hyperbolische Innenkontur aufweisen.

Die einstückige Ausbildung kann eine präzise Ausrichtung der einzelnen Fokussierelemente des Fokuskopfes zueinander gewährleisten. Dadurch kann eine verbesserte Fokussierung des Elektronenstrahls erreicht werden. Zudem kann die Wärmeableitung innerhalb des einstückigen Fokuskopfs verbessert sein, was zu einer höheren thermischen Stabilität führen kann.

Der einstückige Fokuskopf kann verschiedene integrierte Strukturen aufweisen, wie beispielsweise Befestigungselemente und/oder elektrische Kontakte. Diese können direkt in das Werkstück eingearbeitet sein, ohne separate Bauteile zu erfordern.

Die einstückige Ausbildung kann auch die elektrische Leitfähigkeit des Fokuskopfs verbessern, da keine Übergänge oder Verbindungsstellen zwischen einzelnen Komponenten vorhanden sind. Dies kann zu einer gleichmäßigeren Verteilung des elektrischen Feldes und damit zu einer präziseren Fokussierung des Elektronenstrahls führen.

Durch die einstückige Ausbildung kann der Fokuskopf eine höhere mechanische Stabilität aufweisen. Dies kann insbesondere bei hohen thermischen oder mechanischen Belastungen von Vorteil sein, wie sie in einer Röntgenröhre auftreten können.

In einer weiteren vorteilhaften Ausführungsform der vorgeschlagenen Röntgeneinheit kann der Fokuskopf eine Chrom-Nickel-Stahl-Legierung oder eine Molybdän-Legierung umfassen.

Eine Chrom-Nickel-Stahl-Legierung kann als ein Werkstoff ausgebildet sein, der Chrom, Nickel und Stahl als Hauptbestandteile aufweist. Vorteilhafterweise kann der Fokuskopf zumindest teilweise, insbesondere vollständig, aus einer Chrom-Nickel-Stahl-Legierung gebildet, insbesondere hergestellt, sein. Insbesondere kann der Fokuskopf zumindest teilweise, insbesondere vollständig, aus einer Chrom-Nickel-Stahl-Legierung bestehen. Die Chrom-Nickel-Stahl-Legierung kann vorteilhafte Eigenschaften wie eine hohe Korrosionsbeständigkeit, gute mechanische Festigkeit und/oder Temperaturbeständigkeit aufweisen.

Die Chrom-Nickel-Stahl-Legierung des Fokuskopfes kann beispielsweise als austenitischer Edelstahl ausgebildet sein. Beispiele für austenitische Chrom-Nickel-Stahl-Legierungen, die für den Fokuskopf verwendet werden können, umfassen 1.4301 (X5CrNi18-10) oder 1.4401 (X5CrNiMo17-12-2).

Eine Molybdän-Legierung kann als eine Metalllegierung ausgebildet sein, bei der Molybdän das Hauptlegierungselement ist. Molybdän-Legierungen können sich durch hohe Festigkeit, gute Wärmeleitfähigkeit und/oder Beständigkeit gegen Kriechen bei hohen Temperaturen auszeichnen. Beispiele für Molybdän-Legierungen können Titan-Zirkonium-Molybdän (TZM), Molybdän-Hafnium-Kohlenstoff (MHC) oder Molybdän-Lanthanoxid (MLR) umfassen.

Die Verwendung einer Chrom-Nickel-Stahl-Legierung oder einer Molybdän-Legierung für den Fokuskopf kann vorteilhaft sein, da diese Legierungen eine gute thermische Leitfähigkeit aufweisen. Dies kann zu einer verbesserten Wärmeableitung und einer stabileren Fokussierung des Elektronenstrahls führen. Die hohe Temperaturbeständigkeit dieser Legierungen kann vorteilhaft sein, da der Fokuskopf während des Betriebs der Röntgenröhre hohen Temperaturen ausgesetzt sein kann. Die Temperaturbeständigkeit kann dazu beitragen, dass der Fokuskopf seine Form und Funktion auch bei längerer Betriebsdauer beibehält.

Darüber hinaus können die Chrom-Nickel-Stahl-Legierung oder die Molybdän-Legierung des Fokuskopfes jeweils eine hohe Beständigkeit gegen Elektronenbeschuss aufweisen. Dies kann eine Lebensdauer des Fokuskopfes vorteilhafterweise verlängern und/oder die Wartungsintervalle der Röntgeneinheit reduzieren.

Die Chrom-Nickel-Stahl-Legierung oder die Molybdän-Legierung des Fokuskopfes können ferner so ausgebildet sein, dass sie eine geringe Sekundärelektronenemission aufweisen. Dies kann zu einer Verbesserung der Fokussierungsqualität des Elektronenstrahls führen. Zusätzlich können die Chrom-Nickel-Stahl-Legierung oder die Molybdän-Legierung des Fokuskopfes jeweils eine gute Bearbeitbarkeit aufweisen, was die Herstellung von präzisen Fokuskopfgeometrien ermöglichen kann. Dies kann zu einer verbesserten Fokussierung des Elektronenstrahls und damit zu einer höheren Bildqualität der Röntgeneinheit beitragen.

Die Verwendung einer Chrom-Nickel-Stahl-Legierung oder einer Molybdän-Legierung für den Fokuskopf kann somit zu einer Verbesserung der Gesamtleistung, Zuverlässigkeit und Langlebigkeit der Röntgeneinheit beitragen.

In einer weiteren vorteilhaften Ausführungsform der vorgeschlagenen Röntgeneinheit kann der Fokuskopf zur Fokussierung jeweils eines oder beider der von den zwei Emittern emittierbaren Elektronenstrahlen ausgebildet sein.

Die Fokussierung kann dabei elektrostatisch und/oder elektromagnetisch erfolgen. Bei einer elektrostatischen Fokussierung kann der Fokuskopf beispielsweise eine oder mehrere Elektroden umfassen, die auf einem bestimmten elektrischen Potenzial gehalten werden. Durch eine Formgebung und/oder Anordnung dieser Elektroden kann ein elektrisches Feld so geformt werden, dass es den jeweiligen Elektronenstrahl fokussiert.

Bei einer elektromagnetischen Fokussierung kann die Röntgeneinheit eine oder mehrere weitere Spulen umfassen, die dazu ausgebildet sind, ein weiteres Magnetfeld zu erzeugen. Die zumindest eine weitere Spule kann außerhalb des Vakuumgehäuses angeordnet sein. Das weitere Magnetfeld kann mittels eines Jochs der zumindest einen weiteren Spule zum Fokuskopf geführt werden. Dieses Magnetfeld kann so geformt sein, dass es den Elektronenstrahl fokussiert.

Der Fokuskopf kann auch dazu ausgebildet sein, den Elektronenstrahl elektrostatisch und elektromagnetisch zu fokussieren, insbesondere gleichzeitig. Dies kann eine besonders präzise Steuerung des Elektronenstrahls ermöglichen.

Die Fokussierung kann für jeden der beiden Elektronenstrahlen separat einstellbar sein. Dies kann vorteilhaft eine Anpassung der Fokussierung an die jeweiligen Anforderungen des Betriebs der Röntgeneinheit ermöglichen. Beispielsweise kann der Fokuskopf einen Elektronenstrahl für einen kleinen Brennfleck stark fokussieren, während der Fokuskopf den anderen Elektronenstrahl für einen größeren Brennfleck weniger stark fokussiert.

Der Fokuskopf kann ferner dazu ausgebildet sein, beide Elektronenstrahlen gleichzeitig zu fokussieren. Dies kann für Betriebsmodi vorteilhaft sein, in denen beide Emitter gleichzeitig verwendet werden.

Der Fokuskopf kann zur Fokussierung jeweils eines oder beider der von den zwei Emittern emittierbaren Elektronenstrahlen ausgebildet sein. Die Kathode kann zwei verschieden ausgebildete Emitter aufweisen, welche jeweils zur Emission eines Elektronenstrahls ausgebildet sein können. Der Fokuskopf kann dazu ausgebildet sein, die von den Emittern emittierten Elektronenstrahlen zu fokussieren.

Der Fokuskopf kann so ausgebildet sein, dass er die Elektronenstrahlen beider Emitter gleichzeitig oder abwechselnd fokussieren kann. Dies kann durch eine entsprechende Gestaltung der Fokuskopfgeometrie und/oder durch eine dynamische Anpassung des elektrischen Potenzials des Fokuskopfes erreicht werden. Die Fokussierung der Elektronenstrahlen durch den Fokuskopf kann durch eine Steuerungseinheit steuerbar sein. Die Steuerungseinheit kann dazu ausgebildet sein, das elektrische Potenzial des Fokuskopfes anzupassen, um die Fokussierung anzupassen.

Die Ausbildung des Fokuskopfs zur Fokussierung beider Elektronenstrahlen kann eine kompakte Bauweise der Kathode ermöglichen, da nur ein Fokussierungselement für beide Elektronenstrahlen benötigt wird. Dies kann den Platzbedarf innerhalb der Röntgenröhre reduzieren und die Herstellung vereinfachen.

Durch die Möglichkeit, einen oder beide Elektronenstrahlen zu fokussieren, kann der Fokuskopf eine hohe Flexibilität in der Steuerung der Röntgenröhre ermöglichen. Dies kann zu einer verbesserten Bildqualität und einer effizienteren Nutzung der Röntgenröhre in verschiedenen Anwendungsszenarien führen.

In einer weiteren vorteilhaften Ausführungsform der vorgeschlagenen Röntgeneinheit kann der Fokuskopf zumindest durch seine Form zur elektrostatischen Fokussierung des Elektronenstrahls ausgebildet sein.

Der Fokuskopf kann zur elektrostatischen Fokussierung des Elektronenstrahls ausgebildet sein. Die elektrostatische Fokussierung kann durch die Form des Fokuskopfes erreicht werden. Der Fokuskopf kann beispielsweise eine konkave Form aufweisen, um den Elektronenstrahl zu bündeln. Alternativ oder zusätzlich kann der Fokuskopf Elektroden umfassen, an die ein elektrisches Potenzial angelegt werden kann, um ein elektrostatisches Feld zur Fokussierung des Elektronenstrahls zu erzeugen. Insbesondere können die Elektroden in einem Betriebszustand der Röntgeneinheit auf einem gleichen elektrischen Potenzial, insbesondere dem gleichen elektrischen Potenzial wie die Kathode, insbesondere der zumindest eine Emitter, gehalten sein. Bei einer einstückigen Ausbildung des Fokuskopfes kann der Fokuskopf in dem Betriebszustand der Röntgeneinheit beispielsweise auf dem gleichen elektrischen Potenzial wie die Kathode, insbesondere der zumindest eine Emitter, gehalten sein.

Die Form des Fokuskopfes kann speziell an die gewünschte Fokussierung des Elektronenstrahls angepasst sein. Beispielsweise kann der Fokuskopf eine asymmetrische Form aufweisen, um eine astigmatische Fokussierung zu erreichen. Alternativ kann der Fokuskopf eine symmetrische Form aufweisen, um eine symmetrische Fokussierung zu erreichen. Die Form des Fokuskopfes kann ferner dazu ausgebildet sein, unerwünschte Streueffekte des Elektronenstrahls zu minimieren. Die Form des Fokuskopfs kann an die spezifische Geometrie der Röntgenröhre und die gewünschten Fokussierungseigenschaften angepasst sein. Beispielsweise kann der Fokuskopf für unterschiedliche Brennfleckgrößen oder Elektronenstrahlenergien optimiert sein.

Der Fokuskopf kann aus einem elektrisch leitfähigen Material hergestellt sein, um die elektrostatische Fokussierung zu ermöglichen. Geeignete Materialien umfassen beispielsweise Metalle wie Wolfram, Molybdän, eine Molybdän-Legierung und/oder eine Chrom-Nickel-Stahl-Legierung. Eine Oberfläche des Fokuskopfes kann zusätzlich beschichtet sein, um eine Emission von Sekundärelektronen zu reduzieren.

Der Fokuskopf kann in einer Ausführungsform zwei flache Fokussierelemente, insbesondere Fokussierplatten, aufweisen, die einander gegenüberliegend angeordnet sind. Die Fokussierelemente können in einem Betriebszustand der Röntgeneinheit auf einem gleichen elektrischen Potenzial, insbesondere dem gleichen elektrischen Potenzial wie die Kathode, insbesondere der zumindest eine Emitter, gehalten sein.

Durch das gleiche elektrische Potenzial der Fokussierplatten und der Kathode kann ein, insbesondere homogenes, elektrisches Feld im Zwischenraum zwischen der Kathode und dem Anodenteller erzeugbar sein. Dieses elektrische Feld kann eine Bündelung des Elektronenstrahls und eine Reduzierung einer Divergenz des Elektronenstrahls ermöglichen. Eine elektrische Feldstärke zwischen den Fokussierelementen kann durch einen Abstand der der Fokussierelemente beeinflussbar sein.

Diese Konfiguration des Fokuskopfes kann eine effektive Fokussierung des Elektronenstrahls ermöglichen, insbesondere ohne zusätzliche Spannungsquellen oder komplexe Elektrodengeometrien zu benötigen. Die symmetrische Anordnung kann dazu beitragen, Aberrationen des Elektronenstrahls zu minimieren und eine gleichmäßige Fokussierung über den gesamten Strahlquerschnitt zu erreichen.

Die elektrostatische Fokussierung durch den Fokuskopf kann vorteilhaft sein, da sie ohne zusätzliche magnetische Felder auskommt und somit kompakter und energieeffizienter realisiert werden kann als eine elektromagnetische Fokussierung. Zudem kann die elektrostatische Fokussierung schneller anpassbar sein, was eine dynamische Steuerung des Elektronenstrahls ermöglicht.

Die elektrostatische Fokussierung durch den Fokuskopf kann dazu beitragen, den Elektronenstrahl auf einen kleinen Brennfleck auf dem Anodenteller zu konzentrieren. Dies kann eine Auflösung und Bildqualität von mittels der erzeugten Röntgenstrahlung erfassbaren Bilddaten, insbesondere Röntgenbilddaten, verbessern.

In einer weiteren vorteilhaften Ausführungsform der vorgeschlagenen Röntgeneinheit kann das Vakuumgehäuse an einer kathodenseitigen Wand ein zumindest teilweise versenktes Pumpkapillar zur Vakuumierung eines Innenbereichs des Vakuumgehäuses aufweisen.

Das Pumpkapillar kann als eine röhrenförmige Struktur ausgebildet sein, die eine Verbindung zwischen einem Innenbereich des Vakuumgehäuses und einem Außenbereich des Vakuumgehäuses ermöglicht. Das Pumpkapillar kann dazu ausgebildet sein, eine Vakuumpumpe mit dem Innenbereich des Vakuumgehäuses zu verbinden, um eine Evakuierung, insbesondere Vakuumierung, des Innenbereichs zu ermöglichen. Nach erfolgter Evakuierung kann das Pumpkapillar verschließbar sein, beispielsweise durch Abschmelzen, Abklemmen und/oder mittels eines Verschlusselements.

Das zumindest teilweise versenkte Pumpkapillar kann so ausgebildet sein, dass es teilweise oder vollständig in die kathodenseitige Wand des Vakuumgehäuses eingelassen ist. Hierdurch können vorteilhafterweise Gesamtabmessungen der Röntgenröhre reduziert werden. Zudem kann das versenkte Pumpkapillar besser vor mechanischen Beschädigungen geschützt sein.

Die kathodenseitige Wand des Vakuumgehäuses kann eine Wand des Vakuumgehäuses bezeichnen, die der Kathode am nächsten liegt und/oder an der die Kathode befestigt ist. Die Anordnung des Pumpkapillars an der kathodenseitigen Wand kann vorteilhaft sein, da dort typischerweise mehr Platz zur Verfügung steht als an anderen Stellen des Vakuumgehäuses. Zudem kann durch die Anordnung des Pumpkapillars an der kathodenseitigen Wand des Vakuumgehäuses eine effiziente Evakuierung des gesamten Innenbereichs des Vakuumgehäuses ermöglicht werden.

In einer weiteren vorteilhaften Ausführungsform der vorgeschlagenen Röntgeneinheit kann die rotierbare Lagerung des Anodentellers eine Schwingungskompensation umfassen.

Die Schwingungskompensation kann dazu ausgebildet sein, unerwünschte Schwingungen und/oder Vibrationen des Anodentellers während einer Rotation des Anodentellers zu reduzieren oder zu eliminieren. Die Schwingungskompensation kann beispielsweise mechanische Dämpfer, aktive Schwingungsdämpfungssysteme und/oder spezielle Lagerungen umfassen.

Mechanische Dämpfer können beispielsweise Federn und/oder Gummilager umfassen. Ferner kann ein mechanischer Dämpfer als viskose Dämpfung ausgebildet sein, bei der ein Fluid zwischen beweglichen Teilen der Lagerung angeordnet ist. Das Fluid kann dazu ausgebildet sein, Schwingungsenergie in Wärme umzuwandeln und somit zu dissipieren. Diese mechanischen Dämpfer können dazu ausgebildet sein, mechanische Schwingungen zu absorbieren und eine Übertragung der Schwingungen auf andere Teile der Röntgenröhre zu verringern.

Ein aktives Schwingungsdämpfungssystem kann Sensoren umfassen, die Schwingungen des Anodentellers erfassen. Basierend auf den erfassten Schwingungen kann das Schwindungsdämpfungssystem Gegenkräfte erzeugen, um die Schwingungen zu kompensieren. Die Gegenkräfte können beispielsweise durch elektromagnetische Aktuatoren und/oder piezoelektrische Aktuatoren erzeugbar sein.

Spezielle Lagerungen zur Schwingungskompensation können beispielsweise magnetische Lager umfassen. Magnetische Lager können den Anodenteller berührungslos in einer Schwebe halten und somit mechanische Reibung und damit verbundene Schwingungen reduzieren.

Die Schwingungskompensation kann dazu beitragen, eine Präzision und Stabilität des Röntgenstrahls zu verbessern, indem sie unerwünschte Bewegungen des Brennflecks auf dem Anodenteller minimiert. Dies kann zu einer verbesserten Bildqualität in der Röntgenbildgebung führen.

In einer weiteren vorteilhaften Ausführungsform der vorgeschlagenen Röntgeneinheit kann die Röntgenröhre ferner eine Antriebseinheit zur motorischen Rotation des Anodentellers umfassen.

Die Antriebseinheit kann als eine Komponente ausgebildet sein, die dazu ausgebildet ist, eine Rotationsbewegung des Anodentellers zu ermöglichen und aufrechtzuerhalten. Die Antriebseinheit kann beispielsweise einen Elektromotor umfassen, der mechanisch mit dem Anodenteller verbunden ist. Alternativ oder zusätzlich kann die Antriebseinheit auch einen hydraulischen und/oder pneumatischen Antrieb umfassen. Die Antriebseinheit kann ferner eine Steuerungseinheit umfassen, die dazu ausgebildet ist, die Rotationsgeschwindigkeit und -richtung des Anodentellers zu regeln.

Die motorische Rotation des Anodentellers kann als eine durch einen Motor angetriebene Drehbewegung des Anodentellers um die Anodenachse ausgebildet sein. Die motorische Rotation kann eine kontrollierte und gleichmäßige Drehung des Anodentellers ermöglichen, was für eine gleichmäßige Verteilung der thermischen Belastung auf der Anodenoberfläche vorteilhaft sein kann. Die Rotationsgeschwindigkeit kann dabei an die jeweiligen Betriebsbedingungen der Röntgenröhre angepasst sein.

Die Schwingungskompensation kann mit einer Rotationssteuerung des Anodentellers interagieren. Beispielsweise kann die Rotationsgeschwindigkeit basierend auf den erfassten Schwingungen angepasst werden, um Resonanzen zu vermeiden.

Die Erfindung betrifft in einem zweiten Aspekt ein Verfahren zum Betrieb einer vorgeschlagenen Röntgeneinheit. In einem ersten Schritt wird eine Hochspannung zwischen der Kathode und dem Anodenteller bereitgestellt. In einem weiteren Schritt wird die Kathode bestromt, um den Elektronenstrahl bereitzustellen. In einem weiteren Schritt wird der Anodenteller mit einer Rotationsfrequenz innerhalb eines vorgegebenen Rotationsfrequenzbereichs rotiert. In einem weiteren Schritt wird die Spule mit einem Ablenkstrom bestromt, um das Magnetfeld bereitzustellen. In einem weiteren Schritt wird die Ablenkung des Elektronenstrahls durch Anpassen der Bestromung der Spule angepasst. Die vorstehend beschriebenen Schritte können dabei vorteilhafterweise zumindest teilweise, insbesondere vollständig, gleichzeitig oder nacheinander ausgeführt werden. Zudem kann eine Reihenfolge der Ausführung der vorstehend beschriebenen Schritte variiert werden, beispielsweise kann das Rotieren des Anodentellers vor dem Bestromen der Kathode ausgeführt werden.

Die Vorteile des vorgeschlagenen Verfahrens entsprechen im Wesentlichen den Vorteilen der vorgeschlagenen Röntgeneinheit. Hierbei erwähnte Merkmale, Vorteile oder alternative Ausführungsformen können ebenso auch auf die anderen beanspruchten Gegenstände übertragen werden und umgekehrt.

Das Bereitstellen der Hochspannung zwischen der Kathode und dem Anodenteller kann ein Anlegen einer elektrischen Spannung zwischen der Kathode und dem Anodenteller umfassen. Die Hochspannung kann beispielsweise mittels einer Hochspannungseinheit bereitgestellt werden. Die Hochspannung kann vorteilhafterweise im Bereich von 10 kV bis 300 kV liegen.

Die Bestromung der Kathode zum Bereitstellen des Elektronenstrahls kann ein Anlegen eines elektrischen Heizstroms an die Kathode umfassen. Der Heizstrom kann beispielsweise mittels einer Stromversorgungseinheit bereitgestellt werden. Eine Stromstärke des an die Kathode angelegten Heizstroms kann vorteilhafterweise in einem Bereich von 10 mA bis 20000 mA liegen.

Das Rotieren des Anodentellers mit einer Rotationsfrequenz innerhalb eines vorgegebenen Rotationsfrequenzbereichs kann ein Antreiben des Anodentellers mittels einer Antriebseinheit umfassen. Dabei kann der Anodenteller um die Anodenachse rotieren. Die Antriebseinheit kann beispielsweise einen Elektromotor umfassen. Der vorgegebene Rotationsfrequenzbereich kann vorteilhafterweise Rotationsfrequenzen von mindestens 50 Hz, insbesondere zwischen 130 Hz bis 160 Hz, umfassen.

Die Bestromung der Spule mit dem Ablenkstrom zum Bereitstellen des Magnetfelds kann ein Anlegen eines elektrischen Stroms an die Spule umfassen. Der Ablenkstrom kann beispielsweise mittels einer Stromversorgungseinheit bereitgestellt werden. Eine Stromstärke des an die Spule angelegten Ablenkstroms kann vorteilhafterweise in einem Bereich von 0 A bis 20 A liegen.

Das Anpassen der Ablenkung des Elektronenstrahls durch Anpassen der Bestromung der Spule kann ein Verändern des Ablenkstroms, insbesondere einer Stromstärke des Ablenkstroms, umfassen. Das Anpassen der Bestromung der Spule kann beispielsweise mittels einer Steuerungseinheit erfolgen. Die Steuerungseinheit kann dazu ausgebildet sein, den Ablenkstrom in Abhängigkeit von verschiedenen Parametern, wie beispielsweise der Hochspannung, einer Bewegungsgeschwindigkeit der Röntgeneinheit, einer Bewegungsrichtung der Röntgeneinheit und/oder einer Positionierung der Röntgeneinheit, anzupassen.

Das Bereitstellen der Hochspannung zwischen der Kathode und dem Anodenteller kann ein starkes elektrisches Feld innerhalb der Röntgenröhre, insbesondere dem Zwischenraum zwischen Kathode und Anodenteller, erzeugen. Dieses elektrische Feld kann zur Beschleunigung der von der Kathode emittierten Elektronen ausgebildet sein.

Die Bestromung der Kathode mit dem Heizstrom kann zur thermischen Emission von Elektronen führen. Durch den Heizstrom kann die Kathode auf eine hohe Temperatur gebracht werden, wodurch Elektronen aus dem Kathodenmaterial austreten können. Diese freigesetzten Elektronen bilden den Elektronenstrahl.

Das starke elektrische Feld, das durch die Hochspannung erzeugt wird, kann die von der Kathode emittierten Elektronen in Richtung des Anodentellers beschleunigen. Die kinetische Energie der Elektronen kann dabei proportional zur angelegten Spannung steigen.

Die Rotation des Anodentellers mit der definierten Rotationsfrequenz kann eine Verteilung einer thermischen Belastung auf einer größeren Fläche des Anodentellers ermöglichen. Durch die Rotation des Anodentellers kann vorteilhaft verhindert werden, dass sich der Auftreffpunkt des Elektronenstrahls, insbesondere der Brennfleck, zu stark erhitzt. Hierdurch können vorteilhafterweise eine Lebensdauer des Anodentellers verlängert und eine höhere Leistung ermöglicht werden.

Durch die Bestromung der Spule kann ein Magnetfeld erzeugt werden, das den Zwischenraum zwischen Kathode und Anodenteller durchdringt. Dieses Magnetfeld kann dazu ausgebildet sein, den Elektronenstrahl abzulenken und somit eine Position des Brennflecks auf dem Anodenteller zu beeinflussen. Durch das Anpassen der Bestromung der Spule kann die Ablenkung des Elektronenstrahls präzise gesteuert werden. Hierdurch kann eine dynamische Kontrolle über die Position des Brennflecks ermöglicht werden, was für verschiedene Bildgebungstechniken, beispielsweise eine Tomosynthese, vorteilhaft sein kann.

Wenn die beschleunigten Elektronen auf den Anodenteller treffen, können sie abrupt abgebremst werden. Bei diesem Prozess kann ein Teil der kinetischen Energie der Elektronen in Röntgenstrahlung umgewandelt werden.

Die erzeugte Röntgenstrahlung kann durch das Strahlenaustrittsfenster aus der Röntgenröhre austreten. Ferner kann die bereitgestellte Röntgenstrahlung der Röntgeneinheit für eine Bildgebung genutzt werden.

Die bereitgestellte Hochspannung kann sowohl eine Energie der Elektronen als auch eine Intensität und ein Spektrum der erzeugten Röntgenstrahlung beeinflussen. Die Rotationsfrequenz des Anodentellers kann vorteilhafterweise mit einer Leistung des Elektronenstrahls abgestimmt sein, um eine optimale Wärmeverteilung zu gewährleisten. Die Ablenkung des Elektronenstrahls durch das Magnetfeld kann präzise auf die Hochspannung, die Bewegungsgeschwindigkeit der Röntgeneinheit, die Bewegungsrichtung der Röntgeneinheit und/oder die Positionierung der Röntgeneinheit abgestimmt sein, um eine gewünschte Brennfleckposition zu erreichen.

Durch die sorgfältige Abstimmung und Steuerung dieser Parameter kann eine effiziente und präzise Röntgenstrahlungserzeugung erreicht werden, die eine hohe Bildqualität ermöglicht.

In einer weiteren vorteilhaften Ausführungsform des vorgeschlagenen Verfahrens zum Betrieb einer Röntgeneinheit kann die Kathode zumindest einen Emitter und einen Fokuskopf zur Fokussierung des Elektronenstrahls aufweisen. Die Bestromung der Kathode kann eine Bestromung des zumindest einen Emitters umfassen. Das Verfahren kann weiterhin ein Bereitstellen eines elektrischen Potenzials des zumindest einen Emitters oder eines weiteren elektrischen Potenzials an den Fokuskopf zur Fokussierung des Elektronenstrahls umfassen.

Die Bestromung des zumindest einen Emitters kann ein Anlegen des elektrischen Heizstroms an den zumindest einen Emitter, insbesondere ein Emitterblech des zumindest einen Emitters, umfassen. Hierdurch können eine Emission von Elektronen aus dem Emitterblech ermöglicht und der Elektronenstrahl gebildet werden.

Das Bereitstellen des elektrischen Potenzials des zumindest einen Emitters oder eines weiteren elektrischen Potenzials an den Fokuskopf kann ein Anlegen elektrischer Spannungen an die entsprechenden Komponenten umfassen. Das Bereitstellen des jeweiligen elektrischen Potenzials kann durch eine Spannungsquelle und entsprechende elektrische Verbindungen realisiert werden.

Gemäß einer ersten Ausführungsform können das elektrische Potenzial des zumindest einen Emitters und das elektrische Potenzial des Fokuskopfes gleich sein. Insbesondere kann ein einziges elektrisches Potenzial sowohl an den zumindest einen Emitter als auch an den Fokuskopf angelegt werden. Hierdurch können vorteilhaft eine Komplexität des Systems reduziert und eine einfachere Steuerung ermöglicht werden. Die Fokussierung des Elektronenstrahls kann in dieser Ausführungsform hauptsächlich durch eine geometrische Form des Fokuskopfes erreicht werden.

Gemäß einer zweiten Ausführungsform können unterschiedliche elektrische Potenziale an den zumindest einen Emitter und den Fokuskopf angelegt werden. Diese Ausführungsform kann eine flexible Steuerung des Elektronenstrahls ermöglichen. Mittels einer Anpassung einer Potenzialdifferenz zwischen dem zumindest einen Emitter und dem Fokuskopf, insbesondere einer Differenz zwischen dem Potenzial des zumindest einen Emitters und dem weiteren Potenzial, kann die Fokussierung des Elektronenstrahls präzise gesteuert werden. Dies kann besonders vorteilhaft sein, um die Brennfleckgröße und/oder -form dynamisch an verschiedene Bildgebungsanforderungen anzupassen.

Das elektrische Potenzial des zumindest einen Emitters kann als eine elektrische Spannung ausgebildet sein, die an den zumindest einen Emitter angelegt wird. Dieses Potenzial kann die Emission von Elektronen aus dem zumindest einen Emitter beeinflussen. Das weitere elektrische Potenzial kann als eine zusätzliche oder separate elektrische Spannung ausgebildet sein, die an den Fokuskopf angelegt wird. Dieses weitere Potenzial kann zur Steuerung der Fokussierung des Elektronenstrahls dienen.

Die Fokussierung des Elektronenstrahls kann eine Veränderung, insbesondere eine Verringerung, eines Querschnitts des Elektronenstrahls und/oder eine Konzentration des Elektronenstrahls auf einen bestimmten Punkt oder Bereich umfassen. Der Fokuskopf kann zur Fokussierung des Elektronenstrahls ausgebildet sein. Der Fokuskopf kann beispielsweise eine spezielle Geometrie und/oder elektrische Eigenschaften aufweisen, um den Elektronenstrahl zu fokussieren.

Durch die Kombination von Emitter, Fokuskopf und gezielter Anwendung elektrischer Potenziale kann eine verbesserte Fokussierung und Kontrolle des Elektronenstrahls erreicht werden. Dies kann zu einer höheren Bildqualität und einer effizienteren Nutzung der Röntgenstrahlung führen.

In einer weiteren vorteilhaften Ausführungsform des vorgeschlagenen Verfahrens zum Betrieb einer Röntgeneinheit kann die Kathode zwei verschieden ausgebildete Emitter aufweisen, welche jeweils zur Emission eines Elektronenstrahls ausgebildet sein können. Die Emitter können selektiv oder gleichzeitig bestromt werden.

Die selektive Bestromung der Emitter kann eine Bestromung jeweils eines der zwei Emitter umfassen. Insbesondere kann die selektive Bestromung der Emitter umfassen, dass zu einem bestimmten Zeitpunkt nur einer der Emitter bestromt wird. Vorteilhafterweise kann jeweils nur einer der Emitter bestromt werden, um einen Elektronenstrahl mit spezifischen Eigenschaften zu erzeugen. Beispielsweise können durch die selektive Bestromung der Emitter unterschiedliche Brennfleckgrößen und/oder Elektronenstrahlenergien erzeugbar sein. Die Auswahl des jeweils zu bestromenden Emitters kann manuell, insbesondere basierend auf einer Nutzereingabe, oder automatisch erfolgen, beispielsweise basierend auf einem vorgegebenen Messprotokoll. Die Bestromung der Emitter kann durch eine Steuerungseinheit gesteuert werden. Die Steuerungseinheit kann dazu ausgebildet sein, die Bestromung der Emitter basierend auf Betriebsparametern, beispielsweise einer Strahlintensität, Brennfleckgröße und/oder Bildgebungsanforderung, anzupassen.

Die gleichzeitige Bestromung der Emitter kann eine Bestromung von beiden Emittern zu einem bestimmten Zeitpunkt umfassen. Die gleichzeitige Bestromung beider Emitter kann vorteilhafterweise eine Erzeugung eines, insbesondere kombinierten, Elektronenstrahls mit einer höheren Intensität und/oder einer komplexeren Strahlgeometrie ermöglichen. Durch eine Anpassung und/oder Variation einer Stromstärke der Bestromung der einzelnen Emitter kann zudem eine Feinabstimmung von Eigenschaften des, insbesondere kombinierten, Elektronenstrahls erfolgen.

Die Möglichkeit der selektiven oder gleichzeitigen Bestromung der Emitter kann eine Flexibilität und Anpassungsfähigkeit der Röntgeneinheit verbessern. Die selektive oder gleichzeitige Bestromung der Emitter kann in Kombination mit der Ablenkung des Elektronenstrahls durch das Magnetfeld der Spule erfolgen. Dies kann eine präzise Steuerung der Position und/oder Charakteristik des Brennflecks auf dem Anodenteller ermöglichen.

Diese Ausführungsform des Verfahrens kann den Vorteil bieten, dass eine flexible und anpassbare Elektronenstrahlerzeugung ermöglicht wird. Durch die Möglichkeit, zwischen verschiedenen Emittern zu wählen oder diese zu kombinieren, kann das Verfahren an unterschiedliche Bildgebungsanforderungen angepasst werden. Dies kann zu einer verbesserten Bildqualität und einer erhöhten Flexibilität des Röntgengeräts führen.

In einer weiteren vorteilhaften Ausführungsform des vorgeschlagenen Verfahrens zum Betrieb einer Röntgeneinheit kann der vorgegebene Rotationsfrequenzbereich Rotationsfrequenzen von mindestens 50 Hz, insbesondere von 130 Hz bis 160 Hz, umfassen.

Der Rotationsfrequenzbereich kann einen Bereich von Rotationsfrequenzen definieren, innerhalb dessen der Anodenteller der Röntgeneinheit in einem Betriebszustand der Röntgeneinheit rotieren soll. Die Rotationsfrequenz kann dabei eine Anzahl von, insbesondere vollständigen, Umdrehungen des Anodentellers pro Sekunde angeben. Der vorgegebene Rotationsfrequenzbereich von mindestens 50 Hz, insbesondere von 130 Hz bis 160 Hz kann somit einer Rotationsgeschwindigkeit des Anodentellers von mindestens 50, insbesondere von 130 bis 160, Umdrehungen pro Sekunde entsprechen. Dieser Bereich kann geeignet für den Betrieb der Röntgenröhre in einem medizinischen Bildgebungsgerät sein.

Die Vorgabe des Rotationsfrequenzbereichs kann einen stabilen und effizienten Betrieb der Röntgenröhre ermöglichen. Eine zu niedrige Rotationsfrequenz kann zu einer ungleichmäßigen Verteilung der Wärme auf dem Anodenteller führen, während eine zu hohe Rotationsfrequenz mechanische Belastungen und Vibrationen verursachen kann. Die Einhaltung des vorgegebenen Rotationsfrequenzbereichs kann zudem eine gleichmäßige Verteilung des Brennflecks auf dem Anodenteller ermöglichen. Dies kann zu einer längeren Lebensdauer der Drehanode beitragen.

Die Rotationsfrequenz des Anodentellers kann durch eine Antriebseinheit gesteuert werden. Die Antriebseinheit kann beispielsweise einen Elektromotor umfassen, der über eine Welle mit dem Anodenteller verbunden ist. Durch Regelung der Drehzahl der Antriebseinheit, insbesondere des Elektromotors, kann die Rotationsfrequenz des Anodentellers eingestellt werden.

Durch die Vorgabe des Rotationsfrequenzbereichs kann außerdem eine reproduzierbare Bildqualität bei wiederholten Aufnahmen erreicht werden. Dies kann insbesondere bei medizinischen Anwendungen von Bedeutung sein, bei denen eine konstante Bildqualität erforderlich ist.

In einer weiteren vorteilhaften Ausführungsform des vorgeschlagenen Verfahrens zum Betrieb einer Röntgeneinheit kann überwacht werden, ob die momentane Rotationsfrequenz des Anodentellers innerhalb des vorgegebenen Rotationsfrequenzbereichs liegt. Verneinendenfalls kann ein Warnhinweis bereitgestellt werden.

Alternativ oder zusätzlich können weitere Betriebsparameter der Röntgeneinheit überwacht werden, beispielsweise eine Temperatur und/oder eine Stromversorgung und/oder Spannungsversorgung, wobei bei Identifikation einer Abweichung gegenüber einem vordefinierten Wert oder Werteintervall ein weiterer Warnhinweis bereitgestellt werden kann.

Das Überwachen, ob die momentane Rotationsfrequenz des Anodentellers innerhalb des vorgegebenen Rotationsfrequenzbereichs liegt, kann ein kontinuierliches oder periodisches Erfassen und Auswerten der momentanen Rotationsfrequenz des Anodentellers umfassen. Die momentane Rotationsfrequenz kann beispielsweise mittels eines Sensors erfasst werden, der die Rotation des Anodentellers detektiert. Alternativ oder zusätzlich kann das Erfassen der momentanen Rotationsfrequenz mittels einer kalibrierten Antriebseinheit zur Rotation des Anodentellers erfolgen. Das Überwachen kann durch eine Steuerungseinheit der Röntgeneinheit durchgeführt werden.

Der Warnhinweis kann eine visuelle, akustische und/oder haptische Signalisierung umfassen, die auf eine Abweichung der momentanen Rotationsfrequenz vom vorgegebenen Rotationsfrequenzbereich hinweist. Der Warnhinweis kann verschiedene Informationen umfassen, beispielsweise die momentane Rotationsfrequenz, eine Abweichung der momentanen Rotationsfrequenz vom vorgegebenen Rotationsfrequenzbereich und/oder eine Handlungsempfehlung zur Korrektur der Rotationsfrequenz. Der Warnhinweis, insbesondere eine graphische Darstellung des Warnhinweises, kann beispielsweise mittels einer Darstellungseinheit angezeigt werden. Alternativ oder zusätzlich kann der Warnhinweis als akustisches Signal über einen Lautsprecher ausgegeben werden. Alternativ oder zusätzlich kann der Warnhinweis als haptisches Signal, beispielsweise mittels eines Vibrationsmotors, bereitgestellt werden. Der Warnhinweis kann es vorteilhaft ermöglichen, einen Nutzer auf eine mögliche Fehlfunktion und/oder suboptimale Betriebsbedingung der Röntgeneinheit aufmerksam zu machen.

Das Bereitstellen des Warnhinweises kann automatisch durch die Steuerungseinheit der Röntgeneinheit erfolgen, wenn festgestellt wird, dass die momentane Rotationsfrequenz außerhalb des vorgegebenen Rotationsfrequenzbereichs liegt. Die Steuerungseinheit kann dazu ausgebildet sein, den Warnhinweis über eine entsprechende Ausgabeschnittstelle bereitzustellen.

Durch das Überwachen der Rotationsfrequenz und das Bereitstellen eines Warnhinweises bei Abweichungen kann ein zuverlässiger und sicherer Betrieb der Röntgeneinheit gewährleistet werden. Mögliche Probleme und/oder suboptimale Betriebszustände können frühzeitig erkannt und behoben werden, was eine Bildqualität und Lebensdauer der Röntgeneinheit verbessern kann.

Die Erfindung betrifft in einem dritten Aspekt ein Verfahren zur Kalibrierung einer vorgeschlagenen Röntgeneinheit. In einem ersten Schritt a) werden eine Absorptionsmaske zwischen dem Strahlenaustrittsfenster der Röntgeneinheit und einer Röntgenerfassungseinheit positioniert. In einem weiteren Schritt b) wird eine Hochspannung zwischen der Kathode und dem Anodenteller bereitgestellt. In einem weiteren Schritt c) erfolgt eine Bestromung der Kathode zum Bereitstellen des Elektronenstrahls. In einem weiteren Schritt d) wird der Anodenteller mit einer Rotationsfrequenz innerhalb eines vorgegebenen Rotationsfrequenzbereichs rotiert. In einem weiteren Schritt e) erfolgt eine Bestromung der Spule mit einem Ablenkstrom zum Bereitstellen des Magnetfelds. In einem weiteren Schritt f) wird eine Position einer Abbildung der Absorptionsmaske mittels der Röntgenerfassungseinheit erfasst. Die Schritte b) bis f) werden für eine Vielzahl verschiedener Hochspannungen und/oder Ablenkströme wiederholt ausgeführt. Eine Zuordnung zwischen der Ablenkung des Elektronenstrahls und dem Ablenkstrom und/oder zwischen der Ablenkung des Elektronenstrahls und der Hochspannung wird basierend auf der Vielzahl verschiedener Hochspannungen und/oder Ablenkströme und den zugehörigen Positionen der Abbildung identifiziert. In einem weiteren Schritt wird eine die Zuordnung charakterisierende Kalibrierinformation bereitgestellt.

Die vorstehend beschriebenen Schritte können dabei vorteilhafterweise zumindest teilweise, insbesondere vollständig, gleichzeitig oder nacheinander ausgeführt werden. Zudem kann eine Reihenfolge der Ausführung der vorstehend beschriebenen Schritte b) bis e) variiert werden, beispielsweise kann das Rotieren des Anodentellers vor dem Bestromen der Kathode ausgeführt werden.

Die Vorteile des vorgeschlagenen Verfahrens zur Kalibrierung einer Röntgeneinheit entsprechen im Wesentlichen den Vorteilen der vorgeschlagenen Röntgeneinheit und/oder des vorgeschlagenen Verfahrens zum Betrieb einer Röntgeneinheit. Hierbei erwähnte Merkmale, Vorteile oder alternative Ausführungsformen können ebenso auch auf die anderen beanspruchten Gegenstände übertragen werden und umgekehrt.

Die Absorptionsmaske kann als eine Struktur ausgebildet sein, die Röntgenstrahlung teilweise absorbiert. Die Absorptionsmaske kann beispielsweise ein röntgenabsorbierendes Material, insbesondere Blei oder einer Bleilegierung, umfassen. Die Absorptionsmaske kann Muster und/oder Strukturen aufweisen, insbesondere aus dem röntgenabsorbierenden Material, welche dazu ausgebildet sind, eine definierte Abbildung auf der Röntgenerfassungseinheit zu erzeugen. Diese Abbildung kann als eine Art Schattenwurf der Absorptionsmaske verstanden werden, der durch die Röntgenstrahlung erzeugt wird. Eine Position und/oder Form dieser Abbildung auf der Röntgenerfassungseinheit kann dann zur Kalibrierung der Röntgeneinheit verwendet werden.

Das Positionieren der Absorptionsmaske kann ein, insbesondere gezieltes, Platzieren und/oder Ausrichten der Absorptionsmaske zwischen dem Strahlenaustrittsfenster der Röntgeneinheit und der Röntgenerfassungseinheit umfassen. Das Positionieren der Absorptionsmaske kann manuell oder durch automatisierte Mechanismen erfolgen. Zudem kann das Positionieren der Absorptionsmaske eine Feinjustierung in verschiedenen Freiheitsgraden, beispielsweise eine Translation und/oder Rotation der Absorptionsmaske, umfassen. Die Absorptionsmaske wird zwischen dem Strahlenaustrittsfenster der Röntgeneinheit und der Röntgenerfassungseinheit positioniert, insbesondere in einem von der Röntgenstrahlung beleuchteten Bereich.

Die Röntgenstrahlung, die vom Strahlenaustrittsfenster emittiert wird, kann die Absorptionsmaske beleuchten, insbesondere zumindest teilweise durchdringen. Dabei kann ein Teil der Röntgenstrahlung von der Absorptionsmaske absorbiert werden, während ein übriger Teil der Röntgenstrahlung transmittiert wird.

Die Schritte b) bis e) des vorgeschlagenen Verfahrens zur Kalibrierung einer Röntgeneinheit, insbesondere das Bereitstellen der Hochspannung zwischen der Kathode und dem Anodenteller, die Bestromung der Kathode zum Bereitstellen des Elektronenstrahls, das Rotieren des Anodentellers mit der Rotationsfrequenz innerhalb des vorgegebenen Rotationsfrequenzbereichs und die Bestromung der Spule mit dem Ablenkstrom zum Bereitstellen des Magnetfelds, können insbesondere analog zu den entsprechenden Schritten des vorgeschlagenen Verfahrens zum Betrieb einer Röntgeneinheit ausgeführt werden.

Eine Position einer Abbildung der Absorptionsmaske kann mittels der Röntgenerfassungseinheit erfasst werden. Das Erfassen der Position kann ein Detektieren und Lokalisieren der Abbildung auf der Röntgenerfassungseinheit, insbesondere einer röntgensensitiven Schicht der Röntgenerfassungseinheit, umfassen. Die Röntgenerfassungseinheit kann als ein Gerät ausgebildet sein, das dazu ausgebildet ist, Röntgenstrahlung in ein elektrisches Signal und/oder ein Bild umzuwandeln.

Die Schritte b) bis f) können für eine Vielzahl verschiedener Hochspannungen und/oder Ablenkströme wiederholt ausgeführt werden. Das wiederholte Ausführen kann ein mehrfaches Durchlaufen der Schritte mit unterschiedlichen Parametern umfassen. Die Vielzahl kann beispielsweise 5, 10, 20 oder mehr verschiedene Kombinationen von Hochspannungen und Ablenkströmen umfassen.

Die Variation der Hochspannung und/oder des Ablenkstroms kann spezifische Auswirkungen auf die Position der Abbildung der Absorptionsmaske auf der Röntgenerfassungseinheit haben.

Durch eine Erhöhung der Hochspannung zwischen Kathode und Anodenteller kann die Geschwindigkeit der Elektronen im Elektronenstrahl erhöht werden. Dies kann dazu führen, dass die Elektronen weniger stark durch das Magnetfeld abgelenkt werden. Folglich kann sich die Position der Abbildung der Absorptionsmaske auf der Röntgenerfassungseinheit mit zunehmender Hochspannung, insbesondere bei gleichem Ablenkstrom, in Richtung einer Nullposition, insbesondere einer Position in der der Elektronenstrahl nicht ausgelenkt ist, verschieben.

Durch eine Verringerung der Hochspannung zwischen Kathode und Anodenteller kann die Geschwindigkeit der Elektronen im Elektronenstrahl verringert werden. Hierdurch kann es zu einer stärkeren Ablenkung der langsameren Elektronen durch das Magnetfeld kommen. Somit kann einer Verringerung der Hochspannung, insbesondere bei gleichem Ablenkstrom, eine Verschiebung der Abbildung der Absorptionsmaske weiter weg von der Nullposition bewirken.

Vorteilhafterweise kann die Bestromung der Kathode in Abhängigkeit der Hochspannung angepasst werden, insbesondere derart, dass eine Leistung, insbesondere ein Produkt aus Hochspannung und Stromstärke eines Röhrenstroms als Folge der Bestromung der Kathode, im Wesentlichen konstant bleibt, beispielsweise eine Leistung 5 W beträgt. Beispielsweise kann die Stromstärke der Bestromung der Kathode bei einer Erhöhung der Hochspannung zwischen Kathode und Anodenteller entsprechend verringert werden.

Der Röhrenstrom und die Bestromung der Kathode können in einem engen Zusammenhang zueinander stehen. Die Bestromung der Kathode, auch als Heizstrom bezeichnet, kann ein Bereitstellen eines elektrischen Stroms umfassen, der durch die Kathode, insbesondere einen Emitter, beispielsweise einen Glühfaden oder ein Emitterblech, fließt. Dieser Heizstrom kann die Kathode auf hohe Temperaturen, typischerweise im Bereich von 1800°C bis 2400°C, erhitzen, was zur thermischen Emission von Elektronen führen kann.

Der Röhrenstrom hingegen kann sich auf den Strom der Elektronen beziehen, die von der Kathode zur Drehanode fließen. Dieser Strom kann durch die Hochspannung zwischen Kathode und Anode erzeugt und gesteuert werden. Die Menge der emittierten Elektronen und damit der Röhrenstrom können direkt von der Temperatur der Kathode abhängen, welche wiederum durch die Bestromung der Kathode bestimmt werden kann.

Es kann eine nichtlineare Beziehung zwischen der Bestromung der Kathode und dem resultierenden Röhrenstrom bestehen. Bei höherer Bestromung der Kathode kann die Temperatur steigen, was zu einer erhöhten Elektronenemission und somit zu einem höheren Röhrenstrom führen kann. Diese Beziehung kann in etwa dem Richardson-Gesetz folgen, welches die Elektronenemission in Abhängigkeit von der Temperatur beschreibt.

In modernen Röntgenröhren kann der Röhrenstrom durch präzise Steuerung der Bestromung der Kathode reguliert werden. Typischerweise kann der Heizstrom für die Kathode im Bereich von 10 mA bis 20000 mA liegen, während der resultierende Röhrenstrom je nach Anwendung zwischen 0,1 mA und 1000 mA variieren kann.

Eine Änderung des Ablenkstroms in der Spule kann direkt die Stärke des erzeugten Magnetfelds beeinflussen. Ein höherer Ablenkstrom kann ein stärkeres Magnetfeld erzeugen, was zu einer größeren Ablenkung des Elektronenstrahls führen kann. Eine Erhöhung des Ablenkstroms kann, insbesondere bei gleicher Hochspannung, eine Verschiebung der Abbildung der Absorptionsmaske weiter weg von der Nullposition bewirken. Eine Verringerung des Ablenkstroms kann, insbesondere bei gleicher Hochspannung, eine Verschiebung der Abbildung der Absorptionsmaske in Richtung der Nullposition bewirken.

Die Veränderungen in der Position der Abbildung können in verschiedenen Richtungen erfolgen, abhängig von der Orientierung des Magnetfelds und einer Geometrie der Röntgeneinheit. Die Verschiebungen können in horizontaler und/oder vertikaler Richtung auf der Röntgenerfassungseinheit, insbesondere der röntgensensitiven Schicht der Röntgenerfassungseinheit, auftreten.

Eine Zuordnung zwischen der Ablenkung des Elektronenstrahls und dem Ablenkstrom und/oder zwischen der Ablenkung des Elektronenstrahls und der Hochspannung kann basierend auf der Vielzahl verschiedener Hochspannungen und/oder Ablenkströme und den zugehörigen Positionen der Abbildung identifiziert werden. Das Identifizieren der Zuordnung kann ein Analysieren und Korrelieren der erfassten Daten umfassen, um eine Beziehung zwischen den Parametern herzustellen.

Durch eine systematische Variation der Hochspannung und/oder des Ablenkstroms kann eine detaillierte Karte der erfassten Positionen der Abbildung der Absorptionsmaske erstellt werden. Diese Karte kann als Grundlage für die Kalibrierung der Röntgeneinheit dienen und eine präzise Steuerung der Elektronenstrahlablenkung in verschiedenen Betriebszuständen ermöglichen.

Eine die Zuordnung charakterisierende Kalibrierinformation kann bereitgestellt werden. Das Bereitstellen der Kalibrierinformation kann ein Generieren und Speichern von Daten umfassen, die die identifizierte Zuordnung beschreiben. Die Kalibrierinformation kann beispielsweise als eine Tabelle, eine mathematische Funktion und/oder ein Algorithmus ausgebildet sein.

Das Verfahren zur Kalibrierung einer Röntgeneinheit kann den Vorteil bieten, dass eine präzise Steuerung der Elektronenstrahlablenkung in Abhängigkeit von verschiedenen Betriebsparametern ermöglicht wird. Dadurch können eine verbesserte Bildqualität und eine genauere Positionierung des Brennflecks erreicht werden.

In einer weiteren vorteilhaften Ausführungsform des vorgeschlagenen Verfahrens zur Kalibrierung einer Röntgeneinheit kann die Röntgenerfassungseinheit eine Röntgenkamera und/oder einen Röntgendetektor umfassen.

Die Röntgenkamera kann als bildgebendes Gerät ausgebildet sein, welches dazu ausgebildet ist, einfallende Röntgenstrahlung in sichtbares Licht umzuwandeln und dieses aufzuzeichnen. Die Röntgenkamera kann beispielsweise einen Szintillator umfassen, der die Röntgenstrahlung in sichtbares Licht umwandelt, sowie eine lichtempfindliche Kamera zur Aufnahme des sichtbaren Lichts. Als Szintillator kann beispielsweise Cäsiumiodid oder Gadoliniumoxysulfid verwendet werden. Die Röntgenkamera kann vorteilhafterweise einen optischen Sensor, insbesondere einen CCD- oder CMOS-Sensor, zur Detektion des vom Szintillator erzeugten sichtbaren Lichts aufweisen.

Vorteilhafterweise kann die Röntgenkamera einen CCD-Sensor aufweisen. Bei Verwendung eines CCD-Sensors kann die Röntgenkamera eine hohe Empfindlichkeit und ein geringes Rauschen aufweisen. Der CCD-Sensor kann das vom Szintillator emittierte Licht in elektrische Ladungen umwandeln, die dann ausgelesen und in digitale Signale konvertiert werden können.

Alternativ kann die Röntgenkamera einen CMOS-Sensor aufweisen. CMOS-Sensoren können eine schnellere Auslesegeschwindigkeit und einen geringeren Stromverbrauch im Vergleich zu CCD-Sensoren aufweisen. Der CMOS-Sensor kann dazu ausgebildet sein, das vom Szintillator erzeugte Licht direkt in elektrische Signale umzuwandeln.

Zudem kann die Röntgenkamera eine Optik zwischen dem Szintillator und dem Sensor, insbesondere dem CCD- oder CMOS-Sensor, aufweisen. Diese Optik kann dazu ausgebildet sein, das vom Szintillator erzeugte Lichtbild auf den Sensor abzubilden und gegebenenfalls zu vergrößern oder zu verkleinern. Die Optik kann beispielsweise mehrere Linsen und/oder einen Fasertaper umfassen. Die Röntgenkamera kann in einem vordefinierten Abstand oder direkt angrenzend bezüglich der Röntgeneinheit, insbesondere des Strahlenaustrittsfensters, angeordnet sein.

Ein Auslesen des Sensors, insbesondere des CCD- oder CMOS-Sensors, kann durch eine spezielle Elektronik gesteuert werden, die es ermöglicht, verschiedene Belichtungszeiten und/oder Auslesemodi einzustellen. Die Röntgenkamera kann ferner einen Bildverstärker zwischen Szintillator und Sensor umfassen, der dazu ausgebildet ist, eine Lichtausbeute zu erhöhen.

Der Röntgendetektor kann dazu ausgebildet sein, Röntgenstrahlung direkt in elektrische Signale umzuwandeln. Der Röntgendetektor kann beispielsweise einen Halbleiterdetektor umfassen, der dazu ausgebildet ist, die Röntgenstrahlung direkt in elektrische Ladungen umzuwandeln. Das Halbleitermaterial kann beispielsweise amorphes Selen, Cadmiumtellurid und/oder Silizium umfassen. Alternativ kann der Röntgendetektor mehrere Schichten umfassen. Dabei kann die erste Schicht einen Szintillator umfassen, der dazu ausgebildet sein kann, einfallende Röntgenstrahlung in sichtbares Licht umzuwandeln. Als Szintillatormaterial können beispielsweise Cäsiumiodid oder Gadoliniumoxysulfid verwendet werden. Bezüglich einer Einfallsrichtung der Röntgenstrahlung kann hinter der Schicht mit dem Szintillator eine weitere Schicht, umfassend eine Vielzahl von Detektorelementen, angeordnet sein. Die Detektorelemente können jeweils als Photodioden ausgebildet sein. Die Detektorelemente, insbesondere die Photodioden, können dazu ausgebildet sein, das vom Szintillator erzeugte Licht in elektrische Signale umzuwandeln. Die Photodioden können beispielsweise in einer räumlichen Matrix-Anordnung platziert sein, um eine flächige Detektion zu ermöglichen. Eine Ausleseelektronik kann mit den Detektorelementen verbunden sein. Die Ausleseelektronik kann dazu ausgebildet sein, die von den Photodioden erzeugten elektrischen Signale auszulesen und zu digitalisieren. Die digitalisierten Signale können dann zur weiteren Verarbeitung an eine Steuerungseinheit übertragen werden. Insbesondere kann der Röntgendetektor als integrierender Röntgendetektor ausgebildet sein. Der integrierende Röntgendetektor kann dazu ausgebildet sein, die einfallende Röntgenstrahlung über einen bestimmten Zeitraum, insbesondere einen Integrationszeitraum, zu akkumulieren. Dabei können die Detektorelemente die durch die Röntgenstrahlung erzeugten elektrischen Ladungen speichern. Am Ende des Integrationszeitraums kann die gesamte akkumulierte Ladung ausgelesen werden.

Der Röntgendetektor kann beispielsweise als Flachdetektor ausgebildet sein. Der Flachdetektor kann eine Vielzahl von Detektorelementen umfassen, welche jeweils dazu ausgebildet sein können, die einfallende Röntgenstrahlung in elektrische Signale umzuwandeln.

Die Röntgeneinheit und der Röntgendetektor können in definierter Anordnung und in Gegenüberstellung zueinander angeordnet sein. Vorteilhafterweise kann der Röntgendetektor beabstandet von der Röntgeneinheit angeordnet sein, insbesondere derart beabstandet, dass ein Untersuchungsobjekt zwischen der Röntgeneinheit und dem Röntgendetektor anordbar ist.

Die Ablenkung des Elektronenstrahls kann die Position der Abbildung der Absorptionsmaske direkt beeinflussen. Wenn der Elektronenstrahl abgelenkt wird, verschiebt sich der Brennfleck auf dem Anodenteller, was wiederum zu einer Verschiebung der Röntgenstrahlung führen kann. Diese Verschiebung der Röntgenstrahlung kann in einer entsprechenden Verschiebung der Abbildung der Absorptionsmaske auf der Röntgenerfassungseinheit resultieren.

Der Zusammenhang zwischen der Ablenkung des Elektronenstrahls und der Position der Abbildung kann als nahezu linear betrachtet werden. Eine Ablenkung des Elektronenstrahls um einen bestimmten Winkel bezüglich einer Nullposition kann zu einer proportionalen Verschiebung der Abbildung der Absorptionsmaske führen. Der genaue Zusammenhang zwischen der Ablenkung des Elektronenstrahls und der Position der Abbildung kann durch geometrische Berechnungen unter Berücksichtigung eines Abstands zwischen dem Anodenteller und der Röntgenerfassungseinheit sowie des Anodenwinkels bestimmt werden.

In einer weiteren vorteilhaften Ausführungsform des vorgeschlagenen Verfahrens zur Kalibrierung einer Röntgeneinheit können für die wiederholte Ausführung der Schritte b) bis f) jeweils zumindest 4 verschiedene Hochspannungen und/oder Ablenkströme vorgegeben werden.

Die Hochspannungen können vorteilhafterweise in einem Bereich von 10 kV bis 300 kV vorgegeben werden. Insbesondere können für die wiederholte Ausführung der Schritte b) bis f) 23 kV, 28 kV, 35 kV und 40 kV als Hochspannungen vorgegeben werden. Dabei können ein konstanter Ablenkstrom oder verschiedene Ablenkströme vorgegeben werden. Insbesondere kann ein konstanter Ablenkstrom von 10 A vorgegeben werden. Ferner können dabei jeweilige Stromstärken des Röhrenstroms als Folge der Bestromung der Kathode, insbesondere des Heizstroms der Kathode, vorgegeben werden, beispielsweise 217 mA für eine Hochspannung von 23 kV, 175 mA für eine Hochspannung von 28 kV, 142 mA für eine Hochspannung von 35 kV und 125 mA für eine Hochspannung von 40 kV.

Die Ablenkströme können beispielsweise in einem Bereich von 0 A bis 10 A vorgegeben werden. Dabei können eine konstante Hochspannung oder verschiedene Hochspannungen vorgegeben werden.

Das Vorgeben von zumindest 4 verschiedenen Hochspannungen und/oder Ablenkströmen kann eine ausreichende Anzahl von Messpunkten bereitstellen, um eine zuverlässige Zuordnung zwischen der Ablenkung des Elektronenstrahls und dem Ablenkstrom und/oder zwischen der Ablenkung des Elektronenstrahls und der Hochspannung zu ermöglichen. Eine höhere Anzahl von Messpunkten kann eine Genauigkeit der Zuordnung weiter verbessern.

Die wiederholte Ausführung der Schritte b) bis f) kann für jede Kombination der verschiedenen Hochspannungen und Ablenkströme durchgeführt werden. Alternativ kann die wiederholte Ausführung für eine Auswahl von Kombinationen durchgeführt werden, die als repräsentativ für den gesamten Betriebsbereich der Röntgeneinheit angesehen werden.

Das Vorgeben der verschiedenen Hochspannungen und/oder Ablenkströme kann automatisch durch eine Steuerungseinheit der Röntgeneinheit oder des Bildgebungsgeräts erfolgen. Alternativ kann das Vorgeben manuell durch einen Bediener durchgeführt werden.

Die Verwendung von zumindest 4 verschiedenen Hochspannungen und/oder Ablenkströmen kann vorteilhafterweise eine umfassende Charakterisierung des Verhaltens der Röntgeneinheit über einen breiten Betriebsbereich ermöglichen. Dies kann zu einer genaueren Kalibrierung und damit zu einer verbesserten Bildqualität und Positioniergenauigkeit des Brennflecks führen.

In einer weiteren vorteilhaften Ausführungsform des vorgeschlagenen Verfahrens zur Kalibrierung einer Röntgeneinheit kann das Identifizieren der Zuordnung zwischen der Ablenkung des Elektronenstrahls und dem Ablenkstrom und/oder zwischen der Ablenkung des Elektronenstrahls und der Hochspannung des Elektronenstrahls eine jeweilige Interpolation umfassen.

Eine Interpolation kann als ein mathematisches Verfahren zur Schätzung von Zwischenwerten innerhalb einer Reihe von bekannten Datenpunkten verstanden werden. Im Kontext des vorgeschlagenen Verfahrens zur Kalibrierung einer Röntgeneinheit kann die Interpolation ein Bestimmen einer Ablenkung des Elektronenstrahls für einen Ablenkstrom und/oder eine Hochspannung ermöglichen, die nicht direkt gemessen wurden.

Dabei kann eine lineare Interpolation verwendet werden, bei der angenommen wird, dass die Beziehung zwischen den gemessenen Datenpunkten linear ist. Alternativ kann eine polynomiale Interpolation verwendet werden, die eine komplexere Beziehung zwischen den Datenpunkten modelliert. Weitere vorteilhafte Interpolationsmethoden umfassen eine kubische Spline-Interpolation und/oder eine Lagrange-Interpolation.

Durch das Identifizieren der Zuordnung mittels Interpolation kann vorteilhafterweise eine kontinuierliche Funktion für die Beziehung zwischen der Ablenkung des Elektronenstrahls und dem Ablenkstrom und/oder der Hochspannung erstellt werden. Dies kann eine präzisere Steuerung der Ablenkung des Elektronenstrahls ermöglichen, auch für Werte, die zwischen direkt gemessenen Kalibrierpunkten liegen. Dabei kann ein Kalibrierpunkt jeweils einen vorgegebenen Ablenkstrom oder eine vorgegebene Hochspannung und eine zugehörige Ablenkung des Elektronenstrahls umfassen.

Das Ergebnis der Interpolation kann als Lookup-Tabelle und/oder als mathematische Funktion gespeichert werden, um während des Betriebs der Röntgeneinheit einen schnellen Zugriff auf die Zuordnung zu ermöglichen.

Die interpolierte Zuordnung kann verwendet werden, um für beliebige Werte des Ablenkstroms und/oder der Hochspannung die entsprechende Ablenkung des Elektronenstrahls zu bestimmen. Dies ermöglicht eine präzise Steuerung der Ablenkung auch für Betriebszustände, die nicht exakt den während der Kalibrierung gemessenen Punkten entsprechen. Umgekehrt kann die interpolierte Zuordnung auch dazu verwendet werden, den erforderlichen Ablenkstrom und/oder die erforderliche Hochspannung für eine gewünschte Ablenkung des Elektronenstrahls zu ermitteln. Dies kann für eine gezielte Positionierung des Brennflecks auf dem Anodenteller genutzt werden.

Die Verwendung einer Interpolation für die Zuordnung kann mehrere technische Vorteile bieten. Zum einen kann die Anzahl der während der Kalibrierung erforderlichen Messpunkte reduziert werden, was den Kalibrierungsprozess beschleunigt. Zum anderen ermöglicht die kontinuierliche Zuordnung eine feinere und präzisere Steuerung der Ablenkung des Elektronenstrahls, was zu einer verbesserten Bildqualität und einer genaueren Positionierung des Brennflecks führen kann.

Die Erfindung betrifft in einem vierten Aspekt ein Bildgebungsgerät. Das Bildgebungsgerät umfasst eine vorgeschlagene Röntgeneinheit, einen Röntgendetektor und eine Steuerungseinheit. Die Röntgeneinheit und der Röntgendetektor sind in Gegenüberstellung zueinander angeordnet. Der Röntgendetektor ist zur Erfassung von mittels der Röntgeneinheit emittierbarer Röntgenstrahlung ausgebildet. Die Röntgeneinheit ist beweglich bezüglich des Röntgendetektors gelagert. Die Steuerungseinheit ist dazu ausgebildet, eine Bestromung der Spule in Abhängigkeit einer momentanen Positionierung der Röntgeneinheit derart anzupassen, dass der von der Kathode aussendbare Elektronenstrahl mittels des Magnetfelds zur Minimierung einer Bewegung des Brennflecks bezüglich des Röntgendetektors abgelenkt wird.

Die Vorteile des vorgeschlagenen Bildgebungsgeräts entsprechen im Wesentlichen den Vorteilen der vorgeschlagenen Röntgeneinheit, des vorgeschlagenen Verfahrens zum Betrieb einer Röntgeneinheit und/oder des vorgeschlagenen Verfahrens zur Kalibrierung einer Röntgeneinheit. Hierbei erwähnte Merkmale, Vorteile oder alternative Ausführungsformen können ebenso auch auf die anderen beanspruchten Gegenstände übertragen werden und umgekehrt.

Die Röntgeneinheit und der Röntgendetektor sind in Gegenüberstellung zueinander angeordnet. Vorteilhafterweise können dabei das Strahlenaustrittsfenster, insbesondere die Blendeneinheit, der Röntgeneinheit und eine röntgensensitive Schicht, insbesondere eine röntgensensitive Oberfläche, des Röntgendetektors einander zugewandt angeordnet sein. Vorteilhafterweise können die Röntgeneinheit und der Röntgendetektor derart in Gegenüberstellung zueinander angeordnet sein, dass die von der Röntgeneinheit aussendbare Röntgenstrahlung eine röntgensensitive Schicht, insbesondere eine röntgensensitive Oberfläche, des Röntgendetektors beleuchten kann, insbesondere nach Wechselwirkung mit einem zwischen der Röntgeneinheit und dem Röntgendetektor angeordneten Untersuchungsobjekt.

Des Weiteren können die Röntgeneinheit und der Röntgendetektor in einer definierten Anordnung zueinander angeordnet sein.

Der Röntgendetektor kann dazu ausgebildet sein, die von der Röntgeneinheit emittierbare Röntgenstrahlung zu empfangen und in elektrische Signale umzuwandeln. Der Röntgendetektor kann eine Vielzahl von Detektorelementen aufweisen, welche in einer Matrix-Anordnung angeordnet sein können. Diese Detektorelemente können aus Materialien wie amorphem Silizium, amorphem Selen und/oder Cadmiumtellurid gefertigt sein, welche eine hohe Empfindlichkeit für Röntgenstrahlung aufweisen. Der Röntgendetektor kann beispielsweise als Flachdetektor und/oder Zeilendetektor und/oder Mehrzeilendetektor ausgebildet sein.

Der Röntgendetektor kann eine Szintillatorschicht aufweisen, welche zur Umwandlung der einfallenden Röntgenstrahlung in sichtbares Licht ausgebildet ist. Ferner kann der Röntgendetektor Photosensoren, beispielsweise Photodioden, aufweisen, welche dazu ausgebildet sind, das von der Szintillatorschicht bereitgestellte Licht zu erfassen und in elektrische Signale umzuwandeln. Die Szintillatorschicht kann beispielsweise Cäsiumiodid oder Gadoliniumoxysulfid aufweisen.

Um eine hohe Empfindlichkeit zu gewährleisten, kann der Röntgendetektor eine Ausleseelektronik umfassen, die eine schnelle und rauscharme Signalverarbeitung ermöglicht. Der Röntgendetektor kann zudem ein Kühlsystem umfassen, um ein thermisches Rauschen zu reduzieren und die Bildqualität zu verbessern. Das Kühlsystem kann beispielsweise Peltier-Elemente und/oder eine Flüssigkeitskühlung umfassen.

In einer weiteren Ausführungsform kann der Röntgendetektor als energieauflösender Röntgendetektor ausgebildet sein. Der energieauflösende Röntgendetektor kann dazu ausgebildet sein, zusätzlich zu einer Intensität auch eine Energie der einfallenden Röntgenstrahlung, insbesondere der einfallenden Röntgenphotonen, zu erfassen. Hierfür kann der Röntgendetektor eine Halbleiterschicht, insbesondere umfassend Cadmiumtellurid und/oder Cadmiumzinktellurid, aufweisen, welche dazu ausgebildet ist, Röntgenstrahlung, insbesondere Röntgenphotonen, direkt in elektrische Signale umzuwandeln.

In einer weiteren Ausführungsform kann der Röntgendetektor als Mehrschichtdetektor ausgebildet sein. Der Mehrschichtdetektor kann mehrere übereinander angeordnete Detektorschichten umfassen, die jeweils zur Erfassung eines verschiedenen Energiebereichs der Röntgenstrahlung ausgebildet, insbesondere optimiert, sein können.

Eine, insbesondere bezüglich einer Strahleneinfallsrichtung, obere Detektorschicht des Mehrschichtdetektors kann beispielsweise amorphes Silizium oder amorphes Selen umfassen. Diese Detektorschicht kann besonders empfindlich für niederenergetische Röntgenstrahlung sein und kann insbesondere eine Dicke von etwa 200 bis 500 Mikrometern aufweisen. Eine, insbesondere bezüglich der Strahleneinfallsrichtung, untere Detektorschicht kann beispielsweise Cäsiumiodid umfassen. Diese Detektorschicht kann insbesondere eine Dicke von etwa 200 bis 500 Mikrometern aufweisen und kann für die Detektion von Röntgenstrahlung im höheren Energiebereich optimiert sein.

Zwischen den einzelnen Detektorschichten können dünne Filterschichten aus Materialien wie beispielsweise Kupfer und/oder Aluminium angeordnet sein. Diese Filterschichten können dazu ausgebildet sein, ein Energiespektrum der Röntgenstrahlung für die jeweils darunterliegende Detektorschicht zu modifizieren.

Die Verwendung eines Mehrschichtdetektors kann eine verbesserte Energieauflösung und einen erweiterten Dynamikbereich im Vergleich zu herkömmlichen Einzelschichtdetektoren ermöglichen.

Der Röntgendetektor kann ferner eine Streustrahlenreduktionsvorrichtung umfassen, insbesondere um ein Signal-Rausch-Verhältnis zu verbessern. Die Streustrahlenreduktionsvorrichtung kann ein Streustrahlenraster umfassen. Alternativ oder zusätzlich kann die Streustrahlenreduktionsvorrichtung zur Anwendung softwarebasierter Streustrahlenkorrekturalgorithmen auf die bereitgestellten elektrischen Signale ausgebildet sein.

Die Szintillator- oder Halbleiterschicht des Röntgendetektors kann die röntgensensitive Schicht des Röntgendetektors bilden.

Die Röntgeneinheit ist beweglich, insbesondere translatierbar und/oder rotierbar, bezüglich des Röntgendetektors gelagert. Die translatierbare Lagerung kann beispielsweise mittels eines Linearführungssystems implementiert sein. Das Linearführungssystem kann Präzisionsschienen und Gleitlager umfassen, welche eine reibungsarme und präzise lineare Bewegung der Röntgeneinheit ermöglichen. Die Translation kann mittels eines Motors, beispielsweise einem Linearmotor oder einem Spindelantrieb, antreibbar sein. Das Bildgebungsgerät kann ferner eine Positionserfassungseinheit, beispielsweise ein Encoder-System, umfassen, welches zur genauen Positionsbestimmung einer momentanen Positionierung der Röntgeneinheit ausgebildet sein kann. Die rotierbare Lagerung kann beispielsweise mittels eines Drehgelenks und/oder eines Kugellagers implementiert sein. Die Rotation kann mittels eines Motors, beispielsweise eines Servomotors, antreibbar sein. Insbesondere kann der Motor über ein Getriebe mit einem Drehlager verbunden sein. Die Positionserfassungseinheit kann beispielsweise einen, insbesondere optischen und/oder magnetischen, Winkelencoder zur Winkelerfassung aufweisen.

Die bewegliche Lagerung der Röntgeneinheit kann zudem Dämpfungselemente aufweisen, die dazu ausgebildet sind, Vibrationen zu reduzieren und eine stabile Bildgebung zu ermöglichen. Die Dämpfungselemente können beispielsweise hydraulische und/oder pneumatische Dämpfer umfassen.

Für eine präzise Steuerung der Bewegung kann die Steuerungseinheit ferner dazu ausgebildet sein, die Bewegung der Röntgeneinheit, insbesondere in Echtzeit, anzupassen und mit einer Erfassung von Bilddaten, insbesondere Röntgenbilddaten, durch den Röntgendetektor zu synchronisieren.

Die bewegliche Lagerung der Röntgeneinheit kann so ausgelegt sein, dass sie verschiedene Aufnahmegeometrien ermöglicht, beispielsweise eine Tomosynthese und/oder Panoramaaufnahmen eines Untersuchungsobjekts. Vorteilhafterweise kann eine Bewegungsbahn, insbesondere Trajektorie, der Röntgeneinheit bezüglich des Röntgendetektors an spezifische Anforderungen der jeweiligen Bildgebungstechnik anpassbar sein. Insbesondere kann die Steuerungseinheit dazu ausgebildet sein, die Trajektorie der Röntgeneinheit anzupassen.

Die bewegliche Lagerung der Röntgeneinheit kann beispielsweise eine Bewegung der Röntgeneinheit in einer vertikalen Ebene ermöglichen Dabei kann die Trajektorie der Röntgeneinheit einer linearen Bahn folgen. Hierdurch kann eine Anpassung einer Positionierung der Röntgeneinheit an verschiedene Untersuchungsobjekte und/oder Patientenpositionen ermöglicht werden.

Für eine Anwendung im Rahmen einer Tomosynthese kann die Trajektorie der Röntgeneinheit bogenförmig verlaufen. Dabei kann die bogenförmige Trajektorie einen Winkelbereich von etwa ±25°, insbesondere ±7,5°, umfassen, wobei eine Drehachse der Trajektorie in einem vordefinierten Abstand bezüglich des Röntgendetektors, insbesondere einem geometrischen Mittelpunkt eines durchschnittlichen Untersuchungsobjekts, insbesondere einer durchschnittlichen Brust, angeordnet sein kann oder durch den Röntgendetektor, insbesondere parallel zu einer Oberfläche, insbesondere röntgensensitiven Schicht, des Röntgendetektors verlaufen kann. Die Röntgeneinheit kann in diskreten Schritten oder kontinuierlich bewegbar sein.

Vorteilhafterweise kann die Röntgeneinheit dazu ausgebildet sein, eine kombinierte Bewegung auszuführen. Dabei kann die Trajektorie der Röntgeneinheit beispielsweise eine gleichzeitige vertikale Translation und Rotation um eine horizontale Achse umfassen. Die Trajektorie kann hierbei spiralförmig oder helikal ausgebildet sein.

Die momentane Positionierung der Röntgeneinheit kann eine räumliche Position und/oder Ausrichtung und/oder Pose der Röntgeneinheit, insbesondere bezüglich des Röntgendetektors, beschreiben. Die Steuerungseinheit kann dazu ausgebildet sein, die momentane Positionierung der Röntgeneinheit zu erfassen, beispielsweise mittels eines Positionierungssensors. Alternativ oder zusätzlich kann die Röntgeneinheit mittels einer kalibrierten Bewegungseinheit bewegbar gelagert sein. Dabei kann die kalibrierte Bewegungseinheit zum Bereitstellen einer Positionierungsinformation aufweisend eine Information zur momentanen Positionierung der Röntgeneinheit an die Steuerungseinheit ausgebildet sein.

Die Steuerungseinheit ist dazu ausgebildet, in Abhängigkeit, insbesondere basierend auf, der momentanen Positionierung der Röntgeneinheit die Bestromung der Spule anzupassen. Das Anpassen der Bestromung der Spule in Abhängigkeit der momentanen Positionierung der Röntgeneinheit kann ein Bestimmen und Vorgeben, insbesondere Einstellen, des Ablenkstroms für die Bestromung der Spule umfassen. Hierfür kann die Steuerungseinheit dazu ausgebildet sein, auf eine vorgehaltene, insbesondere gespeicherte, Kalibrierinformation zurückzugreifen, welche eine Zuordnung zwischen der Ablenkung des Elektronenstrahls und dem Ablenkstrom charakterisiert. Die Kalibrierinformation kann beispielsweise mittels eines vorgeschlagenen Verfahrens zur Kalibrierung einer Röntgeneinheit bereitgestellt worden sein. Durch das Anpassen der Bestromung der Spule, insbesondere des Ablenkstroms, kann eine Stärke und/oder Richtung des von der Spule erzeugten Magnetfelds beeinflussbar sein.

Die Minimierung der Bewegung des Brennflecks bezüglich des Röntgendetektors kann eine Reduzierung oder Vermeidung einer relativen Verschiebung zwischen dem Brennfleck und dem Röntgendetektor umfassen. Dies kann vorteilhaft sein, um eine gleichbleibende Bildqualität bei der Aufnahme von Bilddaten, insbesondere Röntgenbilddaten, zu erzielen, insbesondere bei Aufnahmesequenzen mit bewegter Röntgeneinheit wie beispielsweise bei der Tomosynthese.

Die Steuerungseinheit kann dazu ausgebildet sein, die Bestromung der Spule, insbesondere den Ablenkstrom, in Abhängigkeit von einer relativen Bewegung zwischen der Röntgeneinheit und dem Röntgendetektor anzupassen, um eine Minimierung der Bewegung des Brennflecks zu erreichen. Eine Änderung des Ablenkstroms kann zu einer Veränderung des von der Spule erzeugten Magnetfelds führen, was wiederum die Position des Elektronenstrahls und somit des Brennflecks auf dem Anodenteller beeinflussen kann.

Bei einer Erhöhung des Ablenkstroms kann sich die Stärke des Magnetfelds vergrößern, was zu einer stärkeren Ablenkung des Elektronenstrahls führen kann. Dies kann eine Verschiebung des Brennflecks auf dem Anodenteller bewirken. Umgekehrt kann eine Verringerung des Ablenkstroms zu einer schwächeren Ablenkung und einer entgegengesetzten Verschiebung des Brennflecks führen.

Um eine relative Bewegung zwischen der Röntgeneinheit und dem Röntgendetektor zu kompensieren, kann der Ablenkstrom, insbesondere kontinuierlich, anpassbar sein. Bewegt sich beispielsweise die Röntgeneinheit in eine bestimmte Richtung, kann die Steuerungseinheit den Ablenkstrom derart verändern, dass der Brennfleck in eine entgegengesetzte Richtung verschoben wird. Die Anpassung des Ablenkstroms kann dabei proportional zu einer Geschwindigkeit und Richtung der relativen Bewegung erfolgen.

In komplexeren Bewegungsabläufen, wie sie bei der Tomosynthese auftreten können, kann eine dynamische Anpassung des Ablenkstroms erforderlich sein. Hierbei kann die Steuerungseinheit dazu ausgebildet sein, insbesondere in Echtzeit, die momentane Position der Röntgeneinheit zu erfassen und den Ablenkstrom entsprechend zu regulieren, um eine möglichst konstante Position des Brennflecks relativ zum Röntgendetektor zu gewährleisten.

Die genaue Anpassung des Ablenkstroms kann von verschiedenen Faktoren abhängen, wie der Geometrie der Röntgeneinheit, der Stärke des erzeugten Magnetfelds und der Energie des Elektronenstrahls. Eine präzise Kalibrierung, beispielsweise mittels eines vorgeschlagenen Verfahrens zur Kalibrierung einer Röntgeneinheit, und die Verwendung der bereitgestellten Kalibrierinformation können ein Ermitteln einer passenden Bestromung der Spule für jede Positionierung der Röntgeneinheit ermöglichen.

Durch diese adaptive Steuerung des Ablenkstroms kann eine Minimierung der Bewegung des Brennflecks erreicht werden.

In einer weiteren vorteilhaften Ausführungsform des vorgeschlagenen Bildgebungsgeräts kann das Bildgebungsgerät weiterhin eine Standeinheit, eine erste Halteeinheit und eine zweite Halteeinheit umfassen. Die erste Halteeinheit kann rotierbar gelagert an der Standeinheit angeordnet sein. Die Röntgeneinheit kann an der ersten Halteeinheit angeordnet sein. Die zweite Halteeinheit kann an der Standeinheit angeordnet sein. Der Röntgendetektor kann an der zweiten Halteeinheit angeordnet sein. Die erste und die zweite Halteeinheit können die Röntgeneinheit und den Röntgendetektor in Gegenüberstellung zueinander halten.

Die Standeinheit kann als, insbesondere stationäre oder bewegbare, Basis oder Grundgerüst des Bildgebungsgeräts ausgebildet sein. Die Standeinheit kann dazu ausgebildet sein, die anderen Komponenten des Bildgebungsgeräts zu tragen und/oder zu stützen. Insbesondere kann die Standeinheit dazu ausgebildet sein, die erste und zweite Halteeinheit zu tragen und zu stabilisieren. Die Standeinheit kann beispielsweise als Bodenstativ, Wandhalterung, Roboterarm oder Deckenhalterung ausgebildet sein.

Die erste Halteeinheit kann als, insbesondere bewegliche, Trägerstruktur, insbesondere Arm und/oder Ausleger, für die Röntgeneinheit ausgebildet sein. Insbesondere kann die erste Halteeinheit die Röntgeneinheit halten, insbesondere tragen. Dabei kann die Röntgenquelle an der ersten Halteeinheit befestigt sein. Die erste Halteeinheit kann rotierbar an der Standeinheit gelagert sein, um eine Bewegung der Röntgeneinheit zu ermöglichen. Die rotierbare Lagerung der ersten Halteeinheit an der Standeinheit kann eine Drehbewegung der Röntgeneinheit um eine oder mehrere Achsen ermöglichen. Dies kann eine flexible Positionierung der Röntgeneinheit relativ zum Röntgendetektor ermöglichen. Die rotierbare Lagerung kann beispielsweise mittels eines Drehgelenks, einer Drehachse und/oder eines Schwenkmechanismus realisiert sein.

Die zweite Halteeinheit kann als, insbesondere feste oder bewegliche, Trägerstruktur, insbesondere Säule, Arm oder Platte, für den Röntgendetektor ausgebildet sein. Insbesondere kann die zweite Halteeinheit den Röntgendetektor halten, insbesondere tragen. Dabei kann der Röntgendetektor an der zweiten Halteeinheit befestigt sein. Die zweite Halteeinheit kann an der Standeinheit angeordnet, insbesondere befestigt, sein, um den Röntgendetektor in einer definierten Position relativ zur Standeinheit zu halten. Die zweite Halteeinheit kann als verstellbare, insbesondere höhenverstellbare, Plattform ausgebildet sein. Zudem kann die zweite Halteeinheit eine Neigungsfunktion, insbesondere eine Neigung um eine horizontale Achse, aufweisen, um eine optimale Positionierung des Untersuchungsobjekts zu ermöglichen.

Das In-Gegenüberstellung-Zueinander-Halten der Röntgeneinheit und des Röntgendetektors durch die erste und zweite Halteeinheit kann eine definierte geometrische Anordnung der Röntgeneinheit relativ zum Röntgendetektor ermöglichen. Diese definierte Anordnung kann für eine präzise Bildgebung vorteilhaft sein. Die Anordnung der Röntgeneinheit an der ersten Halteeinheit und des Röntgendetektors an der zweiten Halteeinheit kann eine präzise Ausrichtung und Positionierung der beiden Komponenten zueinander ermöglichen. Die erste und zweite Halteeinheit können die Röntgeneinheit und den Röntgendetektor in Gegenüberstellung und in einer definierten Anordnung, insbesondere einer definierten geometrischen Beziehung, zueinander halten.

Die erste und die zweite Halteeinheit können aus stabilen und steifen Materialien wie Metall und/oder Verbundwerkstoffen gefertigt sein, um eine präzise Positionierung der Röntgeneinheit und des Röntgendetektors zu gewährleisten. Die Halteeinheiten können zudem mit Dämpfungselementen ausgestattet sein, um Vibrationen zu reduzieren.

Die rotierbare Lagerung der ersten Halteeinheit in Kombination mit der Anordnung der zweiten Halteeinheit kann verschiedene Aufnahmepositionen und -winkel ermöglichen, während die Gegenüberstellung von Röntgeneinheit und Röntgendetektor beibehalten wird. Dies kann eine flexible Bildgebung aus unterschiedlichen Perspektiven ermöglichen.

In einer Ausführungsform kann das Bildgebungsgerät als Mammographieeinheit ausgebildet sein. Die Mammographieeinheit kann speziell für die Untersuchung der weiblichen Brust konzipiert sein und eine hochauflösende Röntgenbildgebung ermöglichen.

Die Röntgeneinheit der Mammographieeinheit kann eine spezielle Röntgenröhre umfassen, die für die Anforderungen einer Brustbildgebung optimiert ist. Diese Röntgenröhre kann einen als Einwinkelteller ausgebildeten Anodenteller mit einem Winkel von 16° aufweisen, um eine optimale Strahlgeometrie für die Brustuntersuchung zu gewährleisten. Der Anodenteller kann aus einer Wolfram-Rhenium-Legierung gefertigt sein, um eine hohe Wärmebelastbarkeit und effiziente Röntgenstrahlenproduktion zu ermöglichen.

Die Spule der Röntgeneinheit kann als Luftspule ausgebildet sein und außerhalb des Vakuumgehäuses angeordnet sein. Diese Anordnung kann eine präzise Steuerung des Magnetfelds ermöglichen, ohne die Vakuumintegrität der Röntgenröhre zu beeinträchtigen. Die Spule kann so dimensioniert sein, dass sie eine Ablenkung des Elektronenstrahls um bis zu 2,5 mm bezüglich einer Nullposition auf dem Anodenteller ermöglicht, was eine flexible Anpassung des Brennflecks erlaubt.

Der Röntgendetektor der Mammographieeinheit kann als digitaler Flachdetektor ausgebildet sein, der eine hohe räumliche Auflösung von beispielsweise 50 bis 100 Mikrometern pro Pixel aufweisen kann.

Die erste Halteeinheit, an der die Röntgeneinheit befestigt ist, kann L- oder J-förmig ausgebildet sein. Die erste Halteeinheit kann eine Rotation der Röntgeneinheit um die Brust des Untersuchungsobjekts ermöglichen. Hierdurch können beispielsweise Tomosynthese-Aufnahmen der zu untersuchenden Brust ermöglicht werden. Die Rotationsbewegung der Röntgeneinheit kann in einem Winkelbereich von ±25° erfolgen, wobei die Steuerungseinheit die Bestromung der Spule, insbesondere kontinuierlich, anpasst, um die Position des Brennflecks relativ zum Röntgendetektor zu optimieren.

Die zweite Halteeinheit, an der der Röntgendetektor befestigt ist, kann als verstellbare Plattform ausgebildet sein. Diese Plattform kann in ihrer Höhe anpassbar sein, um sich an verschiedene Körpergrößen anzupassen. Zudem kann die zweite Halteeinheit eine Neigungsfunktion aufweisen, um eine optimale Positionierung der Brust zu ermöglichen.

Die Mammographieeinheit kann zudem eine spezielle Kompressionseinrichtung umfassen, welche an der zweiten Halteeinheit befestigt sein kann. Diese Kompressionseinrichtung kann dazu ausgebildet sein, die Brust des Untersuchungsobjekts während der Untersuchung zu komprimieren. Die Kompression kann eine Bildqualität verbessern, indem sie eine Dicke eines durchstrahlten Gewebes der Brust des Untersuchungsobjekts reduziert und Bewegungsartefakte minimiert.

Eine Brustwand, welche in der Mammographie eine wichtige Rolle spielt, kann sich auf einen Bereich des Untersuchungsobjekts beziehen, wo die Brust an einen Brustkorb ansetzt. Die Mammographieeinheit kann vorteilhafterweise derart ausgebildet sein, dass sie eine möglichst vollständige Abbildung der Brust des Untersuchungsobjekts bis zur Brustwand ermöglicht. Hierfür kann die Röntgeneinheit, insbesondere die Anodenachse, vorteilhafterweise einen Winkel kleiner 90°, insbesondere 84°, zu einer vertikalen Ebene der Standeinheit, insbesondere einer Vertikalen, aufweisen, was als "Eintankwinkel" bezeichnet wird. Insbesondere kann die Anodenachse einen Winkel zwischen 0° und 30°, insbesondere 6°, bezüglich der Blendenebene der Blendeneinheit aufweisen. Dieser Winkel kann dazu beitragen, eine gleichmäßigere Intensität der Röntgenstrahlung auf einer brustwandnahen Seite zu erreichen und einen Abstand von der Brustwand zum Brennfleck zu verkürzen.

Die Steuerungseinheit der Mammographieeinheit kann spezielle Algorithmen zur Bildverarbeitung und -rekonstruktion umfassen. Diese Algorithmen können dazu ausgebildet sein, eine Sichtbarkeit von Mikrokalzifikationen und anderen subtilen Anzeichen von Brustkrebs zu verbessern. Die Steuerungseinheit kann ferner Funktionen zur automatischen Belichtungssteuerung (engl. automatic exposure control, AEC) implementieren, um eine optimale Strahlendosis für jede individuelle Brust zu gewährleisten.

Insgesamt kann die beschriebene Mammographieeinheit durch die Kombination der präzisen Steuerung des Elektronenstrahls mittels der Spule, der optimierten Geometrie und der fortschrittlichen Bildverarbeitungstechniken eine hochwertige Brustbildgebung mit reduzierter Strahlendosis ermöglichen.

In einer weiteren vorteilhaften Ausführungsform des vorgeschlagenen Bildgebungsgeräts kann die erste Halteeinheit L- oder J-förmig ausgebildet sein und mittels eines ersten Schenkels an der Standeinheit angeordnet sein. Die Röntgeneinheit kann an einem weiteren Schenkel der ersten Halteeinheit angeordnet sein.

Die erste Halteeinheit kann L-förmig oder J-förmig ausgebildet sein. Eine L-förmige Ausbildung kann zwei im rechten Winkel zueinander angeordnete Schenkel umfassen. Eine J-förmige Ausbildung kann einen geraden Schenkel und einen gebogenen Schenkel umfassen. Die L- oder J-förmige Ausbildung kann eine kompakte Bauweise des Bildgebungsgeräts ermöglichen. Der erste Schenkel kann als der in einer vertikalen Ebene, insbesondere Schicht, angeordnete Teil der L- oder J-Form ausgebildet sein und zur Befestigung an der Standeinheit dienen. Der weitere Schenkel kann als der in einer horizontalen Ebene, insbesondere Schicht, angeordnete Teil der L- oder J-Form ausgebildet sein, insbesondere als gebogener Teil der J-Form.

Der erste Schenkel kann als ein Teil der ersten Halteeinheit ausgebildet sein, der zur Verbindung mit der Standeinheit dient. Der erste Schenkel kann beispielsweise als Arm in einer vertikalen Ebene, insbesondere Schicht, ausgebildet sein. Die Anordnung der ersten Halteeinheit mittels des ersten Schenkels an der Standeinheit kann eine lösbare oder feste Verbindung zwischen der ersten Halteeinheit und der Standeinheit umfassen. Die Anordnung des ersten Schenkels an der Standeinheit kann mittels verschiedener Befestigungsmechanismen erfolgen. Beispielsweise kann der erste Schenkel mittels Schraubverbindungen, Bolzenverbindungen, Schweißverbindungen und/oder Klemmverbindungen an der Standeinheit befestigt sein. Die Befestigung kann vorteilhafterweise beweglich ausgeführt sein, um eine Rotation, insbesondere eine Schwenkbewegung, der ersten Halteeinheit zu ermöglichen.

Der weitere Schenkel kann als ein zusätzlicher Teil der ersten Halteeinheit ausgebildet sein, der zur Aufnahme der Röntgeneinheit dient. Der weitere Schenkel kann beispielsweise als Arm in einer horizontalen Ebene, insbesondere Schicht, ausgebildet sein.

Die Anordnung der Röntgeneinheit an dem weiteren Schenkel der ersten Halteeinheit kann eine lösbare oder feste Verbindung zwischen der Röntgeneinheit und dem weiteren Schenkel umfassen. Die Anordnung der Röntgeneinheit kann mittels verschiedener Befestigungsmechanismen erfolgen, beispielsweise einer Schraubverbindung, Bolzenverbindung, Schweißverbindung und/oder Klemmverbindung. Die Positionierung der Röntgeneinheit an dem weiteren Schenkel kann so gewählt sein, dass die Röntgeneinheit in einer geeigneten Position und Ausrichtung zum Röntgendetektor steht.

Die L- oder J-förmige Ausbildung der ersten Halteeinheit mit der Anordnung der Röntgeneinheit an dem weiteren Schenkel kann vorteilhafterweise eine flexible Positionierung der Röntgeneinheit relativ zum Röntgendetektor ermöglichen. Dies kann eine Anpassung an verschiedene Untersuchungssituationen und Patientengrößen erleichtern. Der erste Schenkel kann eine stabile und bewegliche Verbindung zur Standeinheit bereitstellen, während der weitere Schenkel die Röntgeneinheit in einem definierten Abstand zur Standeinheit positionieren kann. Dies kann eine flexible Positionierung der Röntgeneinheit ermöglichen, ohne eine Stabilität des Gesamtsystems zu beeinträchtigen. Durch die Anordnung der Röntgeneinheit an dem weiteren Schenkel kann ein größerer Bewegungsfreiraum für die Röntgeneinheit geschaffen werden. Dies kann insbesondere bei der Durchführung von Tomosynthese-Aufnahmen oder anderen Aufnahmetechniken vorteilhaft sein, bei denen die Röntgeneinheit entlang eines Aufnahmepfads bewegt werden soll. Die L- oder J-förmige Ausbildung der ersten Halteeinheit kann zudem eine kompakte Bauweise des Bildgebungsgeräts ermöglichen. Der Platzbedarf des Geräts kann dadurch reduziert werden, was insbesondere in beengten Räumlichkeiten von Vorteil sein kann. Ein weiterer Vorteil dieser Ausführungsform kann darin bestehen, dass die L- oder J-förmige Struktur eine verbesserte Zugänglichkeit zu einem Untersuchungsbereich des Bildgebungsgeräts ermöglichen kann. Dies kann die Positionierung des Untersuchungsobjekts erleichtern und dem medizinischen Personal mehr Bewegungsfreiheit bei der Durchführung von Untersuchungen bieten.

In einer weiteren vorteilhaften Ausführungsform des vorgeschlagenen Bildgebungsgeräts kann die Röntgeneinheit parallel zu einer Ebene der Standeinheit beweglich gelagert sein. Die Ebene der Standeinheit kann insbesondere eine vertikale Ebene sein. Die Anodenachse kann einen vordefinierten Winkel bezüglich der Ebene der Standeinheit aufweisen. Der vordefinierte Winkel kann kleiner als 90° sein, insbesondere 84° betragen.

Eine vertikale Ebene kann als eine Ebene definiert werden, die senkrecht zur Erdoberfläche ausgerichtet ist. Die vertikale Ebene kann beispielsweise durch die Schwerkraftrichtung und eine horizontale Richtung aufgespannt werden. Dabei kann die vertikale Ebene der Standeinheit vorteilhafterweise an die Standeinheit angrenzen, die Standeinheit berühren oder die Standeinheit schneiden.

Die parallele Beweglichkeit der Röntgeneinheit zu der Ebene der Standeinheit kann eine Bewegung entlang einer Richtung parallel zu der Ebene ermöglichen. Die Röntgeneinheit kann parallel zu der vertikalen Ebene der Standeinheit beweglich gelagert sein. Bewegung der Röntgeneinheit kann eine Translation und/oder Rotation der Röntgeneinheit umfassen. Dabei können während der Bewegung der Röntgeneinheit vorteilhafterweise ein räumlicher Abstand zwischen der Röntgeneinheit und der vertikalen Ebene sowie ein Winkel zwischen der Anodenachse und der vertikalen Ebene konstant bleiben.

Die Ausbildung des vordefinierten Winkels kleiner als 90° kann verschiedene technische Vorteile bieten. Durch die Anordnung der Anodenachse in einem Winkel kleiner als 90° bezüglich der vertikalen Ebene der Standeinheit kann eine vorteilhafte Ausrichtung des von der Röntgeneinheit emittierten Röntgenstrahls erreicht werden. Durch die Neigung der Anodenachse gegenüber der vertikalen Ebene der Standeinheit kann eine verbesserte Strahlgeometrie der Röntgenstrahlung erreicht werden, welche zu einer gleichmäßigeren Verteilung der Röntgenstrahlung auf dem Röntgendetektor führen kann. Dies kann insbesondere bei einer Untersuchung von Brustgewebe vorteilhaft sein, da die gleichmäßigere Verteilung der Röntgenstrahlung auf dem Röntgendetektor eine verbesserte Darstellung von Strukturen ermöglichen kann. Dabei kann der vorgegebene Winkel vorteilhafterweise derart vorgegeben werden, dass die Anodenachse bezüglich der Ebene der Standeinheit auf einer Seite der Röntgeneinheit steigend und auf einer der Röntgeneinheit abgewandten Seite fallend angeordnet ist.

Ein räumlicher Abstand zwischen dem Brennfleck und der Brustwand kann in einem direkten Zusammenhang mit dem vordefinierten Winkel zwischen der Anodenachse und der vertikalen Ebene der Standeinheit stehen. Je kleiner der vordefinierte Winkel zwischen der Anodenachse und der vertikalen Ebene der Standeinheit ist, desto näher kann der Brennfleck an die Brustwand herangeführt werden. Eine Verringerung des räumlichen Abstands zwischen dem Brennfleck und der Brustwand kann zu einer verbesserten Abbildung des brustwandnahen Gewebes führen, was für eine diagnostische Genauigkeit von Bedeutung sein kann.

Bei der spezifischen Ausführungsform mit einem vordefinierten Winkel von 84° zwischen der Anodenachse und der vertikalen Ebene der Standeinheit kann eine besonders vorteilhafte Konfiguration erreicht werden. Dieser Winkel kann einen guten Kompromiss zwischen der Annäherung des Brennflecks an die Brustwand und der Aufrechterhaltung einer verbesserten Strahlgeometrie darstellen Durch den Winkel von 84° kann eine gleichmäßigere Intensitätsverteilung der Röntgenstrahlung auf der brustwandnahen Seite eines Untersuchungsobjekts erreicht werden. Zudem kann durch den vordefinierten Winkel zwischen der Anodenachse und der vertikalen Ebene der Standeinheit von 84° der Abstand von der Brustwand zum Brennfleck um etwa 10 % verkürzt werden.

Die vorgeschlagene Ausführungsform kann vorteilhafterweise eine präzise Positionierung und Ausrichtung der Röntgeneinheit ermöglichen. Zudem können eine verbesserte Strahlgeometrie und Intensitätsverteilung der Röntgenstrahlung erreicht werden, was zu einer verbesserten Bildqualität bei gleichzeitig reduzierter Strahlenbelastung für das Untersuchungsobjekt führen kann.

Die Erfindung betrifft in einem fünften Aspekt ein Verfahren zum Erfassen von Bilddaten mittels eines vorgeschlagenen Bildgebungsgeräts. In einem ersten Schritt wird die Röntgeneinheit entlang eines Aufnahmepfads bezüglich des Röntgendetektors bewegt. Der Aufnahmepfad gibt einen oder mehrere Aufnahmeabschnitte für die Anordnung der Röntgeneinheit vor. In einem weiteren Schritt wird die Röntgeneinheit bei Anordnung in dem Aufnahmeabschnitt oder in einem der mehreren Aufnahmeabschnitte gemäß einem vorgeschlagenen Verfahren zum Betrieb einer Röntgeneinheit betrieben. Dabei erfasst der Röntgendetektor die mittels der Röntgeneinheit emittierte Röntgenstrahlung. In einem weiteren Schritt wird die Bestromung der Spule während der Bewegung der Röntgeneinheit entlang des Aufnahmeabschnitts oder jeweils eines der mehreren Aufnahmeabschnitte des Aufnahmepfads in Abhängigkeit einer momentanen Positionierung der Röntgeneinheit derart angepasst, dass der von der Kathode ausgesendete Elektronenstrahl mittels des Magnetfelds zur Minimierung einer Bewegung des Brennflecks bezüglich des Röntgendetektors abgelenkt wird. In einem weiteren Schritt werden die Bilddaten basierend auf der erfassten Röntgenstrahlung bereitgestellt.

Die Vorteile des vorgeschlagenen Verfahrens zum Erfassen von Bilddaten mittels eines Bildgebungsgeräts entsprechen im Wesentlichen den Vorteilen der vorgeschlagenen Röntgeneinheit, des vorgeschlagenen Bildgebungsgeräts, des vorgeschlagenen Verfahrens zum Betrieb einer Röntgeneinheit und/oder des vorgeschlagenen Verfahrens zur Kalibrierung einer Röntgeneinheit. Hierbei erwähnte Merkmale, Vorteile oder alternative Ausführungsformen können ebenso auch auf die anderen beanspruchten Gegenstände übertragen werden und umgekehrt.

Insbesondere kann ein vorgeschlagenes Bildgebungsgerät dazu ausgebildet sein, eine Ausführungsform des vorgeschlagenen Verfahrens zum Erfassen von Bilddaten auszuführen.

Der Aufnahmepfad kann beispielsweise eine vorgegebene Trajektorie umfassen, entlang derer die Röntgeneinheit bewegt werden kann. Der Aufnahmepfad kann den einen Aufnahmeabschnitt oder die mehreren Aufnahmeabschnitte umfassen. Der eine oder die mehreren Aufnahmeabschnitte können jeweils eine Position entlang des Aufnahmepfads oder einen, insbesondere räumlichen, Abschnitt des Aufnahmepfads umfassen. Dabei kann die Röntgeneinheit zur Emission von Röntgenstrahlung innerhalb des Aufnahmeabschnitts oder innerhalb jeweils eines der mehreren Aufnahmeabschnitte angeordnet werden. Die Anordnung der Röntgeneinheit in dem einen oder den mehreren Aufnahmeabschnitten kann beispielsweise durch eine Steuerungseinheit des Bildgebungsgeräts gesteuert werden. Umfasst der Aufnahmepfad mehrere Aufnahmeabschnitte, so können sich die Aufnahmeabschnitte teilweise überschneiden oder verschieden voneinander sein. Insbesondere können jeweils zwei der Aufnahmeabschnitte des Aufnahmepfads, insbesondere direkt, aneinander angrenzen oder beabstandet voneinander angeordnet sein. Dabei kann der Aufnahmepfad zumindest räumlich, insbesondere räumlich und zeitlich, aufgelöst sein. Beispielsweise kann der Aufnahmepfad eine zeitliche Abfolge der mehreren Aufnahmeabschnitte umfassen. Der Aufnahmepfad kann mittels des einen oder der mehreren Aufnahmeabschnitte die Trajektorie vorgeben, entlang welcher die Röntgeneinheit bezüglich des Röntgendetektors bewegt werden kann. Der Aufnahmepfad kann beispielsweise eine lineare und/oder bogenförmige und/oder kreisförmige Trajektorie umfassen.

Die Röntgeneinheit kann entlang eines Aufnahmepfads bezüglich des Röntgendetektors bewegt werden. Die Röntgeneinheit kann bei Anordnung in dem Aufnahmeabschnitt oder in einem der mehreren Aufnahmeabschnitte gemäß einem Verfahren zum Betrieb einer Röntgeneinheit betrieben werden, insbesondere während der Bewegung der Röntgeneinheit entlang des Aufnahmeanschnitts oder des jeweiligen der mehreren Aufnahmeabschnitte.

Der Röntgendetektor kann die mittels der Röntgeneinheit emittierte Röntgenstrahlung erfassen. Die Bestromung der Spule kann während der Bewegung der Röntgeneinheit entlang des Aufnahmeabschnitts oder entlang jeweils eines der mehreren Aufnahmeabschnitte des Aufnahmepfads in Abhängigkeit einer momentanen Positionierung der Röntgeneinheit derart angepasst werden, dass der von der Kathode ausgesendete Elektronenstrahl mittels des Magnetfelds zur Minimierung einer Bewegung des Brennflecks bezüglich des Röntgendetektors abgelenkt wird. Die Bilddaten können basierend auf der erfassten Röntgenstrahlung bereitgestellt werden.

Das Verfahren zum Betrieb der Röntgeneinheit in dem einen oder den mehreren Aufnahmeabschnitten kann beispielsweise das Bereitstellen einer Hochspannung zwischen der Kathode und dem Anodenteller, die Bestromung der Kathode zum Bereitstellen des Elektronenstrahls, das Rotieren des Anodentellers mit einer Rotationsfrequenz innerhalb eines vorgegebenen Rotationsfrequenzbereichs, die Bestromung der Spule mit einem Ablenkstrom zum Bereitstellen des Magnetfelds sowie das Anpassen der Ablenkung des Elektronenstrahls durch Anpassen der Bestromung der Spule umfassen.

Die Bewegung der Röntgeneinheit entlang des Aufnahmepfads kann eine Relativbewegung zwischen der Röntgeneinheit und dem Röntgendetektor umfassen. Dabei kann die Röntgeneinheit zeitlich nacheinander, insbesondere sequenziell, in den mehreren Aufnahmeabschnitten des Aufnahmepfads angeordnet werden.

Die momentane Positionierung der Röntgeneinheit kann eine räumliche Position und/oder Ausrichtung und/oder Pose der Röntgeneinheit, insbesondere bezüglich des Röntgendetektors, beschreiben. Die momentane Positionierung der Röntgeneinheit kann beispielsweise mittels eines Positionierungssensors erfasst werden. Alternativ oder zusätzlich kann die Röntgeneinheit mittels einer kalibrierten Bewegungseinheit bewegbar gelagert sein. Dabei kann eine Positionierungsinformation aufweisend eine Information zur momentanen Positionierung der Röntgeneinheit von der kalibrierten Bewegungseinheit bereitgestellt werden.

In Abhängigkeit, insbesondere basierend auf, der momentanen Positionierung der Röntgeneinheit kann die Bestromung der Spule angepasst werden. Das Anpassen der Bestromung der Spule in Abhängigkeit der momentanen Positionierung der Röntgeneinheit kann ein Bestimmen und Vorgeben, insbesondere Einstellen, des Ablenkstroms für die Bestromung der Spule umfassen. Das Anpassen der Bestromung der Spule, insbesondere des Ablenkstroms, kann auf einer vorgehaltenen, insbesondere gespeicherten, Kalibrierinformation basieren, welche eine Zuordnung zwischen der Ablenkung des Elektronenstrahls und dem Ablenkstrom charakterisiert. Die Kalibrierinformation kann beispielsweise mittels eines vorgeschlagenen Verfahrens zur Kalibrierung einer Röntgeneinheit bereitgestellt worden sein. Durch das Anpassen der Bestromung der Spule, insbesondere des Ablenkstroms, kann eine Stärke und/oder Richtung des von der Spule erzeugten Magnetfelds beeinflusst werden.

Die Minimierung der Bewegung des Brennflecks bezüglich des Röntgendetektors kann eine Reduzierung oder Vermeidung einer relativen Verschiebung zwischen dem Brennfleck und dem Röntgendetektor umfassen. Dies kann vorteilhaft sein, um eine gleichbleibende Bildqualität bei der Aufnahme von Bilddaten, insbesondere Röntgenbilddaten, zu erzielen, insbesondere bei Aufnahmesequenzen mit bewegter Röntgeneinheit wie beispielsweise bei der Tomosynthese.

Die Bestromung der Spule, insbesondere der Ablenkstrom, kann vorteilhafterweise in Abhängigkeit von einer relativen Bewegung zwischen der Röntgeneinheit und dem Röntgendetektor angepasst werden, um eine Minimierung der Bewegung des Brennflecks zu erreichen. Eine Änderung des Ablenkstroms kann zu einer Veränderung des von der Spule erzeugten Magnetfelds führen, was wiederum die Position des Elektronenstrahls und somit des Brennflecks auf dem Anodenteller beeinflussen kann.

Bei einer Erhöhung des Ablenkstroms kann sich die Stärke des Magnetfelds vergrößern, was zu einer stärkeren Ablenkung des Elektronenstrahls führen kann. Dies kann eine Verschiebung des Brennflecks auf dem Anodenteller bewirken. Umgekehrt kann eine Verringerung des Ablenkstroms zu einer schwächeren Ablenkung und einer entgegengesetzten Verschiebung des Brennflecks führen.

Um eine relative Bewegung zwischen der Röntgeneinheit und dem Röntgendetektor zu kompensieren, kann der Ablenkstrom, insbesondere kontinuierlich, angepasst werden. Bewegt sich beispielsweise die Röntgeneinheit in eine bestimmte Richtung, kann der Ablenkstrom derart verändert werden, dass der Brennfleck in eine entgegengesetzte Richtung verschoben wird. Die Anpassung des Ablenkstroms kann dabei proportional zu einer Geschwindigkeit und Richtung der relativen Bewegung erfolgen.

Vorteilhafterweise können, insbesondere in Echtzeit, die momentane Position der Röntgeneinheit erfasst und der Ablenkstrom entsprechend reguliert werden, um eine möglichst konstante Position des Brennflecks relativ zum Röntgendetektor zu gewährleisten.

Die Bestromung der Spule kann während der Bewegung der Röntgeneinheit entlang des Aufnahmepfads, insbesondere innerhalb des Aufnahmeabschnitts oder jeweils eines der Aufnahmeabschnitte und/oder zwischen jeweils zwei aufeinanderfolgenden, insbesondere benachbarten, Aufnahmeabschnitten des Aufnahmepfads, in Abhängigkeit der momentanen Positionierung der Röntgeneinheit angepasst werden. Die Anpassung der Bestromung kann beispielsweise durch die Steuerungseinheit des Bildgebungsgeräts erfolgen.

Die Erfassung der mittels der Röntgeneinheit emittierten Röntgenstrahlung kann ein Detektieren der Röntgenstrahlung mittels des Röntgendetektors umfassen. Die Erfassung der Röntgenstrahlung kann beispielsweise ein Umwandeln der Röntgenstrahlung in elektrische Signale umfassen. Die Erfassung der Röntgenstrahlung kann beispielsweise mittels einer röntgensensitiven Schicht, insbesondere Oberfläche, des Röntgendetektors erfolgen. Insbesondere kann die Erfassung der Röntgenstrahlung während der Anordnung der Röntgeneinheit in dem Aufnahmeabschnitt oder jeweils einem der mehreren Aufnahmeabschnitte erfolgen.

Das Bereitstellen der Bilddaten kann ein Erzeugen, insbesondere Rekonstruieren, der Bilddaten basierend auf der erfassten Röntgenstrahlung umfassen. Das Bereitstellen der Bilddaten kann beispielsweise ein Speichern der Bilddaten auf einem computerlesbaren Speichermedium und/oder ein Anzeigen der Bilddaten auf einer Darstellungseinheit und/oder ein Übertragen an eine Bereitstellungseinheit umfassen.

Das vorgeschlagene Verfahren kann den Vorteil aufweisen, dass durch die Anpassung der Bestromung der Spule während der Bewegung der Röntgeneinheit eine Minimierung der Bewegung des Brennflecks bezüglich des Röntgendetektors erreicht werden kann. Dadurch kann eine verbesserte Bildqualität der erfassten Bilddaten erzielt werden.

In einer weiteren vorteilhaften Ausführungsform des vorgeschlagenen Verfahrens zum Erfassen von Bilddaten mittels eines Bildgebungsgeräts kann die Bestromung der Spule zusätzlich in Abhängigkeit einer Bewegungsgeschwindigkeit der Röntgeneinheit erfolgen.

Die Bewegungsgeschwindigkeit der Röntgeneinheit kann eine Geschwindigkeit beschreiben, mit der sich die Röntgeneinheit entlang des Aufnahmepfads bewegt. Die Bewegungsgeschwindigkeit der Röntgeneinheit kann beispielsweise mittels eines Sensors erfasst werden. Der Sensor kann als Geschwindigkeitssensor, Beschleunigungssensor und/oder Positionssensor ausgebildet sein.

Die Bestromung der Spule in Abhängigkeit der Bewegungsgeschwindigkeit kann eine Anpassung des Ablenkstroms umfassen. Der Ablenkstrom kann dabei derart angepasst werden, dass die Ablenkung des Elektronenstrahls der Bewegung der Röntgeneinheit entgegenwirkt und somit die Bewegung des Brennflecks bezüglich des Röntgendetektors minimiert wird.

Vorteilhafterweise kann für die Bestromung der Spule in Abhängigkeit der Bewegungsgeschwindigkeit eine zeitliche Komponente berücksichtigt werden. So kann beispielsweise eine zukünftige Positionierung der Röntgeneinheit für einen zukünftigen Zeitpunkt basierend auf einer momentanen Positionierung und momentanen Bewegungsgeschwindigkeit der Röntgeneinheit identifiziert werden, um die Bestromung der Spule proaktiv anzupassen.

Eine für die Minimierung der Bewegung des Brennflecks bezüglich des Röntgendetektors erforderliche Änderungsrate der Bestromung der Spule, insbesondere des Ablenkstroms, kann in einem direkten Zusammenhang zu der Bewegungsgeschwindigkeit der Röntgeneinheit stehen. Bei einer höheren Bewegungsgeschwindigkeit der Röntgeneinheit kann eine schnellere Änderungsrate des Ablenkstroms erforderlich sein, um die Bewegung des Brennflecks bezüglich des Röntgendetektors effektiv zu kompensieren.

Die maximale Änderungsrate des Ablenkstroms kann durch elektrische Eigenschaften der Spule begrenzt sein, insbesondere durch ihre Induktivität. Die Induktivität der Spule kann eine Geschwindigkeit begrenzen, mit der der Ablenkstrom geändert werden kann.

Bei sehr hohen Bewegungsgeschwindigkeiten der Röntgeneinheit kann es erforderlich sein, die Änderung des Ablenkstroms zu antizipieren. Hierfür kann ein prädiktiver Regelungsalgorithmus implementiert werden, welcher dazu ausgebildet ist, die zukünftige Positionierung der Röntgeneinheit basierend auf ihrer momentanen Positionierung und momentanen Geschwindigkeit vorherzusagen und den Ablenkstrom entsprechend vorausschauend anzupassen.

Die Berücksichtigung der Bewegungsgeschwindigkeit bei der Bestromung der Spule kann zu einer verbesserten Bildqualität führen, da die Bewegung des Brennflecks bezüglich des Röntgendetektors präziser minimiert werden kann. Dies kann insbesondere bei schnellen Bewegungen der Röntgeneinheit von Vorteil sein.

In einer weiteren vorteilhaften Ausführungsform des vorgeschlagenen Verfahrens zum Erfassen von Bilddaten mittels eines Bildgebungsgeräts kann die Bestromung der Spule in einem vordefinierten Zeitraum vor dem Bereitstellen der Hochspannung zwischen der Kathode und dem Anodenteller und/oder der Bestromung der Kathode zum Bereitstellen des Elektronenstrahls erfolgen.

Der vordefinierte Zeitraum kann als ein im Voraus festgelegter Zeitabschnitt ausgebildet sein. Der vordefinierte Zeitraum kann abhängig von den spezifischen Eigenschaften der Röntgeneinheit und den Anforderungen des Bildgebungsverfahrens festgelegt werden. Der vordefinierte Zeitraum kann insbesondere in Abhängigkeit von einer Bewegungsgeschwindigkeit der Röntgeneinheit angepasst werden. Bei höheren Bewegungsgeschwindigkeiten kann ein längerer vordefinierter Zeitraum gewählt werden, um eine ausreichende Vorlaufzeit für den Aufbau des Magnetfelds zu gewährleisten. Der vordefinierte Zeitraum kann vorteilhafterweise basierend auf einer Ansprechzeit der Spule angepasst sein.

Gemäß einer ersten Ausführungsform kann das Bereitstellen der Bestromung der Spule in einem vordefinierten Zeitraum vor dem Bereitstellen der Hochspannung zwischen der Kathode und dem Anodenteller erfolgen. Dabei kann die Spule für den vordefinierten Zeitraum mit dem Ablenkstrom versorgt werden, bevor die Hochspannung zwischen der Kathode und dem Anodenteller angelegt wird. Dies kann es vorteilhaft ermöglichen, ein stabiles Magnetfeld aufzubauen, bevor die Hochspannung aktiviert wird. Der vordefinierte Zeitraum kann beispielsweise einige Millisekunden bis zu mehreren Sekunden betragen, abhängig von den spezifischen Anforderungen des Systems. Durch diese zeitliche Versetzung kann sichergestellt werden, dass das Magnetfeld zur Ablenkung des Elektronenstrahls bereits vollständig aufgebaut ist, wenn die Hochspannung angelegt wird.

Gemäß einer weiteren Ausführungsform kann die Bestromung der Spule in einem vordefinierten Zeitraum vor der Bestromung der Kathode zum Bereitstellen des Elektronenstrahls erfolgen. Hierbei kann die Spule für den vordefinierten Zeitraum mit dem Ablenkstrom versorgt werden, bevor die Kathode aktiviert wird, um Elektronen zu emittieren. Dieser Ansatz kann vorteilhaft sein, um sicherzustellen, dass das Magnetfeld bereits vorhanden ist, wenn der Elektronenstrahl erzeugt wird.

Gemäß einer weiteren Ausführungsform kann die Bestromung der Spule in einem vordefinierten Zeitraum sowohl vor dem Bereitstellen der Hochspannung zwischen der Kathode und dem Anodenteller als auch vor der Bestromung der Kathode zum Bereitstellen des Elektronenstrahls erfolgen. Diese Kombination kann eine umfassende Vorbereitung des Magnetfelds ermöglichen. Das Magnetfeld kann vollständig aufgebaut sein, bevor sowohl die Hochspannung angelegt als auch die Kathode aktiviert wird. Dies kann zu einer besonders präzisen Kontrolle des Elektronenstrahls vom Moment seiner Erzeugung an führen. Der vordefinierte Zeitraum kann in dieser Ausführungsform derart bestimmt werden, dass er die längste Ansprechzeit der beteiligten Komponenten berücksichtigt.

Die Bestromung der Spule in dem vordefinierten Zeitraum vor dem Bereitstellen der Hochspannung und/oder der Bestromung der Kathode kann vorteilhaft sein, um ein stabiles Magnetfeld aufzubauen, bevor der Elektronenstrahl erzeugt wird. Dies kann eine präzisere Ablenkung des Elektronenstrahls ermöglichen. Dies kann zu einer höheren Bildqualität bei der Erfassung von Bilddaten mittels des Bildgebungsgeräts führen.

In einer weiteren vorteilhaften Ausführungsform des vorgeschlagenen Verfahrens zum Erfassen von Bilddaten mittels eines Bildgebungsgeräts kann ein initialer Ablenkstrom zur Bestromung der Spule vor Beginn der Bewegung der Röntgeneinheit in Abhängigkeit der bereitgestellten Hochspannung zwischen der Kathode und dem Anodenteller und einer geplanten Bewegungsgeschwindigkeit der Röntgeneinheit vorgegeben werden.

Ein initialer Ablenkstrom kann zur Bestromung der Spule vor Beginn der Bewegung der Röntgeneinheit vorgegeben werden. Der initiale Ablenkstrom kann als ein Startwert für die Bestromung der Spule dienen, insbesondere vor Beginn der Bewegung der Röntgeneinheit. Dieser initiale Ablenkstrom kann basierend auf verschiedenen Parametern bestimmt werden, insbesondere der bereitgestellten Hochspannung zwischen der Kathode und dem Anodenteller sowie einer geplanten Bewegungsgeschwindigkeit der Röntgeneinheit.

Der Ablenkstrom kann die Stärke und/oder Richtung des von der Spule erzeugten Magnetfelds beeinflussen. Ferner kann das Magnetfeld den Elektronenstrahl ablenken. Die Ablenkung des Elektronenstrahls kann Eigenschaften des Brennflecks, beispielsweise eine Ausrichtung und/ oder Form des Brennflecks, beeinflussen. Bei der Ablenkung des Elektronenstrahls kann sich beispielsweise die Form, beispielsweise eine Kontur und/oder Verteilung, insbesondere eine Intensitätsverteilung, des Brennflecks auf dem Anodenteller verändern. Insbesondere kann eine Vergrößerung und/oder Verzerrung des Brennflecks auf dem Anodenteller auftreten, wenn der Elektronenstrahl in einem steileren Winkel auf den Anodenteller trifft.

Der Winkel des Einwinkeltellers der Röntgeneinheit kann ebenfalls einen Einfluss auf die Form und Größe des Brennflecks haben. Ein Einwinkelteller mit einem Winkel von 7° bis 20°, insbesondere 16°, kann dazu beitragen, die effektive Größe des Brennflecks zu verbessern.

Die Vorgabe eines initialen Ablenkstroms vor Beginn der Bewegung der Röntgeneinheit kann eine anfängliche Ablenkung des Brennflecks bewirken, was zu einer optimierten Startposition des Brennflecks auf dem Anodenteller führen kann. Dies kann dazu beitragen, dass der Brennfleck zu Beginn der Bewegung der Röntgeneinheit eine günstige, insbesondere kompakte und/oder gleichmäßige, Form und/oder Position aufweist.

Die initiale Ablenkung des Brennflecks kann beispielsweise dazu beitragen, dass der Brennfleck während der Bewegung der Röntgeneinheit eine kompakte und/oder gleichmäßige Form aufweist. Dies kann insbesondere bei schnellen Bewegungen oder komplexen Aufnahmepfaden vorteilhaft sein, da es eine stabilere Brennfleckgeometrie während der gesamten Aufnahmesequenz ermöglichen kann.

Die Form des Brennflecks kann einen erheblichen Einfluss auf die Bildqualität haben. Ein gut definierter und/oder kompakter Brennfleck kann zu einer höheren räumlichen Auflösung der Bilddaten, insbesondere Röntgenbilddaten, führen. Ein verzerrter und/oder vergrößerter Brennfleck kann hingegen zu einer Verringerung der Bildschärfe führen.

Eine angepasste, insbesondere optimierte, Brennfleckform kann ferner dazu beitragen, eine Intensitätsverteilung der Röntgenstrahlung gleichmäßiger zu gestalten. Dies kann zu einer verbesserten Kontrastauflösung und einer reduzierten Artefaktbildung in den Bilddaten führen. Insbesondere bei Tomosynthese-Aufnahmen, bei denen mehrere Projektionen aus verschiedenen Winkeln erfasst werden, kann eine konsistente Brennfleckform über den gesamten Aufnahmepfad zu einer verbesserten Rekonstruktionsqualität beitragen.

Durch die Kontrolle und Optimierung der Brennfleckform mittels gezielter Ablenkung des Elektronenstrahls kann somit eine Verbesserung der Bildqualität erreicht werden. Dies kann zu einer präziseren Diagnostik und einer potenziell reduzierten Strahlenbelastung für den Patienten führen, da möglicherweise weniger Wiederholungsaufnahmen erforderlich sind.

Die Vorgabe des initialen Ablenkstroms kann in Abhängigkeit der bereitgestellten Hochspannung zwischen der Kathode und dem Anodenteller erfolgen. Die bereitgestellte Hochspannung kann die Energie der Elektronen im Elektronenstrahl beeinflussen und somit die erforderliche Ablenkung durch das Magnetfeld der Spule beeinflussen. Eine höhere Hochspannung kann zu einer geringeren Ablenkung des Elektronenstrahls bei gleichem Magnetfeld führen. Daher kann der initiale Ablenkstrom entsprechend angepasst werden, um eine gewünschte Ablenkung des Elektronenstrahls zu erzielen.

Ferner kann die Vorgabe des initialen Ablenkstroms in Abhängigkeit der geplanten Bewegungsgeschwindigkeit der Röntgeneinheit erfolgen. Die geplante Bewegungsgeschwindigkeit kann die erforderliche Ablenkung des Elektronenstrahls zur Minimierung der Bewegung des Brennflecks bezüglich des Röntgendetektors beeinflussen. Die geplante Bewegungsgeschwindigkeit der Röntgeneinheit kann eine für die Minimierung der Bewegung des Brennflecks bezüglich des Röntgendetektors erforderliche Änderungsrate des Ablenkstroms beeinflussen. Bei einer höheren geplanten Bewegungsgeschwindigkeit kann eine höhere Änderungsrate des Ablenkstroms erforderlich sein, um die Bewegung des Brennflecks bezüglich des Röntgendetektors zu minimieren. Die Vorgabe des initialen Ablenkstroms kann vorteilhafterweise derart erfolgen, dass sie eine optimale Ausgangsbasis für eine nachfolgende dynamische Anpassung schafft, insbesondere unter Berücksichtigung der erforderlichen Änderungsrate des Ablenkstroms.

Die Vorgabe des initialen Ablenkstroms kann beispielsweise anhand einer Kalibrierinformation erfolgen. Die Kalibrierinformation kann eine Zuordnung zwischen der Ablenkung des Elektronenstrahls und dem Ablenkstrom und/oder zwischen der Ablenkung des Elektronenstrahls und der Hochspannung charakterisieren. Vorteilhafterweise kann die Kalibrierinformation mittels eines vorgeschlagenen Verfahrens zur Kalibrierung einer Röntgeneinheit bereitgestellt worden sein.

Der initiale Ablenkstrom kann als Startwert für eine anschließende dynamische Anpassung der Bestromung der Spule während der Bewegung der Röntgeneinheit dienen. Die dynamische Anpassung kann eine kontinuierliche oder schrittweise Änderung des Ablenkstroms umfassen.

Die Bereitstellung des initialen Ablenkstroms kann eine Bestromung der Spule mit dem initialen Ablenkstrom umfassen.

In einer weiteren vorteilhaften Ausführungsform des vorgeschlagenen Verfahrens zum Erfassen von Bilddaten mittels eines Bildgebungsgeräts kann die Röntgeneinheit eine Blendeneinheit umfassen. Die Blendeneinheit kann zur räumlichen Begrenzung der durch das Strahlenaustrittsfenster austretenden Röntgenstrahlung ausgebildet sein. Die Blendeneinheit kann innerhalb des einen oder der mehreren Aufnahmeabschnitte und/oder zwischen jeweils aufeinanderfolgenden Aufnahmeabschnitten des Aufnahmepfads der Röntgeneinheit angepasst werden.

Das Anpassen der Blendeneinheit kann ein Einstellen einer Öffnung der Blendeneinheit umfassen. Die Öffnung der Blendeneinheit kann dabei in ihrer Größe und/oder Form verändert werden. Dabei kann die Blendeneinheit beispielsweise motorisch und/oder elektromechanisch verstellt werden.

Das Anpassen der Blendeneinheit innerhalb des einen oder der mehreren Aufnahmeabschnitte kann ein Verstellen der Blendeneinheit während der Bewegung der Röntgeneinheit innerhalb des jeweiligen Aufnahmeabschnitts, insbesondere während der Emission von Röntgenstrahlung, umfassen. Dabei kann die Anpassung der Blendeneinheit basierend auf dem momentanen Aufnahmeabschnitt erfolgen. Insbesondere kann die Anpassung der Blendeneinheit basierend auf einer relativen Positionierung zwischen der Röntgeneinheit, insbesondere des Brennflecks, und dem Röntgendetektor erfolgen. Die Blendeneinheit kann derart angepasst werden, dass die Blendeneinheit die austretende Röntgenstrahlung, insbesondere während der Anordnung und/oder Bewegung der Röntgeneinheit entlang des jeweiligen Aufnahmeabschnitts, auf eine röntgensensitive Schicht, insbesondere Oberfläche, des Röntgendetektors und/oder eine abzubildende Interessensregion des Untersuchungsobjekts, insbesondere dynamisch, begrenzt. Das Anpassen der Blendeneinheit innerhalb des einen oder der mehreren Aufnahmeabschnitte kann eine kontinuierliche und/oder diskrete Anpassung der Blendeneinheit umfassen.

Das Anpassen der Blendeneinheit zwischen jeweils aufeinanderfolgenden Aufnahmeabschnitten kann ein Verstellen der Blendeneinheit zwischen zwei zeitlich aufeinanderfolgenden Aufnahmeabschnitten des Aufnahmepfads umfassen, insbesondere während die Röntgeneinheit zwischen zwei aufeinanderfolgenden Aufnahmeabschnitten bewegt wird. Die Anpassung der Blendeneinheit kann basierend auf dem Aufnahmepfad, insbesondere dem jeweils nachfolgenden Aufnahmeabschnitt, erfolgen. Insbesondere kann die Anpassung der Blendeneinheit basierend auf einer relativen Positionierung zwischen der Röntgeneinheit, insbesondere des Brennflecks, und dem Röntgendetektor erfolgen. Die Blendeneinheit kann derart angepasst werden, dass die Blendeneinheit die austretende Röntgenstrahlung auf eine röntgensensitive Schicht, insbesondere Oberfläche, des Röntgendetektors und/oder eine abzubildende Interessensregion des Untersuchungsobjekts begrenzt. Das Anpassen der Blendeneinheit zwischen jeweils aufeinanderfolgenden Aufnahmeabschnitten kann eine sprunghafte Anpassung der Blendeneinheit umfassen.

Des Weiteren kann die Blendeneinheit innerhalb des einen oder der mehreren Aufnahmeabschnitte und/oder zwischen jeweils aufeinanderfolgenden Aufnahmeabschnitten des Aufnahmepfads der Röntgeneinheit derart angepasst werden, dass die Röntgenstrahlung in Abhängigkeit der Ablenkung des Brennflecks den Röntgendetektor teilweise überstrahlt.

Die Anpassung der Blendeneinheit kann ferner die Veränderung der Brennfleckposition durch die Ablenkung des Elektronenstrahls berücksichtigen. Wenn sich der Brennfleck auf dem Anodenteller verschiebt, kann die Blendeneinheit entsprechend nachgeführt werden, um eine optimale Ausnutzung der Detektorfläche zu gewährleisten.

Des Weiteren kann die Blendeneinheit asymmetrisch eingestellt werden, um eine Strahlgeometrie der Röntgenstrahlung an spezifische Aufnahmesituationen anzupassen. Dies kann beispielsweise bei Tomosynthese-Aufnahmen relevant sein, bei denen sich ein Winkel zwischen der Röntgeneinheit und dem Röntgendetektor entlang des Aufnahmepfads ändert.

Vorteilhafterweise kann durch das Anpassen der Blendeneinheit innerhalb des einen oder der mehreren Aufnahmeabschnitte und/oder zwischen jeweils aufeinanderfolgenden Aufnahmeabschnitten des Aufnahmepfads der Röntgeneinheit eine verbesserte räumliche Begrenzung der durch das Strahlenaustrittsfenster austretenden Röntgenstrahlung erreicht werden. Hierdurch kann eine Strahlenbelastung für das Untersuchungsobjekt weiter reduziert werden. Ferner kann hierdurch eine verbesserte Bildqualität der erfassten Bilddaten erreicht werden.

Die präzise Anpassung der Blendeneinheit kann dazu beitragen, Streustrahlung zu reduzieren und die Bildqualität zu verbessern. Gleichzeitig kann eine optimierte Einblendung der Röntgenstrahlung die Strahlenexposition des Untersuchungsobjekts minimieren.

Gemäß einer Variante kann die Blendeneinheit vorteilhafterweise während des Erfassens der Röntgenstrahlung, insbesondere während eines Auslesens des Röntgendetektors, nicht angepasst, insbesondere nachgeführt, werden. Stattdessen kann die Blendeneinheit zwischen den einzelnen Aufnahmen, insbesondere den mehreren Aufnahmeabschnitten, angepasst werden.

In einer weiteren vorteilhaften Ausführungsform des vorgeschlagenen Verfahrens zum Erfassen von Bilddaten mittels eines Bildgebungsgeräts kann das Anpassen der Blendeneinheit innerhalb des einen oder der mehreren Aufnahmeabschnitte des Aufnahmepfads entgegen einer Bewegungsrichtung der Röntgeneinheit erfolgen.

Die Bewegungsrichtung kann eine räumliche Richtung sein, in der sich die Röntgeneinheit innerhalb des jeweiligen Aufnahmeabschnitts des Aufnahmepfads bewegt. Das Anpassen der Blendeneinheit entgegen der Bewegungsrichtung der Röntgeneinheit kann ein Anpassen der Blendeneinheit in einer zur Bewegungsrichtung der Röntgeneinheit entgegengesetzten Richtung umfassen. Das Anpassen der Blendeneinheit entgegen der Bewegungsrichtung kann bewirken, dass die räumliche Begrenzung der Röntgenstrahlung der Bewegung der Röntgeneinheit entgegenwirkt. Insbesondere kann die Röntgenstrahlung durch das Anpassen der Blendeneinheit entgegen der Bewegungsrichtung der Röntgeneinheit auf einer Seite entlang der Bewegungsrichtung der Röntgeneinheit stärker begrenzt und auf einer gegenüberliegenden Seite weniger begrenzt werden. Dies kann beispielsweise mittels der Blendeneinheit durch eine Verringerung des Strahlenfeldes in Bewegungsrichtung und/oder eine Erweiterung des Strahlenfeldes entgegen der Bewegungsrichtung erfolgen.

Das Anpassen der Blendeneinheit innerhalb des einen oder der mehreren Aufnahmeabschnitte kann kontinuierlich oder schrittweise erfolgen. Bei einem kontinuierlichen Anpassen kann die Blendeneinheit fortlaufend während der Bewegung der Röntgeneinheit angepasst werden. Bei einem schrittweisen Anpassen kann die Blendeneinheit in diskreten Schritten innerhalb des einen oder der mehreren Aufnahmeabschnitte angepasst werden.

Durch das Anpassen der Blendeneinheit entgegen der Bewegungsrichtung der Röntgeneinheit kann eine verbesserte räumliche Begrenzung der austretenden Röntgenstrahlung erreicht werden. Dies kann dazu beitragen, die Strahlenbelastung für ein Untersuchungsobjekt zu reduzieren und gleichzeitig die Bildqualität zu verbessern.

In einer weiteren vorteilhaften Ausführungsform des vorgeschlagenen Verfahrens zum Erfassen von Bilddaten mittels eines Bildgebungsgeräts kann das Anpassen der Blendeneinheit zwischen jeweils aufeinanderfolgenden Aufnahmeabschnitten des Aufnahmepfads entlang einer Bewegungsrichtung der Röntgeneinheit erfolgen.

Die Bewegungsrichtung kann eine räumliche Richtung sein, in der sich die Röntgeneinheit innerhalb des jeweiligen Aufnahmeabschnitts des Aufnahmepfads bewegt. Das Anpassen der Blendeneinheit entlang der Bewegungsrichtung kann ein Anpassen der Blendeneinheit in Richtung der Bewegung der Röntgeneinheit umfassen. Dies kann vorteilhafterweise ermöglichen, dass die Blendeneinheit für den jeweils nächsten, insbesondere zeitlich nachfolgenden, Aufnahmeabschnitt bereits korrekt positioniert ist. Aufeinanderfolgende Aufnahmeabschnitte können Abschnitte des Aufnahmepfads sein, die zeitlich oder räumlich direkt aufeinander folgen. Zwischen den aufeinanderfolgenden Aufnahmeabschnitten kann die Röntgeneinheit bewegt werden, ohne dass eine Aufnahme von Bilddaten erfolgt.

Insbesondere kann die Röntgenstrahlung durch das Anpassen der Blendeneinheit entlang der Bewegungsrichtung der Röntgeneinheit auf einer Seite entlang der Bewegungsrichtung der Röntgeneinheit weniger begrenzt und auf einer gegenüberliegenden Seite stärker begrenzt werden. Dies kann beispielsweise mittels der Blendeneinheit durch eine Verringerung des Strahlenfeldes entgegen der Bewegungsrichtung und/oder eine Erweiterung des Strahlenfeldes entlang der Bewegungsrichtung erfolgen.

Das Anpassen der Blendeneinheit zwischen den Aufnahmeabschnitten kann vorteilhafterweise ermöglichen, dass für jeden Aufnahmeabschnitt eine optimale Einstellung der Blendeneinheit vorgenommen werden kann. Dies kann zu einer verbesserten Bildqualität und/oder einer reduzierten Strahlenbelastung für das Untersuchungsobjekt führen.

In einer weiteren vorteilhaften Ausführungsform des vorgeschlagenen Verfahrens zum Erfassen von Bilddaten mittels eines Bildgebungsgeräts kann die Bestromung der Kathode zum Bereitstellen des Elektronenstrahls bei Anordnung der Röntgeneinheit in jeweils einem des einen oder der mehreren Aufnahmeabschnitte gepulst erfolgen.

Die Bestromung der Kathode zum Bereitstellen des Elektronenstrahls kann ein Anlegen eines elektrischen Stroms an die Kathode umfassen. Die Bestromung kann dabei gepulst erfolgen. Dabei kann der Strom in kurzen, aufeinanderfolgenden Strompulsen an die Kathode angelegt werden. Die gepulste Bestromung kann eine Abfolge von Strompulsen umfassen, wobei jeder Strompuls eine definierte Pulsdauer und Pulsamplitude aufweisen kann. Zwischen den Strompulsen können Pausen eingefügt sein, in denen kein oder nur ein geringer Strom fließt. Die gepulste Bestromung der Kathode kann durch Vorgabe einer Pulsdauer, Pulsfrequenz und/oder Pulsamplitude anpassbar sein. Die Pulsfrequenz, Pulsdauer und Pulsamplitude der gepulsten Bestromung der Kathode können vorteilhafterweise an die jeweiligen Aufnahmeanforderungen angepasst werden. Die Pulsdauer kann beispielsweise im Bereich von Mikro- oder Millisekunden liegen. Die Pulsfrequenz kann an die Bewegungsgeschwindigkeit der Röntgeneinheit und die gewünschte räumliche Auflösung angepasst werden. Eine höhere Pulsfrequenz kann eine feinere räumliche Abtastung ermöglichen, erfordert jedoch möglicherweise eine schnellere Bewegung der Röntgeneinheit. Die Pulsamplitude der Strompulse kann an eine gewünschte Intensität der Röntgenstrahlung angepasst werden. Höhere Pulsamplituden, insbesondere Stromamplituden, können zu einer intensiveren Röntgenstrahlung führen, erhöhen jedoch auch eine thermische Belastung der Kathode und des Anodentellers.

Eine höhere Pulsdauer kann eine Gesamtdosis erhöhen und zu einer verbesserten Bildqualität führen. Dabei kann eine höhere Pulsdauer ferner zu einer stärkeren thermischen Belastung des Anodentellers führen. Des Weiteren kann eine höhere Pulsdauer ein Risiko von Bewegungsunschärfe bei sich bewegenden Objekten erhöhen. Eine niedrigere Pulsdauer kann die Gesamtdosis reduzieren und kann die Strahlenbelastung des Untersuchungsobjekts verringern. Ferner kann eine niedrigere Pulsdauer die thermische Belastung des Anodentellers verringern. Eine niedrigere Pulsdauer kann Bewegungsartefakte reduzieren, kann jedoch Rauschen im Bild erhöhen.

Vorteilhafterweise kann die gepulste Bestromung der Kathode mit der Bewegung der Röntgeneinheit entlang des Aufnahmepfads, insbesondere der Anordnung der Röntgeneinheit in dem Aufnahmeabschnitt oder in jeweils einem der mehreren Aufnahmeabschnitte, synchronisiert sein. Vorteilhafterweise können die Strompulse derart an die Kathode bereitgestellt werden, dass sie genau dann erfolgen, wenn sich die Röntgeneinheit in dem Aufnahmeabschnitt oder in einem der mehreren Aufnahmeabschnitte befindet. Dies kann eine präzise räumliche Zuordnung der erfassten Röntgenstrahlung ermöglichen.

Die gepulste Bestromung kann ferner mit der Rotation des Anodentellers synchronisiert werden. Dies kann ermöglichen, dass der Elektronenstrahl auf einen gekühlten Bereich des Anodentellers trifft, was eine Wärmebelastung reduzieren und die Lebensdauer des Anodentellers verlängern kann.

Die gepulste Bestromung der Kathode kann vorteilhafterweise eine präzise Steuerung der Elektronenstrahlerzeugung ermöglichen. Durch die gepulste Bestromung der Kathode bei Anordnung der Röntgeneinheit in dem Aufnahmeabschnitt oder in einem der mehreren Aufnahmeabschnitte kann eine präzise zeitliche und räumliche Steuerung der Röntgenemission erreicht werden. Dies kann zu einer verbesserten Bildqualität und einer reduzierten Strahlenbelastung für das Untersuchungsobjekt führen. Ferner kann die gepulste Bestromung der Kathode vorteilhaft eine thermische Belastung der Kathode reduzieren, da in den Pausen zwischen den Strompulsen eine Abkühlung erfolgen kann. Dies kann eine Lebensdauer der Kathode verlängern. Ferner kann durch die gepulste Bestromung der Kathode die Dosis der emittierten Röntgenstrahlung präzise gesteuert werden. Zudem kann die Effizienz des Bildgebungsverfahrens erhöht werden, da die Röntgenstrahlung gezielt nur in den relevanten Aufnahmeabschnitten emittiert wird.

In einer weiteren vorteilhaften Ausführungsform des vorgeschlagenen Verfahrens zum Erfassen von Bilddaten mittels eines Bildgebungsgeräts kann das Bereitstellen der Bilddaten eine Rekonstruktion der Bilddaten basierend auf der erfassten Röntgenstrahlung und einer Information zu dem Aufnahmeabschnitt oder zu den jeweiligen Aufnahmeabschnitten der mehreren Aufnahmeabschnitte der Röntgeneinheit bezüglich des Röntgendetektors umfassen.

Die Rekonstruktion der Bilddaten kann ein Verarbeiten der erfassten Röntgenstrahlung umfassen. Die Rekonstruktion kann beispielsweise mittels einer Rekonstruktionseinheit erfolgen. Die Rekonstruktionseinheit kann als Teil der Steuerungseinheit oder als separate Einheit ausgebildet sein.

Die Information zu dem Aufnahmeabschnitt oder zu den jeweiligen Aufnahmeabschnitten der mehreren Aufnahmeabschnitte der Röntgeneinheit bezüglich des Röntgendetektors kann Informationen zu den räumlichen Positionen und/oder Ausrichtungen und/oder Posen der Röntgeneinheit und/oder des Röntgendetektors umfassen, insbesondere relativ zueinander. Diese Informationen können beispielsweise mittels Sensoren erfasst und/oder basierend auf Steuerungssignalen der Bewegung der Röntgeneinheit abgeleitet werden.

Die erfasste Röntgenstrahlung kann als Rohdaten von dem Röntgendetektor an die Rekonstruktionseinheit bereitgestellt werden. Die Rohdaten können dabei Informationen über eine Intensität der detektierten Röntgenstrahlung an verschiedenen Positionen des Röntgendetektors, insbesondere der röntgensensitiven Schicht des Röntgendetektors, umfassen.

Die Rekonstruktion basierend auf der erfassten Röntgenstrahlung, insbesondere den Rohdaten, und der Information zu dem Aufnahmeabschnitt oder den jeweiligen Aufnahmeabschnitten der mehreren Aufnahmeabschnitte kann ein Anwenden eines Rekonstruktionsalgorithmus, insbesondere einer gefilterten Rückprojektion, einer iterativen Rekonstruktionsmethode und/oder einer tomographischen Rekonstruktion, umfassen. Dabei können die Bilddaten als dreidimensionale Volumendaten rekonstruiert werden. Alternativ oder zusätzlich können die Bilddaten als zweidimensionale Projektionsbilder rekonstruiert werden.

Vorteilhafterweise kann durch die Rekonstruktion basierend auf der Information zu dem Aufnahmeabschnitt oder den jeweiligen Aufnahmeabschnitten der mehreren Aufnahmeabschnitte eine präzise räumliche Zuordnung der erfassten Röntgenstrahlung ermöglicht werden. Dies kann zu einer verbesserten Bildqualität der rekonstruierten Bilddaten führen.

Die rekonstruierten Bilddaten können anschließend in einem geeigneten Bildformat gespeichert und zur weiteren Verarbeitung und/oder Anzeige bereitgestellt werden. Die Bilddaten können beispielsweise als dreidimensionaler Datensatz oder als Schichtbilder vorliegen.

Ein technischer Vorteil dieses Verfahrens kann darin bestehen, dass durch die Berücksichtigung der genauen Aufnahmeabschnitte bei der Rekonstruktion eine hohe Bildqualität und räumliche Auflösung erreicht werden kann. Zudem kann die Rekonstruktion eine effiziente Nutzung der aufgenommenen Daten ermöglichen und somit zu einer Reduzierung der Strahlendosis für das Untersuchungsobjekt beitragen.

Die Erfindung betrifft in einem sechsten Aspekt ein Computerprogrammprodukt mit einem Computerprogramm, welches direkt in einen Speicher einer Steuerungseinheit ladbar ist. Das Computerprogrammprodukt kann Programmabschnitte umfassen. Die Programmabschnitte können dazu ausgebildet sein, alle Schritte eines Verfahrens zum Betrieb einer Röntgeneinheit, eines Verfahrens zur Kalibrierung einer Röntgeneinheit und/oder eines Verfahrens zum Erfassen von Bilddaten mittels eines Bildgebungsgeräts auszuführen. Die Ausführung der Programmabschnitte kann erfolgen, wenn die Programmabschnitte von der Steuerungseinheit ausgeführt werden.

Das Computerprogrammprodukt kann eine Software mit einem Quellcode, der noch kompiliert und gebunden oder der nur interpretiert werden muss, oder einen ausführbaren Softwarecode umfassen, der zur Ausführung nur noch in die Steuerungseinheit zu laden ist. Durch das Computerprogrammprodukt kann das Verfahren zum Betrieb einer Röntgeneinheit, das Verfahren zur Kalibrierung einer Röntgeneinheit und/oder das Verfahren zum Erfassen von Bilddaten mittels eines Bildgebungsgeräts mittels einer Steuerungseinheit schnell, identisch wiederholbar und robust ausgeführt werden. Das Computerprogrammprodukt kann so konfiguriert sein, dass es mittels der Steuerungseinheit die erfindungsgemäßen Verfahrensschritte ausführen kann.

Das Computerprogrammprodukt kann beispielsweise auf einem computerlesbaren Speichermedium gespeichert oder auf einem Netzwerk oder Server hinterlegt sein, von wo es in den Prozessor einer Steuerungseinheit geladen werden kann, der mit der Steuerungseinheit direkt verbunden oder als Teil der Steuerungseinheit ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Steuerungseinheit ein erfindungsgemäßes Verfahren durchführen. Beispiele für elektronisch lesbare Datenträger können eine DVD, ein Magnetband, eine Festplatte oder ein USB-Stick sein, auf welchem elektronisch lesbare Steuerinformationen, insbesondere Software, gespeichert sein können. Wenn diese Steuerinformationen von dem Datenträger gelesen und in eine Steuerungseinheit gespeichert werden, können alle erfindungsgemäßen Ausführungsformen der vorab beschriebenen Verfahren durchgeführt werden.

Eine weitgehend softwaremäßige Realisierung kann den Vorteil haben, dass schon bisher verwendete Steuerungseinheiten auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie zum Beispiel eine Dokumentation und/oder zusätzliche Komponenten, sowie Hardware-Komponenten, wie zum Beispiel Hardware-Schlüssel (Dongles etc.) zur Nutzung der Software, umfassen.

Weitere vorteilhafte Ausführungsformen der Erfindung sind im Folgenden beschrieben:
Ausführungsform A1: Röntgeneinheit nach einem der Ansprüche 1 bis 12, wobei der Anodenteller als Einwinkelteller ausgebildet ist.
Ausführungsform A2: Röntgeneinheit gemäß Ausführungsform A1,
   wobei der Einwinkelteller einen Winkel von 7° bis 20°, insbesondere 16°, aufweist.
Ausführungsform A3: Röntgeneinheit nach einem der Ansprüche 1 bis 12 oder nach Ausführungsform A1 oder A2,
   wobei der Anodenteller einen Durchmesser von 50 mm bis 200 mm, insbesondere 90 mm, aufweist.
Ausführungsform A4: Röntgeneinheit nach einem der Ansprüche 1 bis 12 oder nach einer der Ausführungsformen A1 bis A3,
   wobei der Anodenteller ein Trägersubstrat umfasst, wobei auf einer der Kathode zugewandten Seite eine Schicht aus einem weiteren Substrat aufgebracht ist.
Ausführungsform A5: Röntgeneinheit nach Ausführungsform A4,
   wobei das Trägersubstrat Titan-Zirkonium-Molybdän umfasst.
Ausführungsform A6: Röntgeneinheit nach Ausführungsform A4 oder A5,
   wobei das weitere Substrat eine Wolfram-Rhenium-Legierung umfasst.
Ausführungsform A7: Röntgeneinheit nach einem der Ansprüche 1 bis 12 oder nach einer der Ausführungsformen A1 bis A6,
   wobei die Kathode und der Anodenteller einen mittleren Abstand von 5 mm bis 50 mm, insbesondere 8 mm, aufweisen.
Ausführungsform A8: Röntgeneinheit nach einem der Ansprüche 1 bis 12 oder nach einer der Ausführungsformen A1 bis A7,
   wobei das Vakuumgehäuse jeweilige Durchleitungen zur Bestromung der Kathode und der Drehanode aufweist.
Ausführungsform A9: Röntgeneinheit nach einem der Ansprüche 1 bis 12 oder nach einer der Ausführungsformen A1 bis A8,
   wobei das Vakuumgehäuse aus einem Substrat gebildet wird, das eine Glas-Keramik umfasst.
Ausführungsform A10: Röntgeneinheit nach einem der Ansprüche 1 bis 12 oder nach einer der Ausführungsformen A1 bis A9,
   weiterhin umfassend eine Hochspannungseinheit, welche dazu ausgebildet ist, eine Hochspannung an die Röntgenröhre bereitzustellen,
   wobei die Hochspannungseinheit zusätzlich innerhalb des Eintankgehäuses angeordnet ist.
Ausführungsform A11: Röntgeneinheit nach Ausführungsform A10,
   wobei das Eintankgehäuse jeweilige Durchleitungen zum Bereitstellen einer Bestromung der Hochspannungseinheit und der Spule aufweist.
Ausführungsform A12: Röntgeneinheit nach einem der Ansprüche 1 bis 12 oder nach einer der Ausführungsformen A1 bis A11,
   wobei das Magnetfeld dazu ausgebildet ist, den Elektronenstrahl derart abzulenken, dass der Brennfleck auf dem Anodenteller um bis zu 2,5 mm bezüglich einer Nullposition auslenkbar ist.
Ausführungsform A13: Röntgeneinheit nach einem der Ansprüche 1 bis 12 oder nach einer der Ausführungsformen A1 bis A12,
   wobei eine Windungszahl der Spule in Abhängigkeit einer vorgegebenen Maximalablenkung des Elektronenstrahls angepasst ist.
Ausführungsform A14: Röntgeneinheit nach Anspruch 10 oder nach Anspruch 10 und einer der Ausführungsformen A1 bis A13,
   wobei der Wandabschnitt des Vakuumgehäuses eine Chrom-Nickel-Stahl-Legierung umfasst.
Ausführungsform A15: Röntgeneinheit nach Ausführungsform A14,
   wobei die Chrom-Nickel-Stahl-Legierung als austenitische Stahl-Legierung ausgebildet ist.
Ausführungsform A16: Röntgeneinheit nach einem der Ansprüche 1 bis 12 oder nach einer der Ausführungsformen A1 bis A15,
   wobei die Kathode zumindest einen Emitter aufweist, welcher zur Emission des Elektronenstrahls ausgebildet ist.
Ausführungsform A17: Röntgeneinheit nach Ausführungsform A16,
   wobei der zumindest eine Emitter als Flachemitter ausgebildet ist, welcher ein geschlitztes Emitterblech zur Emission des Elektronenstrahls aufweist.
Ausführungsform A18: Röntgeneinheit nach einem der Ansprüche 1 bis 12 oder nach einer der Ausführungsformen A1 bis A17,
   wobei die Kathode zwei verschieden ausgebildete Emitter aufweist, welche jeweils zur Emission eines Elektronenstrahls ausgebildet sind.
Ausführungsform A19: Röntgeneinheit nach Anspruch 11 oder 12 oder nach Anspruch 11 oder 12 und einer der Ausführungsformen A1 bis A18,
   wobei der Fokuskopf einstückig ausgebildet ist.
Ausführungsform A20: Röntgeneinheit nach Anspruch 11 oder 12 oder nach Ausführungsform A19,
   wobei der Fokuskopf eine Chrom-Nickel-Stahl-Legierung oder eine Molybdän-Legierung umfasst.
Ausführungsform A21: Röntgeneinheit nach Ausführungsform A18 und einem der Ansprüche 11 oder 12 oder nach Ausführungsform A18 und einer der Ausführungsformen A19 oder A20, wobei der Fokuskopf zur Fokussierung jeweils eines oder beider der von den zwei Emittern emittierbaren Elektronenstrahlen ausgebildet ist.
Ausführungsform A22: Röntgeneinheit nach einem der Ansprüche 1 bis 12 oder nach einer der Ausführungsformen A1 bis A21,
   wobei das Vakuumgehäuse an einer kathodenseitigen Wand ein zumindest teilweise versenktes Pumpkapillar zur Vakuumierung eines Innenbereichs des Vakuumgehäuses aufweist.
Ausführungsform A23: Röntgeneinheit nach einem der Ansprüche 1 bis 12 oder nach einer der Ausführungsformen A1 bis A22,
   wobei die rotierbare Lagerung des Anodentellers eine Schwingungskompensation umfasst.
Ausführungsform A24: Röntgeneinheit nach einem der Ansprüche 1 bis 12 oder nach einer der Ausführungsformen A1 bis A23,
   wobei die Röntgenröhre ferner eine Antriebseinheit zur motorischen Rotation des Anodentellers umfasst.
Ausführungsform B1: Verfahren nach Anspruch 13 oder 14,
   wobei die Kathode zwei verschieden ausgebildete Emitter aufweist, welche jeweils zur Emission eines Elektronenstrahls ausgebildet sind, wobei die Emitter selektiv oder gleichzeitig bestromt werden.
Ausführungsform B2: Verfahren nach einem der Ansprüche 13 oder 14 oder nach Ausführungsform B1,
   wobei der vorgegebene Rotationsfrequenzbereich Rotationsfrequenzen von 130 Hz bis 160 Hz umfasst.
Ausführungsform B3: Verfahren nach einem der Ansprüche 13 oder 14 oder nach Ausführungsform B1 oder B2,
   wobei überwacht wird, ob die momentane Rotationsfrequenz des Anodentellers innerhalb des vorgegebenen Rotationsfrequenzbereichs liegt,
   wobei verneinendenfalls ein Warnhinweis bereitgestellt wird.

Das vorgeschlagene Verfahren zum Betrieb einer Röntgeneinheit, insbesondere nach Anspruch 13 oder 14 oder nach einer der Ausführungsformen B1 bis B3, kann insbesondere auch zum Betrieb einer Röntgeneinheit nach einer der Ausführungsformen A1 bis A24 ausgebildet sein.
Ausführungsform C1: Verfahren zur Kalibrierung einer Röntgeneinheit nach einem der Ansprüche 1 bis 12 oder einer der Ausführungsformen A1 bis A24, umfassend:
   a) Positionieren einer Absorptionsmaske zwischen dem Strahlenaustrittsfenster der Röntgeneinheit und einer Röntgenerfassungseinheit,
   b) Bereitstellen einer Hochspannung zwischen der Kathode und dem Anodenteller,
   c) Bestromung der Kathode zum Bereitstellen des Elektronenstrahls,
   d) Rotieren des Anodentellers mit einer Rotationsfrequenz innerhalb eines vorgegebenen Rotationsfrequenzbereichs,
   e) Bestromung der Spule mit einem Ablenkstrom zum Bereitstellen des Magnetfelds,
   f) Erfassen einer Position einer Abbildung der Absorptionsmaske mittels der Röntgenerfassungseinheit,
      wobei die Schritte b) bis f) für eine Vielzahl verschiedener Hochspannungen und/oder Ablenkströme wiederholt ausgeführt werden,
      wobei eine Zuordnung zwischen der Ablenkung des Elektronenstrahls und dem Ablenkstrom und/oder zwischen der Ablenkung des Elektronenstrahls und der Hochspannung basierend auf der Vielzahl verschiedener Hochspannungen und/oder Ablenkströme und den zugehörigen Positionen der Abbildung identifiziert wird,
   g) Bereitstellen einer die Zuordnung charakterisierenden Kalibrierinformation.
Ausführungsform C2: Verfahren nach Ausführungsform C1,
   wobei die Röntgenerfassungseinheit eine Röntgenkamera und/oder einen Röntgendetektor umfasst.
Ausführungsform C3: Verfahren nach Ausführungsform C1 oder C2,
   wobei für die wiederholte Ausführung der Schritte b) bis f) jeweils zumindest 4 verschiedene Hochspannungen und/oder Ablenkströme vorgegeben werden.
Ausführungsform C4: Verfahren nach einer der Ausführungsformen C1 bis C3,
   wobei das Identifizieren der Zuordnung zwischen der Ablenkung des Elektronenstrahls und dem Ablenkstrom und/oder zwischen der Ablenkung des Elektronenstrahls und der Hochspannung des Elektronenstrahls eine jeweilige Interpolation umfasst.
Ausführungsform D1: Bildgebungsgerät nach Anspruch 15 oder 16,
   weiterhin umfassend eine Standeinheit, eine erste und eine zweite Halteeinheit,
   wobei die erste Halteeinheit rotierbar gelagert an der Standeinheit angeordnet ist,
   wobei die Röntgeneinheit an der ersten Halteeinheit angeordnet ist, wobei die zweite Halteeinheit an der Standeinheit angeordnet ist,
   wobei der Röntgendetektor an der zweiten Halteeinheit angeordnet ist,
   wobei die erste und die zweite Halteeinheit die Röntgeneinheit und den Röntgendetektor in Gegenüberstellung zueinander halten.
Ausführungsform D2: Bildgebungsgerät nach Ausführungsform D1,
   wobei die erste Halteeinheit L- oder J-förmig ausgebildet und mittels eines ersten Schenkels an der Standeinheit angeordnet ist,
   wobei die Röntgeneinheit an einem weiteren Schenkel der ersten Halteeinheit angeordnet ist.

Das vorgeschlagene Bildgebungsgerät, insbesondere nach Anspruch 15 oder 16 oder nach einer der Ausführungsformen D1 oder D2, kann insbesondere auch eine Röntgeneinheit nach einer der Ausführungsformen A1 bis A24 umfassen.
Ausführungsform E1: Verfahren nach einem der Ansprüche 17 bis 20,
   wobei die Bestromung der Spule zusätzlich in Abhängigkeit einer Bewegungsgeschwindigkeit der Röntgeneinheit erfolgt.
Ausführungsform E2: Verfahren nach einem der Ansprüche 17 bis 20 oder nach Ausführungsform E1,
   wobei die Bestromung der Spule in einem vordefinierten Zeitraum vor dem Bereitstellen der Hochspannung zwischen der Kathode und dem Anodenteller und/oder der Bestromung der Kathode zum Bereitstellen des Elektronenstrahls erfolgt.
Ausführungsform E3: Verfahren nach einem der Ansprüche 17 bis 20 oder nach einer der Ausführungsformen E1 oder E2,
   wobei ein initialer Ablenkstrom zur Bestromung der Spule vor Beginn der Bewegung der Röntgeneinheit in Abhängigkeit der bereitgestellten Hochspannung zwischen der Kathode und dem Anodenteller und einer geplanten Bewegungsgeschwindigkeit der Röntgeneinheit vorgegeben wird.
Ausführungsform E4: Verfahren nach einem der Ansprüche 17 bis 20 oder nach einer der Ausführungsformen E1 bis E3,
   wobei die Bestromung der Kathode zum Bereitstellen des Elektronenstrahls bei Anordnung der Röntgeneinheit in jeweils einem des einen oder der mehreren Aufnahmeabschnitte des Aufnahmepfads gepulst erfolgt.
Ausführungsform E5: Verfahren nach einem der Ansprüche 17 bis 20 oder nach einer der Ausführungsformen E1 bis E4,
   wobei das Bereitstellen der Bilddaten eine Rekonstruktion der Bilddaten basierend auf der erfassten Röntgenstrahlung und einer Information zu dem Aufnahmeabschnitt oder den jeweiligen Aufnahmeabschnitten der mehreren Aufnahmeabschnitte der Röntgeneinheit bezüglich des Röntgendetektors umfasst.

Das vorgeschlagene Verfahren zum Erfassen von Bilddaten, insbesondere nach einem der Ansprüche 17 bis 20 oder nach einer der Ausführungsformen E1 bis E5, kann insbesondere auch zum Erfassen von Bilddaten mittels eines Bildgebungsgeräts nach einer der Ausführungsformen D1 oder D2 ausgebildet sein. Zudem kann in einer vorteilhaften Ausführungsform des vorgeschlagenen Verfahrens zum Erfassen von Bilddaten, insbesondere nach einem der Ansprüche 17 bis 20 oder einer der Ausführungsformen E1 bis E4, die Röntgeneinheit bei Anordnung in dem einen Aufnahmeabschnitt oder einem der mehreren Aufnahmeabschnitte gemäß einem Verfahren nach einer der Ausführungsformen B1 bis B3 betrieben werden.

Ausführungsform F: Computerprogrammprodukt mit einem Computerprogramm, welches direkt in einen Speicher einer Steuerungseinheit ladbar ist, mit Programmabschnitten, um alle Schritte eines Verfahrens nach einer der Ausführungsformen B1 bis B3, C1 bis C4 und/oder E1 bis E5 auszuführen, wenn die Programmabschnitte von der Steuerungseinheit ausgeführt werden.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet. Es zeigen:
- Fig. 1 bis 3: schematische Darstellungen verschiedener vorteilhafter Ausführungsform einer vorgeschlagenen Röntgeneinheit,
- Fig. 4: eine schematische Darstellung eines Querschnitts einer vorteilhaften Ausführungsform der Spule senkrecht zu einer Wicklungsrichtung der Spule,
- Fig. 5: eine schematische Darstellung eines Querschnitts einer vorteilhaften Ausführungsform der Spule parallel zu einer Wicklungsrichtung der Spule,
- Fig. 6: eine schematische Darstellung einer weiteren vorteilhaften Ausführungsform einer vorgeschlagenen Röntgeneinheit,
- Fig. 7: eine schematische Darstellung einer vorteilhaften Ausführungsform einer Kathode,
- Fig. 8 bis 10: schematische Darstellungen verschiedener vorteilhafter Ausführungsformen eines vorgeschlagenen Verfahrens zum Betrieb einer Röntgeneinheit,
- Fig. 11: eine schematische Darstellung einer vorteilhaften Ausführungsform eines vorgeschlagenen Verfahrens zur Kalibrierung einer Röntgeneinheit,
- Fig. 12 bis 16: schematische Darstellungen verschiedener vorteilhafter Ausführungsformen eines vorgeschlagenen Bildgebungsgeräts,
- Fig. 17 bis 20: schematische Darstellungen verschiedener vorteilhafter Ausführungsformen eines vorgeschlagenen Verfahrens zum Erfassen von Bilddaten mittels eines Bildgebungsgeräts,
- Fig. 21: eine schematische Darstellung einer beispielhaften Ablenkstrom-Zeit-Kurve,
- Fig. 22: eine schematische Darstellung eines beispielhaften Ablaufdiagramms zur Verwendung in einem vorgeschlagenen Verfahren zum Erfassen von Bilddaten mittels eines Bildgebungsgeräts.

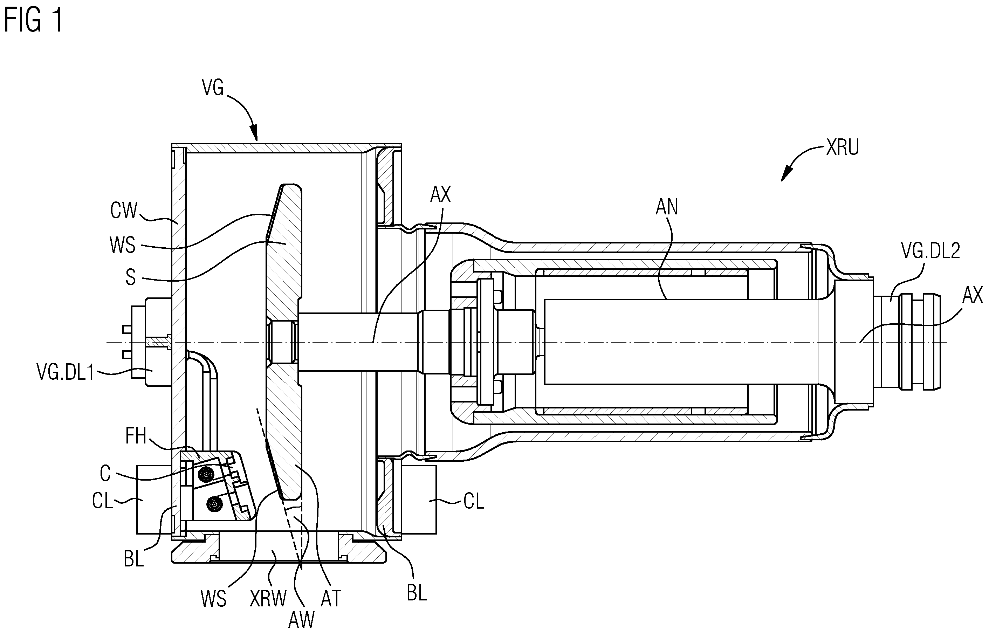

Fig. 1 zeigt eine schematische Darstellung einer vorteilhaften Ausführungsform einer vorgeschlagenen Röntgeneinheit XRU. Die Röntgeneinheit kann eine Röntgenröhre und eine Spule CL umfassen. Dabei kann die Röntgenröhre eine Kathode C, eine Drehanode und ein Vakuumgehäuse VG umfassen. Die Drehanode kann einen um eine Anodenachse AX rotierbar gelagerten Anodenteller AT umfassen. Zudem können die Kathode C und die Drehanode, insbesondere der Anodenteller AT, innerhalb des Vakuumgehäuses VG angeordnet sein. Die Spule CL kann außerhalb des Vakuumgehäuses VG angeordnet sein. Des Weiteren können die Spule CL und die Röntgenröhre voneinander galvanisch getrennt sein. Die Spule CL kann dazu ausgebildet sein, ein Magnetfeld zu erzeugen, welches einen Zwischenraum zwischen der Kathode C und dem Anodenteller AT durchdringen kann. Das Magnetfeld kann dazu ausgebildet sein, einen in einem Brennfleck der Röntgenröhre auf den Anodenteller AT auftreffenden, von der Kathode C ausgehenden Elektronenstrahl abzulenken. Das Vakuumgehäuse VG kann an einer bezüglich der Anodenachse AX radial angeordneten Seite ein Strahlenaustrittsfenster XRW aufweisen, welches zum Durchlass einer von dem Brennfleck auf dem Anodenteller AT ausgehenden Röntgenstrahlung ausgebildet ist.

Vorteilhafterweise kann der Anodenteller AT als Einwinkelteller ausgebildet sein. Insbesondere kann der Winkelteller einen Winkel AW von 7° bis 20°, insbesondere 16°, aufweisen. Der Anodenteller AT kann einen Durchmesser von 50 mm bis 200 mm, insbesondere 90 mm, aufweisen.

Vorteilhafterweise kann der Anodenteller AT ein Trägersubstrat S umfassen. Dabei kann auf einer der Kathode C zugewandten Seite eine Schicht aus einem weiteren Substrat WS aufgebracht sein. Das Trägersubstrat S kann Titan-Zirkonium-Molybdän umfassen. Das weitere Substrat WS kann eine Wolfram-Rhenium-Legierung umfassen.

Die Kathode C und der Anodenteller AT können einen mittleren Abstand von 5 mm bis 50 mm, insbesondere 8 mm, zueinander aufweisen.

Das Vakuumgehäuse VG kann jeweilige Durchleitungen VG.DL1 und VG.DL2 zur Bestromung der Kathode C und der Drehanode aufweisen. Vorteilhafterweise können die Durchleitungen VG.DL1 zur Bestromung der Kathode C an einer kathodenseitigen Wand CW des Vakuumgehäuses VG angeordnet sein.

Das Vakuumgehäuse VG kann aus einem Substrat gebildet werden, das eine Glas-Keramik umfassen kann.

Das Magnetfeld kann dazu ausgebildet sein, den Elektronenstrahl derart abzulenken, dass der Brennfleck auf dem Anodenteller AT um bis zu 2,5 mm bezüglich einer Nullposition auslenkbar ist.

Der Elektronenstrahl kann in einem Betriebszustand der Röntgeneinheit XRU zumindest teilweise innerhalb eines Öffnungsbereichs der Spule CL angeordnet sein. Insbesondere kann der Brennfleck der Röntgenröhre in dem Betriebszustand der Röntgeneinheit innerhalb des Öffnungsbereichs der Spule CL angeordnet sein. Die Spule CL kann als Luftspule ausgebildet sein. Zudem kann eine Windungszahl der Spule CL kann in Abhängigkeit einer vorgegebenen Maximalablenkung des Elektronenstrahls angepasst sein. Die Spule CL und der Elektronenstrahl können durch einen aus einem unmagnetischen metallischen Material geringer elektrischer Leitfähigkeit gebildeten Wandabschnitt BL des Vakuumgehäuses VG voneinander getrennt sein. Der Wandabschnitt BL des Vakuumgehäuses VG kann eine Chrom-Nickel-Stahl-Legierung umfassen. Die Chrom-Nickel-Stahl-Legierung kann als austenitische Stahl-Legierung ausgebildet sein.

Die Kathode C kann einen Fokuskopf FH zur Fokussierung des Elektronenstrahls aufweisen. Der Fokuskopf FH kann insbesondere einstückig ausgebildet sein. Des Weiteren kann der Fokuskopf FH eine Chrom-Nickel-Stahl-Legierung oder eine Molybdän-Legierung umfassen.

Vorteilhafterweise kann die rotierbare Lagerung des Anodentellers AT eine Schwingungskompensation umfassen.

Die Röntgenröhre kann ferner eine Antriebseinheit AN zur motorischen Rotation des Anodentellers AT umfassen.

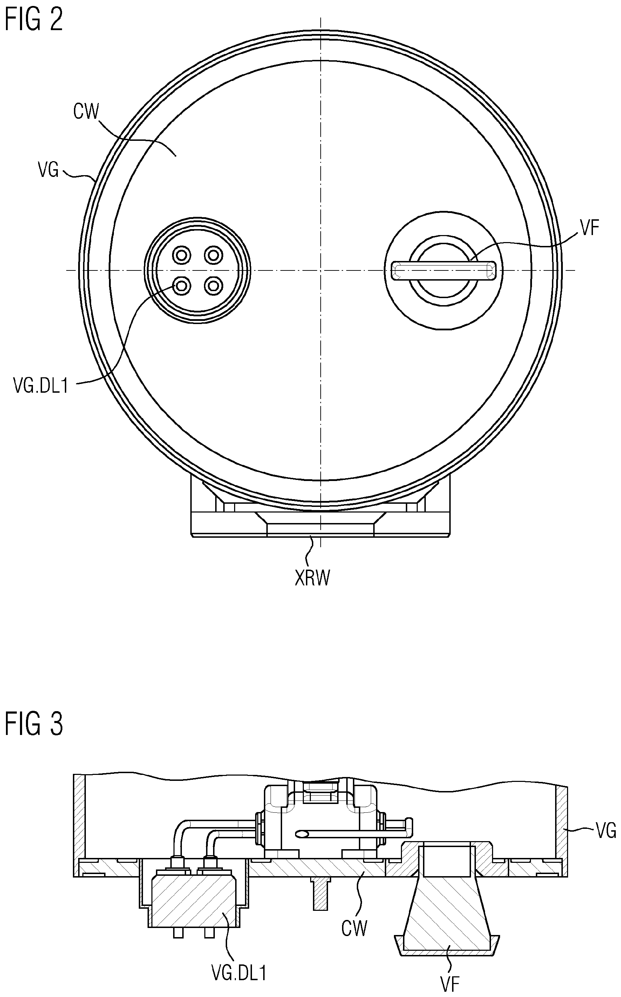

Fig. 2 zeigt eine Aufsicht auf die kathodenseitige Wand CW des Vakuumgehäuses VG der in Fig. 1 schematisch dargestellten Ausführungsform der vorgeschlagenen Röntgeneinheit XRU. Das Vakuumgehäuse VG kann kathodenseitig eine im Wesentlichen runde, insbesondere kreisförmige, äußere Form, insbesondere Kontur, aufweisen. Dabei kann die Anodenachse AX beispielsweise durch einen Mittelpunkt der kathodenseitigen Wand CW des Vakuumgehäuses verlaufen.

Dabei können die Durchleitungen VG.DL1 zur Bestromung der Kathode C in einem räumlichen Bereich, insbesondere einer symmetrischen Anordnung, der kathodenseitigen Wand CW des Vakuumgehäuses angeordnet sein. In der in Fig. 2 dargestellten Ausführungsform kann die Kathode C zwei Emitter, insbesondere Flachemitter, aufweisen. Dabei kann die Röntgeneinheit XRU jeweils zwei Durchleitungen VG.DL1 zur separaten Bestromung der zwei Emitter aufweisen. Wie in Fig. 2 schematisch dargestellt, können die vier Durchleitungen VG.DL1 beispielsweise innerhalb einer kreisförmigen Aussparung der kathodenseitigen Wand CW des Vakuumgehäuses VG angeordnet sein. Dabei können die Durchleitungen VG.DL1 beispielsweise an vier Eckpunkten eines Quadrats angeordnet sein.

Des Weiteren kann das Vakuumgehäuse VG an der kathodenseitigen Wand CW ein zumindest teilweise versenktes Pumpkapillar VF zur Vakuumierung eines Innenbereichs des Vakuumgehäuses VG aufweisen.

Das zumindest teilweise versenkte Pumpkapillar VF und die Anordnung der Durchleitungen VG.DL1 zur Bestromung der Kathode C können beispielsweise auf einem gemeinsamen Durchmesser der kathodenseitigen Wand CW des Vakuumgehäuses angeordnet sein. Dabei kann der gemeinsame Durchmesser einen rechten Winkel bezüglich des radial angeordneten Strahlenaustrittsfensters XRW des Vakuumgehäuses VG aufweisen.

Fig. 3 zeigt eine Ansicht auf einen Querschnitt der kathodenseitigen Wand CW des Vakuumgehäuses VG der in den Figuren 1 und 2 schematisch dargestellten Ausführungsform der vorgeschlagenen Röntgeneinheit XRU, insbesondere entlang des gemeinsamen Durchmessers der kathodenseitigen Wand CW entlang welchem die Anordnung der Durchleitungen VD.DL1 zur Bestromung der Kathode C und das Pumpkapillar VF angeordnet sind.

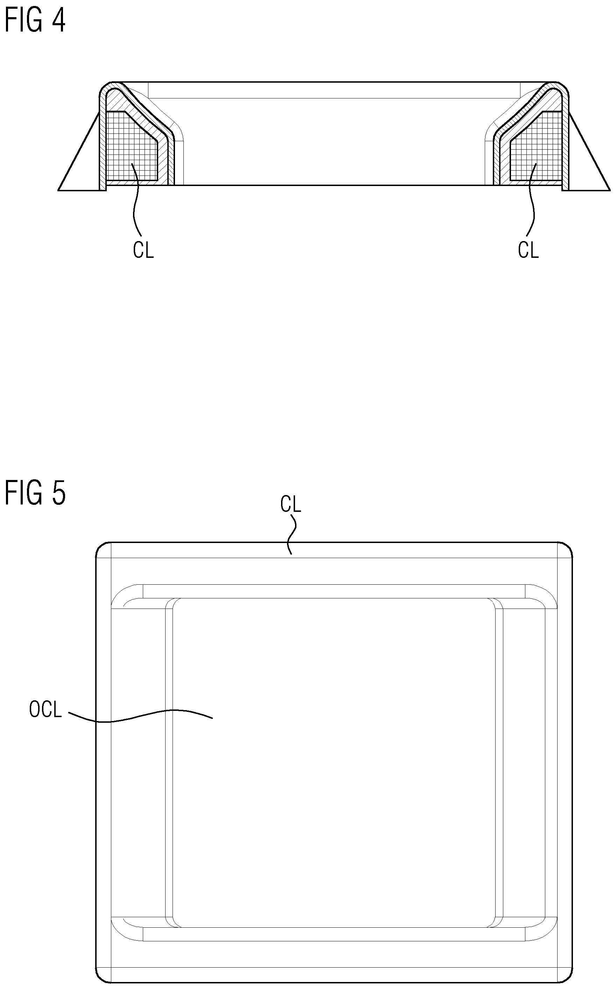

Fig. 4 zeigt eine schematische Darstellung eines Querschnitts einer vorteilhaften Ausführungsform der Spule CL senkrecht zu einer Wicklungsrichtung der Spule CL. Dabei kann die Spule CL eine Spulengeometrie aufweisen, welche an eine Geometrie der Röntgenröhre, insbesondere des Vakuumgehäuses VG, angepasst ist. Vorteilhafterweise kann die Spulengeometrie eine Querschnittsgeometrie der Spule CL umfassen. Dabei kann die Querschnittsgeometrie der Spule CL zumindest an einer Innenseite der Spule CL an die Geometrie des Vakuumgehäuses VG innerhalb eines Öffnungsbereichs der Spule CL angepasst sein. Ferner kann die Spule CL als Luftspule ausgebildet sein. In der in Fig. 4 schematisch dargestellten Ausführungsform kann eine Wicklungsrichtung der Spule CL senkrecht zu einer Zeichenebene der Darstellung, insbesondere einer Querschnittsfläche des Querschnitts, verlaufen. Die Querschnittsgeometrie der Spule kann dabei zumindest abschnittsweise trapezförmig ausgebildet sein.

Fig. 5 zeigt eine schematische Darstellung eines Querschnitts einer vorteilhaften Ausführungsform der Spule CL parallel zu einer Wicklungsrichtung der Spule CL. Dabei kann die Spule CL ein Spulengehäuse aufweisen, welches die Spule CL umgibt, insbesondere umschließt. Das Spulengehäuse kann im Wesentlichen der Spulengeometrie, insbesondere einer äußeren Form, insbesondere Kontur, der Spule CL folgen. Die Spule CL kann in der in Fig. 5 schematisch dargestellten Ausführungsform im Wesentlichen rechteckig, insbesondere quadratisch, um ihren Öffnungsbereich OCL herum angeordnet, insbesondere gewickelt, sein. Ferner kann die Spule CL, insbesondere das Spulengehäuse, an zwei gegenüberliegenden Innenseiten bezüglich des Öffnungsbereichs OCL eine Abschrägung, insbesondere einen trapezförmigen Querschnitt, aufweisen. Hierdurch kann eine Anpassung der Spulengeometrie, insbesondere der Querschnittsgeometrie, an der Innenseite der Spule CL an die Geometrie des Vakuumgehäuses VG innerhalb des Öffnungsbereichs OCL der Spule CL erreicht werden.

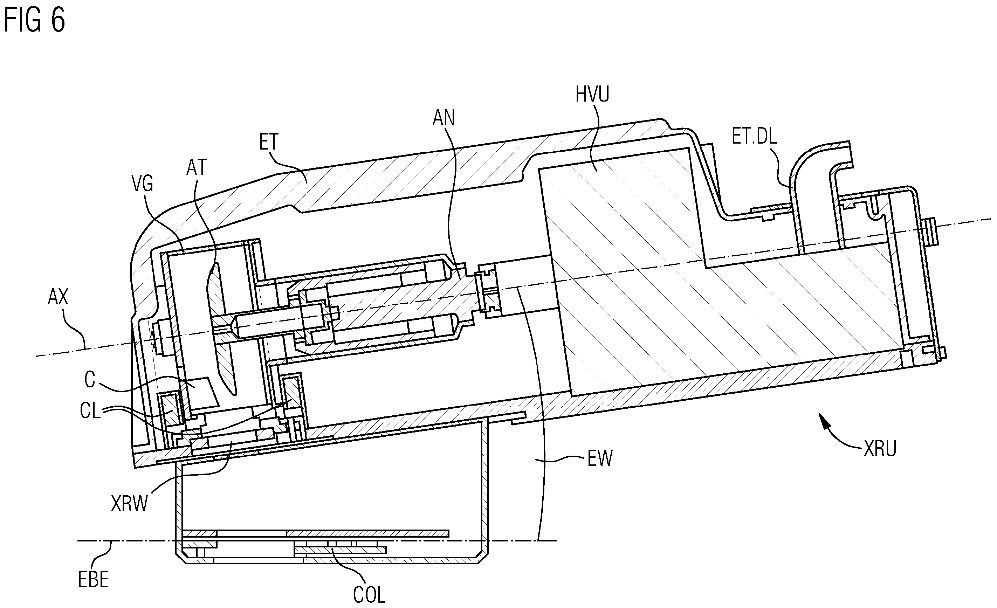

Fig. 6 zeigt eine schematische Darstellung einer weiteren vorteilhaften Ausführungsform einer vorgeschlagenen Röntgeneinheit XRU. Dabei kann die Röntgeneinheit XRU weiterhin ein leckdichtes Eintankgehäuse ET umfassen, wobei die Röntgenröhre und die Spule CL innerhalb des Eintankgehäuses ET angeordnet sein können. Des Weiteren kann das Eintankgehäuse ET mit einem Kühl-Isoliermedium gefüllt sein.

Die Röntgeneinheit XRU kann weiterhin eine Hochspannungseinheit HVU umfassen, welche dazu ausgebildet sein kann, die Hochspannung an die Röntgenröhre, insbesondere die Kathode C und den Anodenteller AT, bereitzustellen. Dabei kann die Hochspannungseinheit HVU zusätzlich innerhalb des Eintankgehäuses ET angeordnet sein.

Vorteilhafterweise kann das Eintankgehäuse ET jeweilige Durchleitungen ET.DL zum Bereitstellen einer Bestromung der Hochspannungseinheit HVU und der Spule CL aufweisen.

Die Röntgeneinheit XRU kann weiterhin eine Blendeneinheit COL umfassen, welche zur räumlichen Begrenzung der durch das Strahlenaustrittsfenster XRW austretenden Röntgenstrahlung ausgebildet sein kann. Dabei kann die Anodenachse AX einen vorgegebenen Winkel EW, insbesondere 6°, bezüglich einer Blendenebene EBE der Blendeneinheit COL aufweisen. Durch die Neigung der Anodenachse AX bezüglich der Blendenebene EBE um den vorgegebenen Winkel EW kann ein Abstand zwischen dem Brennfleck und einer Brustwand eines Untersuchungsobjekts reduziert werden. Für einen vorgegebenen Winkel EW von 6° kann der Abstand zwischen dem Brennfleck und der Brustwand um etwa 10 % verkürzt werden.

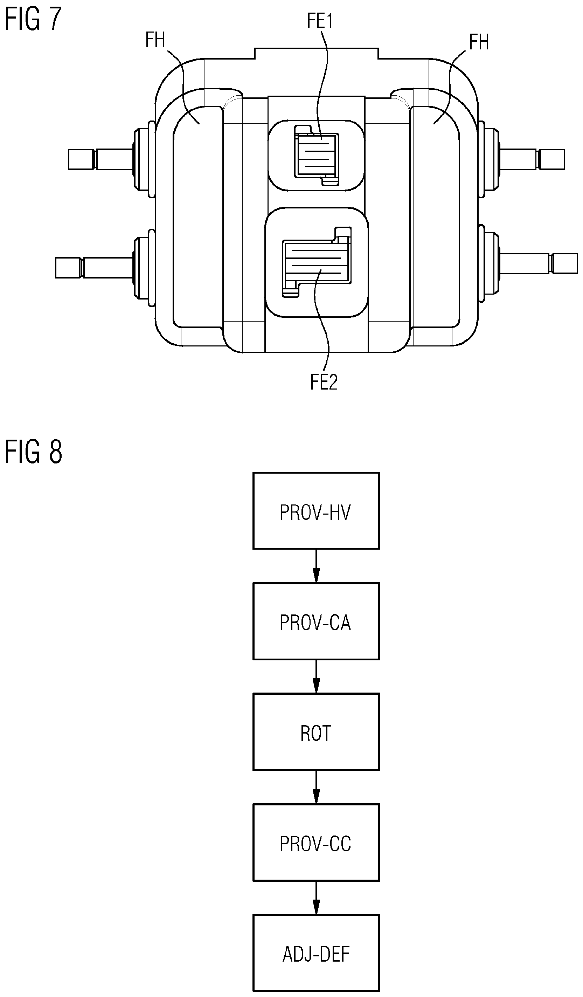

Fig. 7 zeigt eine schematische Darstellung einer vorteilhaften Ausführungsform einer Kathode C. Vorteilhafterweise kann die Kathode C zwei verschieden ausgebildete Flachemitter FE1 und FE2 aus jeweils einem geschlitzten Emitterblech aufweisen. Dabei können die beiden Flachemitter FE1 und FE2 jeweils zur Emission eines Elektronenstrahls ausgebildet sein. Die Kathode C kann einen Fokuskopf FH zur Fokussierung des Elektronenstrahls aufweisen. Dabei kann der Fokuskopf FH einstückig ausgebildet sein. Zudem kann der Fokuskopf FH zur Fokussierung jeweils eines oder beider der von den zwei Flachemittern FE1 und FE2 emittierbaren Elektronenstrahlen ausgebildet sein. Insbesondere kann der Fokuskopf FH zumindest durch seine Form zur elektrostatischen Fokussierung des Elektronenstrahls ausgebildet sein.

Fig. 8 zeigt eine schematische Darstellung einer vorteilhaften Ausführungsform eines vorgeschlagenen Verfahrens zum Betrieb einer vorgeschlagenen Röntgeneinheit XRU. Dabei kann eine Hochspannung zwischen der Kathode C und dem Anodenteller AT bereitgestellt werden PROV-HV. Die Kathode C kann bestromt werden PROV-CA, um einen Elektronenstrahl zu erzeugen. Ferner kann der Anodenteller AT mit einer Rotationsfrequenz innerhalb eines vorgegebenen Rotationsfrequenzbereichs rotiert werden. Des Weiteren kann die Spule CL mit einem Ablenkstrom bestromt werden PROV-CC, um ein Magnetfeld zu erzeugen. Zudem kann eine Ablenkung des Elektronenstrahls durch Anpassen der Bestromung der Spule CL angepasst werden ADJ-DEF.

Vorteilhafterweise kann die Kathode C zwei verschiedene Emitter, insbesondere Flachemitter FE1 und FE2, aufweisen, die jeweils zur Emission eines Elektronenstrahls ausgebildet sind. Dabei können die zwei Emitter selektiv oder gleichzeitig bestromt werden PROV-CA.

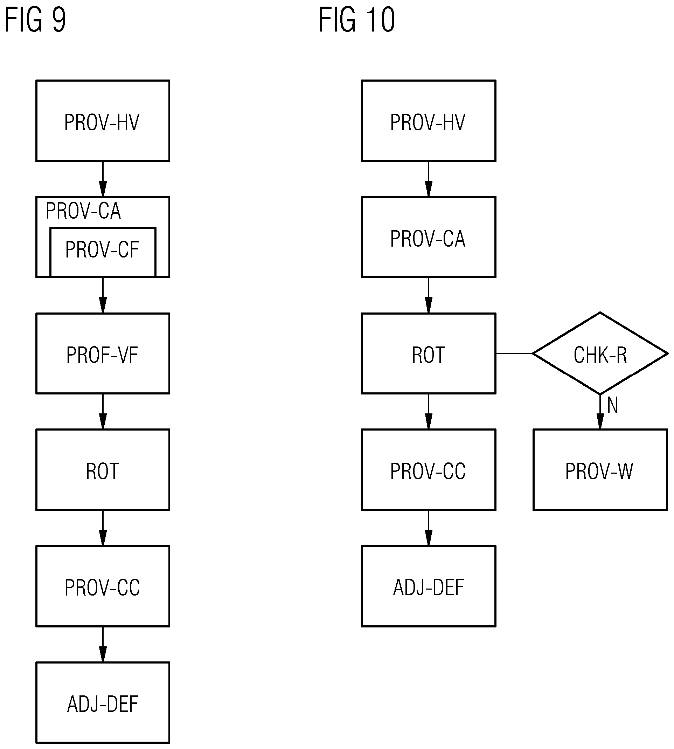

Fig. 9 zeigt eine schematische Darstellung einer weiteren vorteilhaften Ausführungsform eines vorgeschlagenen Verfahrens zum Betrieb einer vorgeschlagenen Röntgeneinheit XRU. Dabei kann die Kathode C zumindest einen Emitter, insbesondere Flachemitter, und einen Fokuskopf FH zur Fokussierung des Elektronenstrahls aufweisen. Die Bestromung der Kathode C kann eine Bestromung PROV-CF des zumindest einen Emitters umfassen. Zusätzlich kann ein elektrisches Potenzial an den zumindest einen Emitter oder ein weiteres elektrisches Potenzial an den Fokuskopf FH zur Fokussierung des Elektronenstrahls bereitgestellt werden PROV-VF.

Fig. 10 zeigt eine schematische Darstellung einer weiteren vorteilhaften Ausführungsform eines vorgeschlagenen Verfahrens zum Betrieb einer vorgeschlagenen Röntgeneinheit XRU. Dabei kann der vorgegebene Rotationsfrequenzbereich Rotationsfrequenzen von 130 Hz bis 160 Hz umfassen. Zudem kann überwacht werden CHK-R, ob eine momentane Rotationsfrequenz des Anodentellers AT innerhalb des vorgegebenen Rotationsfrequenzbereichs liegt. Verneinendenfalls N kann ein Warnhinweis bereitgestellt werden PROV-W.

Vorteilhafterweise kann die Rotationsfrequenz des Anodentellers in Abhängigkeit von einer momentanen Temperatur der Röntgenröhre innerhalb des vorgegebenen Rotationsfrequenzbereichs geregelt werden.

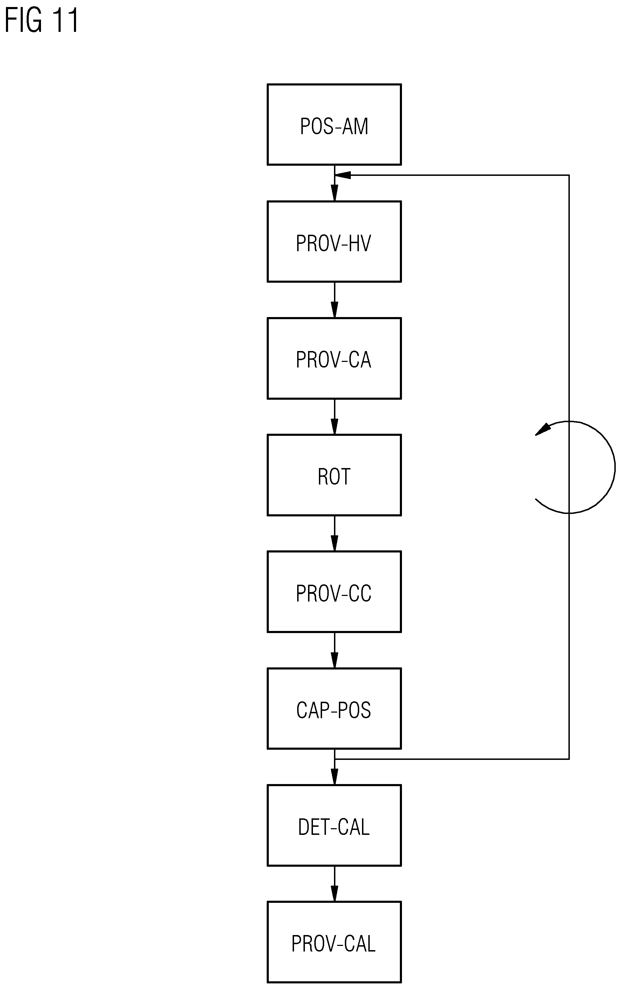

Fig. 11 zeigt eine schematische Darstellung einer vorteilhaften Ausführungsform eines vorgeschlagenen Verfahrens zur Kalibrierung einer vorgeschlagenen Röntgeneinheit XRU. In einem ersten Schritt a) kann eine Absorptionsmaske zwischen dem Strahlenaustrittsfenster XRW der Röntgeneinheit XRU und einer Röntgenerfassungseinheit positioniert werden POS-AM. In einem weiteren Schritt b) kann eine Hochspannung zwischen der Kathode C und dem Anodenteller AT bereitgestellt werden PROV-HV. In einem weiteren Schritt c) kann die Kathode C bestromt werden PROV-CA, um den Elektronenstrahl bereitzustellen. In einem weiteren Schritt d) kann der Anodenteller AT mit einer Rotationsfrequenz innerhalb eines vorgegebenen Rotationsfrequenzbereichs rotiert werden ROT. In einem weiteren Schritt e) kann die Spule CL mit einem Ablenkstrom bestromt werden PROV-CC, um das Magnetfeld bereitzustellen. In einem weiteren Schritt f) kann eine Position einer Abbildung der Absorptionsmaske mittels der Röntgenerfassungseinheit erfasst werden CAP-POS. Vorteilhafterweise können die Schritte b) bis f) für eine Vielzahl verschiedener Hochspannungen und/oder Ablenkströme wiederholt ausgeführt werden. Dabei kann eine Zuordnung zwischen der Ablenkung des Elektronenstrahls und dem Ablenkstrom und/oder der Hochspannung basierend auf der Vielzahl verschiedener Hochspannungen und/oder Ablenkströme und den zugehörigen Positionen der Abbildung identifiziert werden DET-CAL. Zudem kann in einem weiteren Schritt g) eine die Zuordnung charakterisierende Kalibrierinformation bereitgestellt werden PROV-CAL.

Die Röntgenerfassungseinheit kann beispielsweise eine Röntgenkamera und/oder einen Röntgendetektor umfassen. Für die wiederholte Ausführung der Schritte b) bis f) können vorteilhafterweise jeweils zumindest vier verschiedene Hochspannungen und/oder Ablenkströme vorgegeben werden. Des Weiteren kann das Identifizieren der Zuordnung DET-CAL zwischen der Ablenkung des Elektronenstrahls und dem Ablenkstrom und/oder der Hochspannung eine jeweilige Interpolation umfassen.

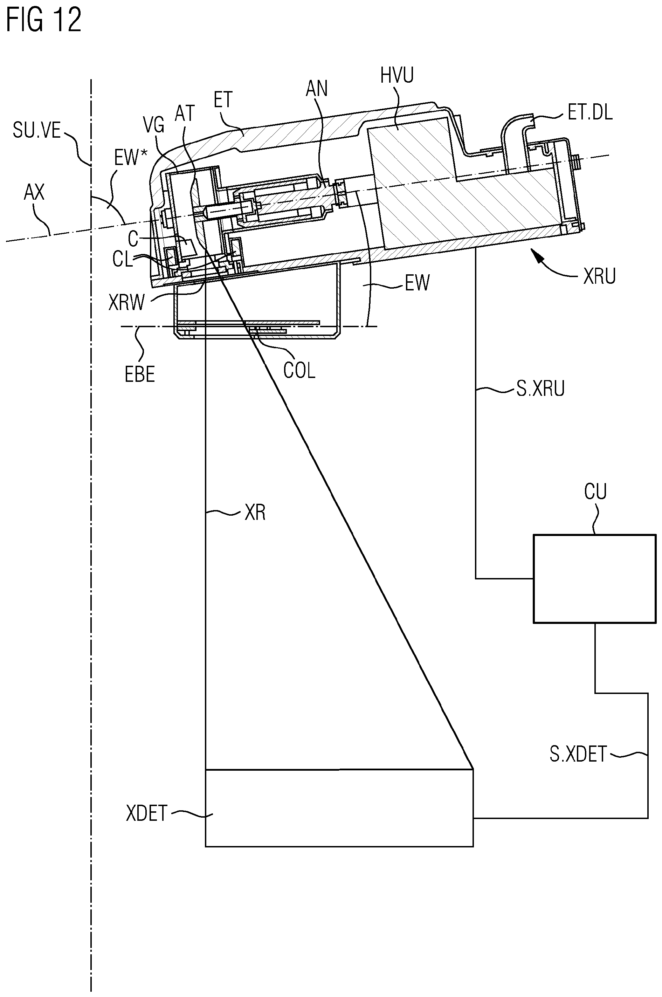

Fig. 12 zeigt eine schematische Darstellung einer vorteilhaften Ausführungsform eines vorgeschlagenen Bildgebungsgeräts umfassend eine vorgeschlagene Röntgeneinheit XRU, einen Röntgendetektor XDET und eine Steuerungseinheit CU. Dabei können die Röntgeneinheit XRU und der Röntgendetektor XDET in Gegenüberstellung zueinander angeordnet sein. Ferner kann der Röntgendetektor XDET zur Erfassung von mittels der Röntgeneinheit XRU emittierbarer Röntgenstrahlung ausgebildet sein. Die Steuerungseinheit CU kann dazu ausgebildet sein, die Röntgeneinheit XRU mittels eines Signals S.XRU zu steuern, beispielsweise zum Aussenden der Röntgenstrahlung. Ferner kann der Röntgendetektor XDET dazu ausgebildet sein, ein Signal S.XDET in Abhängigkeit der erfassten Röntgenstrahlung an die Steuerungseinheit CU bereitzustellen. Vorteilhafterweise kann die Röntgeneinheit XRU beweglich bezüglich des Röntgendetektors XDET gelagert sein. Ferner kann die Steuerungseinheit CU dazu ausgebildet sein, eine Bestromung der Spule CL in Abhängigkeit einer momentanen Positionierung der Röntgeneinheit XRU derart anzupassen, dass der von der Kathode C aussendbare Elektronenstrahl mittels des Magnetfelds zur Minimierung einer Bewegung des Brennflecks bezüglich des Röntgendetektors XDET abgelenkt wird.

In Fig. 12 ist ein Betriebszustand des Bildgebungsgeräts schematisch dargestellt. Dabei kann die Röntgeneinheit XRU gemäß einem Verfahren zum Betrieb einer Röntgeneinheit XRU betrieben werden. Hierbei kann die Röntgeneinheit XRU Röntgenstrahlung XR zur Beleuchtung des Röntgendetektors XDET aussenden.

Das Bildgebungsgerät kann ferner eine Standeinheit, eine erste und eine zweite Halteeinheit umfassen (hier nicht gezeigt). Dabei kann die erste Halteeinheit rotierbar gelagert an der Standeinheit angeordnet sein. Ferner kann die Röntgeneinheit XRU an der ersten Halteeinheit angeordnet sein. Zudem kann die zweite Halteeinheit an der Standeinheit angeordnet sein. Dabei kann der Röntgendetektor XDET an der zweiten Halteeinheit angeordnet sein. Dabei können die erste und die zweite Halteeinheit die Röntgeneinheit und den Röntgendetektor in Gegenüberstellung zueinander halten. Vorteilhafterweise kann die Röntgeneinheit XRU parallel zu einer, insbesondere vertikalen, Ebene SU.VE der Standeinheit beweglich gelagert sein. Dabei kann die Anodenachse AX einen vordefinierten Winkel EW* kleiner 90°, insbesondere 84°, bezüglich der Ebene SU.VE der Standeinheit SU aufweisen. Durch die Neigung der Anodenachse AX bezüglich der Ebene SU.VE der Standeinheit SU um den vorgegebenen Winkel EW* kann ein Abstand zwischen dem Brennfleck und einer Brustwand eines Untersuchungsobjekts reduziert werden. Für einen vorgegebenen Winkel EW* von 84° kann der Abstand zwischen dem Brennfleck und der Brustwand um etwa 10 % verkürzt werden.

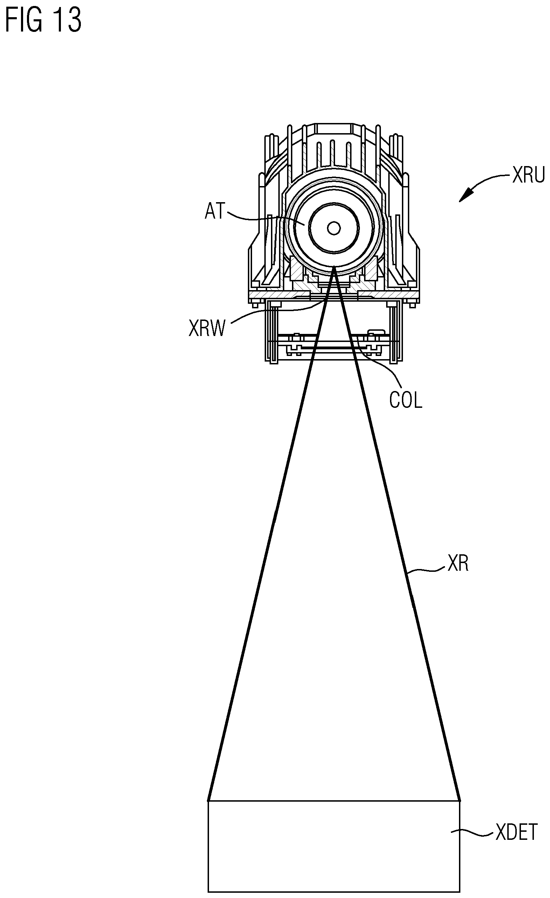

Fig. 13 zeigt eine Ansicht des in Fig. 12 schematisch dargestellten Bildgebungsgeräts senkrecht zur Blendenebene EBE der Blendeneinheit COL.

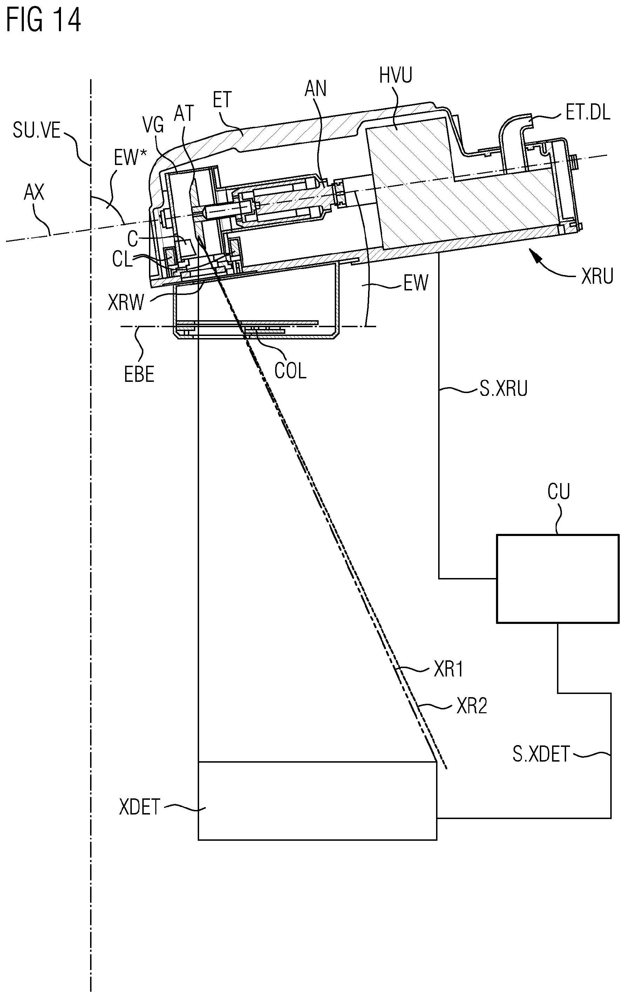

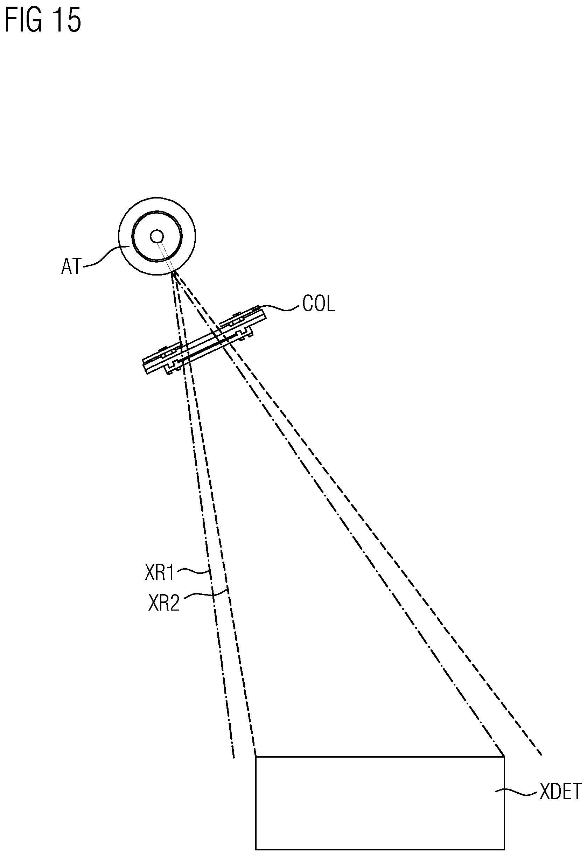

Fig. 14 und 15 zeigen schematische Darstellungen einer vorteilhaften Ausführungsform des Bildgebungsgeräts. Dabei ist in den Fig. 14 und 15 ein Verlauf der Röntgenstrahlung für zwei verschiedene Betriebszustände des Bildgebungsgeräts mit jeweiligen Positionierungen der Röntgeneinheit XRU, insbesondere des Anodentellers AT, bezüglich des Röntgendetektors illustriert. XR1 und XR2 illustrieren jeweils einen räumlichen Verlauf der Röntgenstrahlung bei den verschiedenen Positionierungen der Röntgeneinheit XRU bezüglich des Röntgendetektors XDET. Dabei kann die Steuerungseinheit CU in den beiden Betriebszuständen die Bestromung der Spule CL in Abhängigkeit der jeweiligen Positionierung der Röntgeneinheit XRU derart anpassen, dass der von der Kathode C ausgesendete Elektronenstrahl mittels des Magnetfelds zur Minimierung einer Bewegung des Brennflecks bezüglich des Röntgendetektors XDET abgelenkt wird.

Des Weiteren kann die Blendeneinheit COL in den beiden Betriebszuständen derart eingestellt sein, dass die jeweilige Röntgenstrahlung XR1 und XR2 den Röntgendetektor XDET in Abhängigkeit der Ablenkung des Brennflecks teilweise überstrahlt.

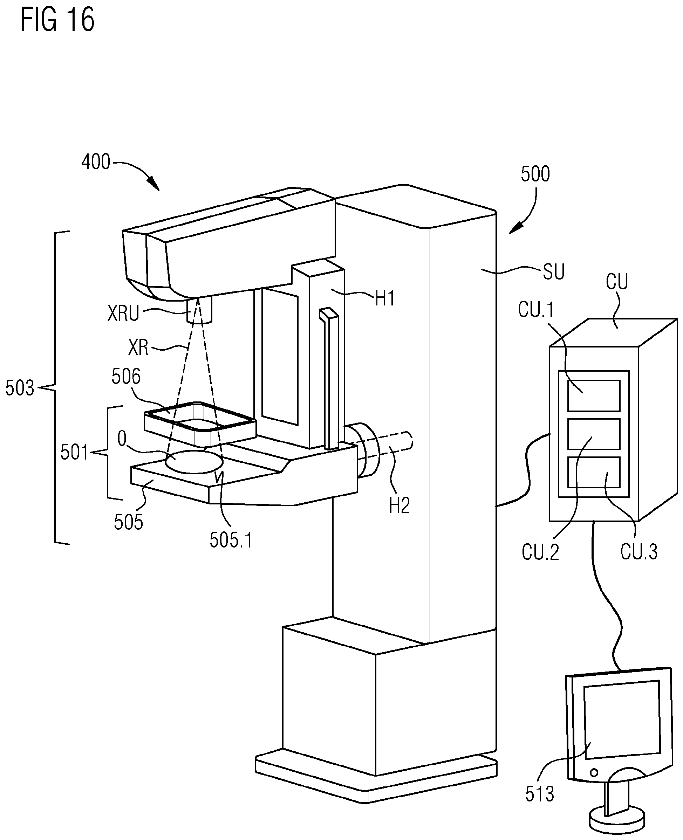

Fig. 16 zeigt eine schematische Darstellung einer weiteren vorteilhaften Ausführungsform eines vorgeschlagenen Bildgebungsgeräts. Das Bildgebungsgerät kann dabei insbesondere als Mammographiesystem 500 ausgebildet sein.

Das Mammographiesystem 500 ist in Fig. 16 beispielhaft und grob schematisch dargestellt. Das Mammographiesystem 500 kann insbesondere für eine Tomosynthese ausgebildet sein. Relative Richtungen wie "oben", "unten" usw. beziehen sich auf das Mammographiesystem 500, das für den bestimmungsgemäßen Betrieb eingerichtet wurde. Das Mammographiesystem 500 kann eine Standeinheit SU, eine erste Halteeinheit H1 und eine zweite Halteeinheit H2 umfassen. Die Standeinheit SU kann beispielsweise eine stehende Säule umfassen. Die stehende Säule kann am Boden montiert sein. Die erste Halteeinheit H1 kann rotierbar gelagert an der Standeinheit SU, insbesondere der stehenden Säule, angeordnet sein. Ferner kann die Röntgeneinheit XRU an der ersten Halteeinheit H1 angeordnet sein. Ferner kann die zweite Halteeinheit H2 an der Standeinheit SU, insbesondere der stehenden Säule 507 angeordnet sein. Dabei kann der Röntgendetektor XDET an der zweiten Halteeinheit H2 angeordnet sein. Die erste und die zweite Halteeinheit können die Röntgeneinheit XRU und den Röntgendetektor XDET in Gegenüberstellung zueinander halten, insbesondere in einer Quellen-Detektor-Anordnung 503. Der Röntgendetektor XDET kann an seiner Oberfläche, insbesondere einer der Röntgeneinheit XRU zugewandten Seite, einer Abdeckung XDET.1 umfassen. Die Quellen-Detektor-Anordnung 503 kann beweglich mit der Standeinheit SU verbunden sein, so dass eine Höhe der Abdeckung XDET.1 des Röntgendetektors XDET, auf eine Brusthöhe eines Untersuchungsobjekts einstellbar ist.

Die erste Halteeinheit H1 kann vorteilhafterweise L- oder J-förmig ausgebildet und mittels eines ersten Schenkels an der Standeinheit SU, insbesondere der Säule, angeordnet sein. Dabei kann die Röntgeneinheit XRU an einem weiteren Schenkel der ersten Halteeinheit H1 angeordnet sein.

Eine Brust O des Untersuchungsobjekts (hier schematisch dargestellt) kann ein zu untersuchendes Objekt bilden und in Ausführungen für eine Untersuchung auf der Abdeckung XDET.1 des Röntgendetektors XDET positioniert sein. Die Abdeckung XDET.1 kann hierbei eine zweite Kompressionsplatte ausbilden. Über der Brust O und der Abdeckung XDET.1 kann eine Kompressionsplatte 506 angeordnet sein, die beweglich bzw. verfahrbar mit der Quellen-DetektorAnordnung 503 verbunden ist. Für die Untersuchung kann die Brust O komprimiert und gleichzeitig fixiert werden, indem die Kompressionsplatte 506 darauf abgesenkt wird, so dass Druck auf die Brust O zwischen der Kompressionsplatte 506 und der Abdeckung XDET.1 ausgeübt wird. Alternativ kann für die Kompression anstelle der Abdeckung XDET.1 des Röntgendetektors XDET eine zweite Kompressionsplatte verwendet werden, sodass die Brust O zwischen zwei Kompressionsplatten komprimiert wird. Die beiden Kompressionsplatten oder die Kompressionsplatte 506 und die Abdeckung XDET.1 des Röntgendetektors XDET können von einer Kompressionseinheit 501 umfasst sein. Die Kompressionseinheit 501 kann außerdem noch eine Verfahreinheit umfassen, um die Kompressionsplatte 506 bzw. die Kompressionsplatten zum Komprimieren zu verfahren.

Die Röntgeneinheit XRU kann gegenüber dem Röntgendetektor XDET derart angeordnet sein, dass der Röntgendetektor XDET von der Röntgeneinheit XRU emittierte Röntgenstrahlung XR detektiert, nachdem zumindest ein Teil der Röntgenstrahlung XR die Brust O passiert hat.

Die Röntgenquelle XRU kann in Ausführungen relativ zum Röntgendetektor XDET mittels der ersten Halteeinheit H1, insbesondere eines Dreharms, beispielsweise in einem Bereich von ±25° um eine Grundposition, in der der Dreharm senkrecht zur Abdeckung XDET.1 des Röntgendetektors XDET steht, bewegbar bzw. rotierbar bzw. verschwenkbar ausgebildet sein. Vorteilhafterweise kann die Röntgeneinheit XRU parallel zu einer, insbesondere vertikalen, Ebene der Standeinheit, insbesondere der Säule 507, beweglich gelagert sein. Dabei kann die Anodenachse AX der Röntgeneinheit XRU einen vordefinierten Winkel kleiner 90°, insbesondere 84°, bezüglich der Ebene der Standeinheit SU aufweisen.

Das Mammographiesystem 500 kann insbesondere eine Steuerungseinheit CU mit einer Schnittstelle CU.1, einer Recheneinheit CU.2 und einer Speichereinheit CU.3 umfassen. Die Steuerungseinheit CU kann insbesondere mit einem Terminal 513 verbunden sein, das beispielsweise über eine Benutzeroberfläche oder Anzeigeeinheit verfügt, über die ein Benutzer Befehle an das Mammographiesystem 500 übermitteln oder Messergebnisse, beispielsweise die erfassten Bilddaten, insbesondere Projektionsaufnahmen bzw. Röntgenaufnahmen, abrufen kann. Die Steuerungseinheit CU kann sich im selben Raum wie das Mammographiesystem 500 befinden, es kann sich aber auch in einem angrenzenden Kontrollraum oder in einer noch größeren räumlichen Entfernung befinden.

Die Steuerungseinheit CU kann insbesondere ein Computer, ein Mikrocontroller oder ein integrierter Schaltkreis (engl. integrated circuit, IC) sein. Alternativ kann die Steuerungseinheit CU ein reales oder virtuelles Computer-Netzwerk sein (eine technische Bezeichnung für ein reales Computer-Netzwerk ist "Cluster", eine technische Bezeichnung für ein virtuelles Computer-Netzwerk ist "Cloud"). Die Steuerungseinheit CU kann als virtuelles System ausgebildet sein, welches auf einem Computer oder einem realen Computer-Netzwerk oder einem virtuellen Computer-Netzwerk ausgeführt wird (eine technische Bezeichnung ist "Virtualization").

Die Schnittstelle CU.1 kann eine Hardware- oder Software-Schnittstelle sein (beispielsweise ein PCI-Bus, CAN-Bus, USB oder Firewire). Die Recheneinheit CU.2 kann Hardware- und/oder Software-Bestandteile umfassen, beispielsweise einen Mikroprozessor oder einen sogenannten FPGA (Field Programmable Gate Array). Die Speichereinheit CU.3 kann als nicht permanent arbeitender Arbeitsspeicher (Random Access Memory, RAM) oder als permanenter Massenspeicher (Festplatte, USB-Stick, SD-Karte, Solid State Disk (SSD)) ausgebildet sein.

Die Schnittstelle CU.1 kann insbesondere eine Mehrzahl an Sub-Schnittstellen umfassen, die unterschiedliche Verfahrensschritte des jeweiligen erfindungsgemäßen Verfahrens ausführen. Mit anderen Worten kann die Schnittstelle CU.1 als eine Mehrzahl an Schnittstellen CU.1 ausgebildet sein. Die Recheneinheit CU.2 kann insbesondere eine Mehrzahl an Sub-Recheneinheiten umfassen, die unterschiedliche Verfahrensschritte des jeweiligen erfindungsgemäßen Verfahrens ausführen. Mit anderen Worten kann die Recheneinheit CU.2 als eine Mehrzahl an Recheneinheiten CU.2 ausgebildet sein.

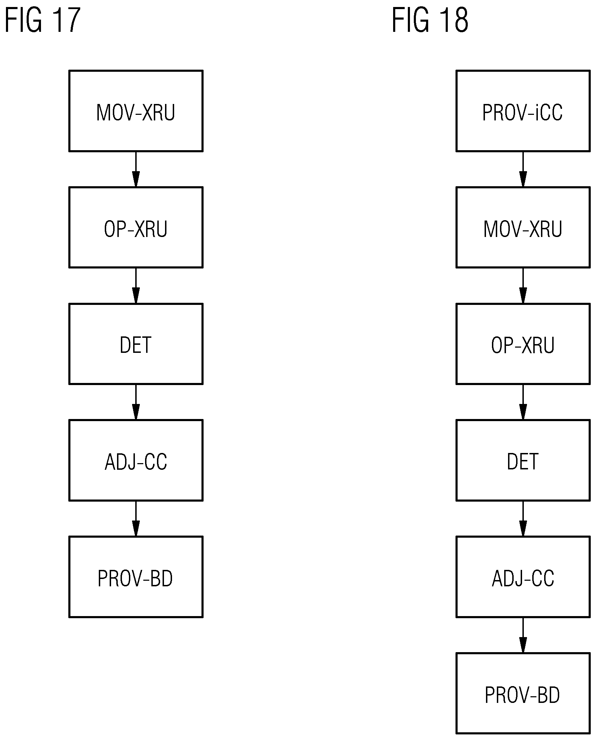

Fig. 17 zeigt eine schematische Darstellung einer vorteilhaften Ausführungsform eines vorgeschlagenen Verfahrens zum Erfassen von Bilddaten mittels eines vorgeschlagenen Bildgebungsgeräts. Dabei kann die Röntgeneinheit XRU entlang eines Aufnahmepfads bezüglich des Röntgendetektors bewegt werden MOV-XRU. Der Aufnahmepfad kann einen oder mehrere Aufnahmeabschnitte für die Anordnung der Röntgeneinheit XRU vorgeben. Die Röntgeneinheit XRU kann bei Anordnung in dem Aufnahmeabschnitt oder einem der mehreren Aufnahmeabschnitte gemäß einem vorgeschlagenen Verfahren zum Betrieb einer Röntgeneinheit XRU betrieben werden OP-XRU. Der Röntgendetektor XDET kann die mittels der Röntgeneinheit XRU emittierte Röntgenstrahlung erfassen DET. Die Bestromung der Spule CL kann während der Bewegung der Röntgeneinheit XRU entlang des Aufnahmeabschnitts oder eines der mehreren Aufnahmeabschnitte des Aufnahmepfads in Abhängigkeit einer momentanen Positionierung der Röntgeneinheit XRU derart angepasst werden ADJ-CC, dass der von der Kathode C ausgesendete Elektronenstrahl mittels des Magnetfelds zur Minimierung einer Bewegung des Brennflecks bezüglich des Röntgendetektors abgelenkt wird. Die Bilddaten können basierend auf der erfassten Röntgenstrahlung bereitgestellt werden PROV-BD.

Die Bestromung der Spule CL kann zusätzlich in Abhängigkeit einer Bewegungsgeschwindigkeit der Röntgeneinheit XRU erfolgen.

Die Bestromung PROV-CA der Kathode C zum Bereitstellen des Elektronenstrahls bei Anordnung der Röntgeneinheit XRU in jeweils einem des einen oder der mehreren Aufnahmeabschnitte des Aufnahmepfads kann gepulst erfolgen.

Bei sehr kurzen Pulsen kann die Minimierung einer Bewegung des Brennflecks bezüglich des Röntgendetektors deaktiviert werden. Dies kann in bestimmten Aufnahmeabschnitten des Aufnahmepfads vorteilhaft sein.

In manchen Fällen, insbesondere bei geringen Kompressionsdicken der Brust O im Rahmen einer Mammographieuntersuchung, kann die Minimierung der Bewegung des Brennflecks bezüglich des Röntgendetektors möglicherweise nicht erforderlich sein. Hierfür kann die Minimierung selektiv deaktivierbar sein.

Fig. 18 zeigt eine schematische Darstellung einer weiteren vorteilhaften Ausführungsform eines vorgeschlagenen Verfahrens zum Erfassen von Bilddaten mittels eines vorgeschlagenen Bildgebungsgeräts. Dabei kann die Bestromung der Spule in einem vordefinierten Zeitraum vor dem Bereitstellen der Hochspannung PROV-HV zwischen der Kathode C und dem Anodenteller AT und/oder der Bestromung PROV-CA der Kathode C zum Bereitstellen des Elektronenstrahls erfolgen. Insbesondere kann ein initialer Ablenkstrom zur Bestromung der Spule CL vor Beginn der Bewegung MOV-XRU der Röntgeneinheit XRU in Abhängigkeit der bereitgestellten Hochspannung zwischen der Kathode C und dem Anodenteller AT und einer geplanten Bewegungsgeschwindigkeit der Röntgeneinheit XRU vorgegeben werden PROV-iCC.

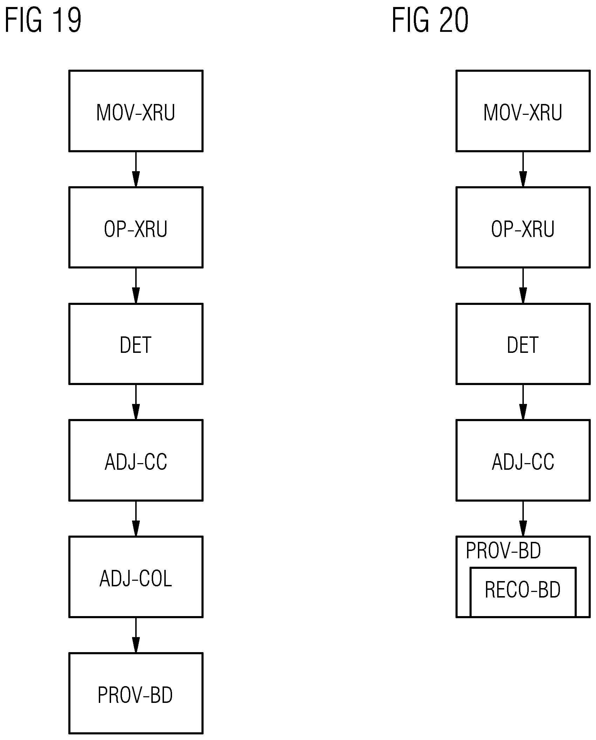

Fig. 19 zeigt eine schematische Darstellung einer weiteren vorteilhaften Ausführungsform eines vorgeschlagenen Verfahrens zum Erfassen von Bilddaten mittels eines vorgeschlagenen Bildgebungsgeräts. Dabei kann die Röntgeneinheit XRU eine Blendeneinheit COL umfassen, welche zur räumlichen Begrenzung der durch das Strahlenaustrittsfenster XRW austretenden Röntgenstrahlung ausgebildet ist. Die Blendeneinheit COL kann innerhalb des einen oder der mehreren Aufnahmeabschnitte und/oder zwischen jeweils aufeinanderfolgenden Aufnahmeabschnitten des Aufnahmepfads der Röntgeneinheit XRU angepasst werden ADJ-COL. Das Anpassen ADJ-COL der Blendeneinheit COL kann innerhalb des einen oder der mehreren Aufnahmeabschnitte des Aufnahmepfads entgegen einer Bewegungsrichtung der Röntgeneinheit XRU erfolgen. Ferner kann das Anpassen ADJ-COL der Blendeneinheit COL zwischen jeweils aufeinanderfolgenden Aufnahmeabschnitten des Aufnahmepfads entlang einer Bewegungsrichtung der Röntgeneinheit XRU erfolgen.

Fig. 20 zeigt eine schematische Darstellung einer weiteren vorteilhaften Ausführungsform eines vorgeschlagenen Verfahrens zum Erfassen von Bilddaten mittels eines vorgeschlagenen Bildgebungsgeräts. Dabei kann das Bereitstellen der Bilddaten PROV-BD eine Rekonstruktion RECO-BD der Bilddaten basierend auf der erfassten Röntgenstrahlung und einer Information zu dem Aufnahmeabschnitt oder den jeweiligen Aufnahmeabschnitten der mehreren Aufnahmeabschnitte der Röntgeneinheit XRU bezüglich des Röntgendetektors XDET umfassen.

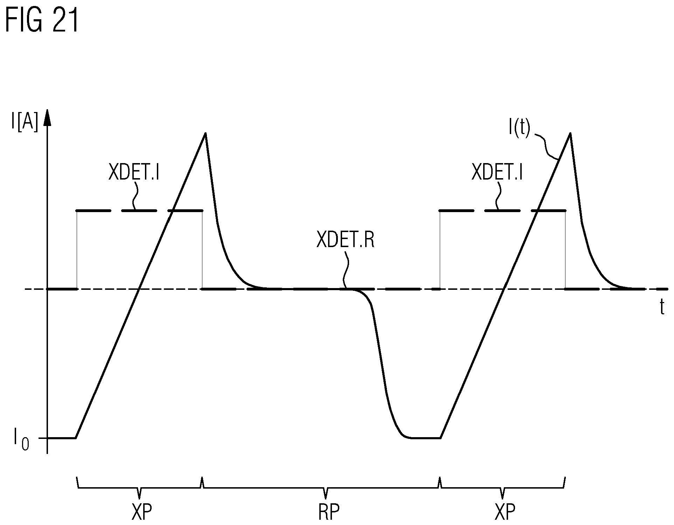

Fig. 21 zeigt eine schematische Darstellung einer beispielhaften Ablenkstrom-Zeit-Kurve I(t). Der in Fig. 21 illustrierte Graph veranschaulicht zwei Hauptzyklen des Betriebs der Röntgeneinheit XRU, eine Röntgenstrahlungsphase XP und eine Auslesephase RP.

In der Röntgenstrahlungsphase XP kann die Röntgeneinheit XRU entlang eines Aufnahmeabschnitts des Aufnahmepfads bewegt werden. Dabei kann die Röntgeneinheit XRU gemäß einem vorgeschlagenen Verfahren zum Betrieb einer Röntgeneinheit XRU betrieben werden und Röntgenstrahlung aussenden. Während der Röntgenstrahlungsphase kann der Röntgendetektor XDET die von der Röntgeneinheit XRU emittierte Röntgenstrahlung erfassen. Hierfür kann der Röntgendetektor XDET beispielsweise in einem integrierenden Modus XDET.I betrieben werden und die einfallende Röntgenstrahlung integrieren. Während der Röntgenstrahlungsphase RP kann der Ablenkstrom I ausgehend von einem initialen Ablenkstrom I₀ in der Spule ansteigen. Der initiale Ablenkstrom I₀ kann abhängig von der Hochspannung und einer, insbesondere geplanten, Bewegungsgeschwindigkeit der Röntgeneinheit XRU sein. Der Anstieg des Ablenkstroms kann dabei abhängig von einer Bewegungsgeschwindigkeit der Röntgeneinheit XRU sein. Hierdurch kann die Ablenkung des Elektronenstrahls vorteilhafterweise die Bewegung der Röntgeneinheit XRU bezüglich des Röntgendetektors XDET kompensieren.

In der Auslesephase RP fließt vorteilhafterweise kein Ablenkstrom durch die Spule. Während der Auslesephase RP kann ein Auslesen des Röntgendetektors XDET erfolgen. Hierfür kann der Röntgendetektor XDET in einem Auslesemodus XDET.R betrieben werden.

Kurz vor Beginn der nächsten Röntgenstrahlungsphase XP kann die Spule mit dem initialen Ablenkstrom I₀ bestromt werden.

In einer Ausführungsform kann die Ablenkung des Elektronenstrahls pro Aufnahme asymmetrisch erfolgen. Dies kann dazu führen, dass der Brennfleck oder dessen Abbildung auf dem Strahlenaustrittsfenster XRW nicht statisch verbleibt.

Die Ablenkung des Elektronenstrahls kann von der Hochspannung und der Bestromung der Kathode abhängig sein. Bei unterschiedlichen Aufnahmedauern kann die Ablenkung vorzeitig beendet werden, sodass nicht der gesamte Strombereich der Ablenkung, insbesondere des Ablenkstroms, durchlaufen wird.

Vorteilhafterweise kann die Regelung der Ablenkung nicht auf eine statische Position des Brennflecks auf dem Strahlenaustrittsfenster XRW ausgerichtet sein. Stattdessen kann auf dem Röntgendetektor XDET während der Röntgenstrahlungsphase XP eine beabsichtigte Verwischung des Brennflecks erzeugt werden, wobei sich die Brennfleckgeometrie von "kleiner" zu "normal" verändern kann. Dies kann zu einem dynamischen Gewinn in der Modulationsübertragungsfunktion (MTF) und Auflösung bei den individuellen Aufnahmen führen.

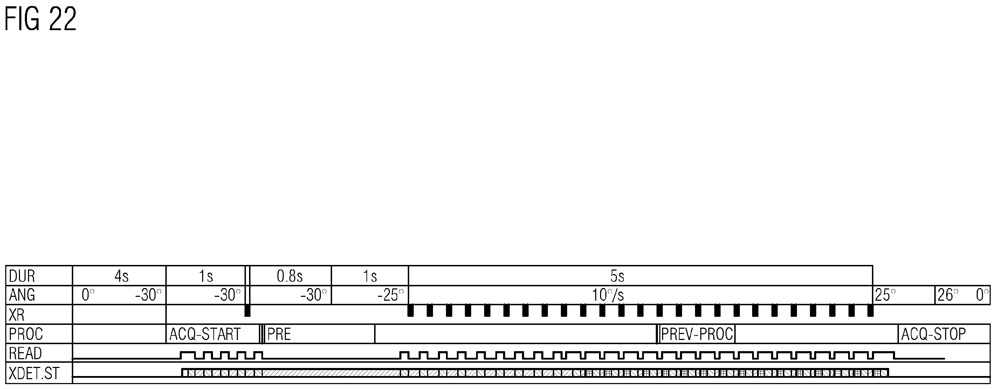

Fig. 22 zeigt eine schematische Darstellung eines beispielhaften Ablaufdiagramms zur Verwendung in einem vorgeschlagenen Verfahren zum Erfassen von Bilddaten mittels eines vorgeschlagenen Bildgebungsgeräts, insbesondere eines Tomosynthese-Scans mit einer automatischen Belichtungsregelung bei einer Bildaufnahmerate von 5 Bildern pro Sekunde.

In der ersten Zeile DUR des Ablaufdiagramms sind die jeweiligen beispielhaften Zeitdauern für jede dargestellte Phase dargestellt. In einer weiteren Zeile ANG des Ablaufdiagramms ist ein Winkel zwischen der Röntgeneinheit XRU und dem Röntgendetektor XDET angegeben. Der beispielhafte Ablauf beginnt mit einer 4-sekündigen Bewegung der Röntgeneinheit XRU zu einer Position auf einer Normalen einer der Röntgeneinheit zugewandten, insbesondere röntgensensitiven, Oberfläche des Röntgendetektors XDET. Dabei kann ein Winkel zwischen der Röntgeneinheit XRU und dem Röntgendetektor XDET von 0° auf -30° geändert werden.

In einer weiteren Zeile XR des Ablaufdiagramms ist das jeweilige Emittieren von Röntgenstrahlung durch die Röntgeneinheit XRU schematisch dargestellt.

Nach einer 1-sekündigen Vorbereitungsphase für den Röntgendetektor XDET und die Hochspannungseinheit HVU kann eine kurze Wartezeit für die automatische Belichtungsregelung folgen. Anschließend kann eine Tomosynthese-Geschwindigkeit ausgewählt und die Bewegung der Röntgeneinheit XRU bezüglich des Röntgendetektors XDET gestartet werden. Der eigentliche Tomosynthese-Scan kann beispielsweise 5 Sekunden dauern, während derer der Winkel zwischen der Röntgeneinheit XRU und dem Röntgendetektor XDET von -25° auf +25° geändert wird. Vorteilhafterweise kann der Tomosynthese-Scan starten, wenn die Röntgeneinheit XRU einen Winkel von -25° bezüglich des Röntgendetektors XDET aufweist.

Während des Tomosynthese-Scans können mehrere Röntgenpulse, insbesondere Pulse von Röntgenstrahlung, ausgelöst werden, insbesondere mittels einer gepulsten Bestromung der Kathode C.

In einer weiteren Zeile PROC des Ablaufdiagramms sind verschiedene Schritte einer Bildverarbeitung illustriert. In einem ersten Schritt ACQ-START kann die Aufnahme gestartet werden. Dabei können zunächst Voraufnahmen erfasst werden. In einem weiteren Schritt PRE kann eine Analyse der Voraufnahmen erfolgen, beispielsweise für die automatische Belichtungsregelung (AEC). In einem weiteren Schritt PREV-PROC kann während der Aufnahme der Bilddaten eine Verarbeitung der jeweils bislang aufgenommenen Bilddaten für eine Voransicht erfolgen. In einem weiteren Schritt ACQ-STOP können die erfassten Bilddaten an die Bildverarbeitung bereitgestellt und die Aufnahme gestoppt werden.

Eine weitere Zeile READ des Ablaufdiagramms illustriert ein READ-Signal an den Röntgendetektor XDET, was eine kontinuierliche Datenerfassung während des Tomosynthese-Scans verdeutlicht. Ein Status, insbesondere Betriebsmodus, des Röntgendetektors XDET ist in einer weiteren Zeile XDET.ST des Ablaufdiagramms dargestellt. Der Status des Röntgendetektors XDET kann zwischen einem Leerlauf, Integrieren und Auslesen wechseln, was die verschiedenen Betriebszustände des Röntgendetektors XDET während des Scanvorgangs zeigt.

Die in den beschriebenen Figuren enthaltenen schematischen Darstellungen bilden keinerlei Maßstab oder Größenverhältnisse ab.

Es wird abschließend noch einmal darauf hingewiesen, dass es sich bei den vorhergehend detailliert beschriebenen Verfahren sowie bei den dargestellten Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Element" nicht aus, dass die betreffenden Komponenten aus mehreren zusammenwirkenden Teil-Komponenten bestehen, die gegebenenfalls auch räumlich verteilt sein können.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

## Patentansprüche

1. Verfahren zum Erfassen von Bilddaten mittels eines Bildgebungsgeräts,
wobei eine Röntgeneinheit (XRU) entlang eines Aufnahmepfads bezüglich eines Röntgendetektors (XDET) bewegt wird (MOV-XRU),
wobei der Aufnahmepfad einen oder mehrere Aufnahmeabschnitte für die Anordnung der Röntgeneinheit (XRU) vorgibt,
wobei ein Betreiben der Röntgeneinheit (XRU) bei Anordnung in dem einen Aufnahmeabschnitt oder einem der mehreren Aufnahmeabschnitte folgende Verfahrensschritte umfasst:
- Bereitstellen einer Hochspannung (PROV-HV) zwischen einer Kathode (C) und einem Anodenteller (AT),
- Bestromung (PROV-CA) der Kathode (C) zum Bereitstellen eines Elektronenstrahls,
- Rotieren (ROT) des Anodentellers (AT) mit einer Rotationsfrequenz innerhalb eines vorgegebenen Rotationsfrequenzbereichs,
- Bestromung (PROV-CC) einer Spule (CL) mit einem Ablenkstrom zum Bereitstellen eines Magnetfelds,
- Anpassen (ADJ-DEF) der Ablenkung des Elektronenstrahls durch Anpassen der Bestromung der Spule (CL),
wobei der Röntgendetektor (XDET) die mittels der Röntgeneinheit (XRU) emittierte Röntgenstrahlung erfasst (DET),
wobei die Bestromung der Spule (CL) während der Bewegung der Röntgeneinheit (XRU) entlang des Aufnahmeabschnitts oder jeweils eines der mehreren Aufnahmeabschnitte des Aufnahmepfads in Abhängigkeit einer momentanen Positionierung der Röntgeneinheit (XRU) derart angepasst wird (ADJ-CC), dass der von der von der Kathode (C) ausgesendete Elektronenstrahl mittels des Magnetfelds zur Minimierung einer Bewegung des Brennflecks bezüglich des Röntgendetektors (XDET) abgelenkt wird,
wobei die Bestromung der Spule in einem vordefinierten Zeitraum vor dem Bereitstellen der Hochspannung zwischen der Kathode und dem Anodenteller und/oder der Bestromung der Kathode zum Bereitstellen des Elektronenstrahls erfolgt,
wobei die Bilddaten basierend auf der erfassten Röntgenstrahlung bereitgestellt werden (PROV-BD),
wobei das Bildgebungsgerät die Röntgeneinheit (XRU), den Röntgendetektor (XDET) und eine Steuerungseinheit (CU) umfasst,
wobei die Röntgeneinheit (XRU) und der Röntgendetektor (XDET) in Gegenüberstellung zueinander angeordnet sind,
wobei der Röntgendetektor (XDET) zur Erfassung von mittels der Röntgeneinheit (XRU) emittierbarer Röntgenstrahlung ausgebildet ist,
wobei die Röntgeneinheit (XRU) beweglich bezüglich des Röntgendetektors (XDET) gelagert ist,
wobei die Steuerungseinheit (CU) dazu ausgebildet ist, eine Bestromung der Spule (CL) in Abhängigkeit einer momentanen Positionierung der Röntgeneinheit (XRU) derart anzupassen, dass der von der Kathode (C) aussendbare Elektronenstrahl mittels des Magnetfelds zur Minimierung einer Bewegung des Brennflecks bezüglich des Röntgendetektors (XDET) abgelenkt wird,
wobei die Röntgeneinheit (XRU) eine Röntgenröhre und die Spule (CL) umfasst,
wobei die Röntgenröhre die Kathode (C), eine Drehanode und ein Vakuumgehäuse (VG) umfasst,
wobei die Drehanode den um eine Anodenachse (AX) rotierbar gelagerten Anodenteller (AT) umfasst,
wobei die Kathode (C) und die Drehanode innerhalb des Vakuumgehäuses (VG) angeordnet sind,
wobei die Spule (CL) außerhalb des Vakuumgehäuses (VG) angeordnet ist,
wobei die Spule (CL) und die Röntgenröhre voneinander galvanisch getrennt sind,
wobei die Spule (CL) dazu ausgebildet ist, das Magnetfeld zu erzeugen, welches einen Zwischenraum zwischen der Kathode (C) und dem Anodenteller (AT) durchdringt,
wobei das Magnetfeld dazu ausgebildet ist, den in einem Brennfleck der Röntgenröhre auf den Anodenteller (AT) auftreffenden, von der Kathode (C) ausgehenden Elektronenstrahl abzulenken,
wobei das Vakuumgehäuse (VG) an einer bezüglich der Anodenachse (AX) radial angeordneten Seite ein Strahlenaustrittsfenster (XRW) aufweist, welches zum Durchlass einer von dem Brennfleck auf dem Anodenteller (AT) ausgehenden Röntgenstrahlung ausgebildet ist.

2. Verfahren nach Anspruch 1,
wobei die Röntgeneinheit (XRU) eine Blendeneinheit (COL) umfasst, welche zur räumlichen Begrenzung der durch das Strahlenaustrittsfenster (XRW) austretenden Röntgenstrahlung ausgebildet ist,
wobei die Blendeneinheit (COL) innerhalb des einen oder der mehreren Aufnahmeabschnitte und/oder zwischen jeweils aufeinanderfolgenden Aufnahmeabschnitten des Aufnahmepfads der Röntgeneinheit (XRU) angepasst wird (ADJ-COL).

3. Verfahren nach Anspruch 2,
wobei das Anpassen (ADJ-COL) der Blendeneinheit (COL) innerhalb des einen oder der mehreren Aufnahmeabschnitte des Aufnahmepfads entgegen einer Bewegungsrichtung der Röntgeneinheit (XRU) erfolgt.

4. Verfahren nach einem der Ansprüche 2 oder 3,
wobei das Anpassen (ADJ-COL) der Blendeneinheit (COL) zwischen jeweils aufeinanderfolgenden Aufnahmeabschnitten des Aufnahmepfads entlang einer Bewegungsrichtung der Röntgeneinheit (XRU) erfolgt.

5. Verfahren nach einem der Ansprüche 1 bis 4,
wobei die Bestromung der Spule zusätzlich in Abhängigkeit einer Bewegungsgeschwindigkeit der Röntgeneinheit erfolgt.

6. Verfahren nach einem der Ansprüche 1 bis 5,
wobei ein initialer Ablenkstrom zur Bestromung der Spule vor Beginn der Bewegung der Röntgeneinheit in Abhängigkeit der bereitgestellten Hochspannung zwischen der Kathode und dem Anodenteller und einer geplanten Bewegungsgeschwindigkeit der Röntgeneinheit vorgegeben wird.

7. Verfahren nach einem der Ansprüche 1 bis 6,
wobei die Bestromung der Kathode zum Bereitstellen des Elektronenstrahls bei Anordnung der Röntgeneinheit in jeweils einem des einen oder der mehreren Aufnahmeabschnitte des Aufnahmepfads gepulst erfolgt.

8. Verfahren nach einem der Ansprüche 1 bis 7,
wobei das Bereitstellen der Bilddaten eine Rekonstruktion der Bilddaten basierend auf der erfassten Röntgenstrahlung und einer Information zu dem Aufnahmeabschnitt oder den jeweiligen Aufnahmeabschnitten der mehreren Aufnahmeabschnitte der Röntgeneinheit bezüglich des Röntgendetektors umfasst.

9. Verfahren nach einem der vorhergehenden Ansprüche,
wobei die Röntgeneinheit (XRU) weiterhin ein leckdichtes Eintankgehäuse (ET) umfasst,
wobei die Röntgenröhre und die Spule (CL) innerhalb des Eintankgehäuses (ET) angeordnet sind,
wobei das Eintankgehäuse (ET) mit einem Kühl-Isoliermedium gefüllt ist.

10. Verfahren nach einem der vorhergehenden Ansprüche,
wobei die Röntgeneinheit (XRU) weiterhin eine Blendeneinheit (COL) umfasst, welche zur räumlichen Begrenzung der durch das Strahlenaustrittsfenster (XRW) austretenden Röntgenstrahlung ausgebildet ist.

11. Verfahren nach Anspruch 10,
wobei die Anodenachse (AX) einen vorgegebenen Winkel (EW), insbesondere 6°, bezüglich einer Blendenebene (EBE) der Blendeneinheit (COL) aufweist.

12. Verfahren nach einem der vorhergehenden Ansprüche,
wobei die Spule (CL) eine Spulengeometrie aufweist, welche an eine Geometrie der Röntgenröhre, insbesondere des Vakuumgehäuses (VG), angepasst ist.

13. Verfahren nach Anspruch 12,
wobei die Spulengeometrie eine Querschnittsgeometrie der Spule (CL) umfasst, wobei die Querschnittsgeometrie der Spule (CL) zumindest an einer Innenseite der Spule (CL) an die Geometrie des Vakuumgehäuses (VG) innerhalb eines Öffnungsbereichs der Spule (CL) angepasst ist.

14. Verfahren nach einem der vorhergehenden Ansprüche,
wobei der Elektronenstrahl in einem Betriebszustand der Röntgeneinheit (XRU) zumindest teilweise innerhalb eines Öffnungsbereichs der Spule (CL) angeordnet ist.

15. Verfahren nach Anspruch 14,
wobei der Brennfleck der Röntgenröhre (XRU) in dem Betriebszustand der Röntgeneinheit (XRU) innerhalb des Öffnungsbereichs der Spule (CL) angeordnet ist.

16. Verfahren nach einem der vorhergehenden Ansprüche,
wobei die Spule (CL) als Luftspule ausgebildet ist.

17. Verfahren nach einem der vorhergehenden Ansprüche,
wobei die Spule (CL) und der Elektronenstrahl durch einen aus einem unmagnetischen metallischen Material geringer elektrischer Leitfähigkeit gebildeten Wandabschnitt (BL) des Vakuumgehäuses (VG) voneinander getrennt sind.

18. Verfahren nach einem der vorhergehenden Ansprüche,
wobei die Kathode (C) einen Fokuskopf (FH) zur Fokussierung des Elektronenstrahls aufweist.

19. Verfahren nach Anspruch 18,
wobei der Fokuskopf (FH) zumindest durch seine Form zur elektrostatischen Fokussierung des Elektronenstrahls ausgebildet ist.

20. Verfahren nach einem der vorhergehenden Ansprüche,
wobei die Kathode (C) zumindest einen Emitter und einen Fokuskopf (FH) zur Fokussierung des Elektronenstrahls aufweist,
wobei die Bestromung der Kathode (C) eine Bestromung des zumindest einen Emitters umfasst,
wobei das Verfahren weiterhin ein Bereitstellen (PROV-CF) eines elektrischen Potenzials des zumindest einen Emitters oder eines weiteren elektrischen Potenzials an den Fokuskopf (FH) zur Fokussierung des Elektronenstrahls umfasst.

21. Computerprogrammprodukt mit einem Computerprogramm, welches direkt in einen Speicher einer Steuerungseinheit (CU) ladbar ist, mit Programmabschnitten, um alle Schritte eines Verfahrens nach einem der Ansprüche 1 bis 20 auszuführen, wenn die Programmabschnitte von der Steuerungseinheit (CU) ausgeführt werden.
